(19) Europäisches Patentamt
European Patent Office
Office européen des brevets

(11) Veröffentlichungsnummer: **0 146 785**
**B1**

(12) # EUROPÄISCHE PATENTSCHRIFT

(45) Veröffentlichungstag der Patentschrifft: **07.02.90**

(21) Anmeldenummer: **84114022.1**

(22) Anmeldetag: **20.11.84**

(51) Int. Cl. ⁵: **C 12 N 15/00**, C 12 N 9/99, C 12 P 21/00, C 07 K 7/10, A 61 K 37/64, A 61 K 39/395, C 12 N 5/00, G 01 N 33/53 // C12R1:19, C12R1:125, C12R1:865

(54) **Verfahren zur Herstellung von Protease-Inhibitoren.**

(30) Priorität: **21.11.83 CH 6422/83**
**13.04.84 CH 1863/84**

(43) Veröffentlichungstag der Anmeldung: **03.07.85 Patentblatt 85/27**

(45) Bekanntmachung des Hinweises auf die Patenterteilung: **07.02.90 Patentblatt 90/06**

(84) Bennante Vertragsstaaten: **AT BE CH DE FR GB IT LI LU NL SE**

(56) Entgegenhaltungen:
**DE-A-2 808 396**

**HOPPE-SEYLER'S ZEITSCHRIFT FUER PHYSIOLOGISCHE CHEMIE; Band 361, Heft 12, Dezember 1980(Berlin, New York) U. Seemüller et al. "Structure of the Elastase-Cathepsin G Inhibitor of the Leech Hirudo se-Cathepsin G Inhibitor of the Leech Hirudo medicinalis" Seite 1841-1846**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**
Patentinhaber: **PLANTORGAN WERK Heinrich G.E. Christensen KG**
**Hornbusch 1**
**D-2903 Bad Zwischenahn (DE)**

(72) Erfinder: **Rink, Hans**
**Rebenstrasse 10, CH-4125 Riehen (CH)**
Erfinder: **Liersch, Manfred, Dr.**
**Rütiring 26, CH-4125 Riehen (CH)**
Erfinder: **Sieber, Peter**
**Bachmattweg 10, CH-4153 Reinach (CH)**
Erfinder: **Rittel, Werner, Dr.**
**Finkelerweg 6, CH-4144 Arlesheim (CH)**
Erfinder: **Meyer, Franfois, Dr.**
**Lerchenberg 42, CH-8046 Zürich (CH)**
Erfinder: **Seemüller, Ursula, Dr.**
**Destouchesstrasse 60, D-8000 München 40 (DE)**
Erfinder: **Fritz, Hans, Prof. Dr.**
**Neulingerstrasse 15, D-8011 Hohenbrunn (DE)**
Erfinder: **Märki, Walter, Dr.**
**Bremenstallstrasse 30, CH-4313 Möhlin (CH)**
Erfinder: **Alkan, Sefik, Prof. Dr.**
**Binsenackerstrasse 3, CH-4125 Riehen (CH)**

(74) Vertreter: **Zumstein, Fritz jun., Dr.**
**Dr. F. Zumstein sen. Dr. E. Assmann Dr. R. Koenigsberger Dipl.-Ing. F. Klingseisen Dr. F. Zumstein jun. Bräuhausstrasse 4**
**D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 146 785 B1

LIBERGRAF, STOCKHOLM 1990

**Beschreibung**

Die Erfindung betrifft als Egline bezeichnete Protease-Inhibitoren sowie Verfahren zur Herstellung der genannten Egline mit Hilfe transformierter *E. coli* oder *Saccharomyces cerevisiae* Zellen.

Aus der Deutschen Offenlegungsschrift 2 808 396 sind zwei aus Blutegeln (*Hirudo medicinalis*) isolierte Protease-Inhibitoren, die als Eglin B und Eglin C bezeichnet werden, bekannt. Diese Polypeptide bestehen aus je 70 Aminosäuren, haben ein Molekulargewicht von ca. 8100 und sind starke Inhibitoren für Chymotrypsin, Subtilisin, für die tierischen und menschlichen Granulozyten-Proteasen Elastase und Cathepsin G sowie für die Mastzell-Protease Chymase (1). Weniger stark gehemmt werden Trypsin-ähnliche Proteasen.

Eglin C hat die folgende Primärstruktur (2):

ThrGluPheGlySerGluLeuLysSerPheProGluValValGlyLysThrVal
AspGlnAlaArgGluTyrPheThrLeuHisTyrProGlnTyrAspValTyrPheLeuProGluGly
SerProValThrLeuAspLeuArgTyrAsnArgValArgValPheTyrAsnProGlyThrAsnVal
ValAsnHisValProHisValGly

Eglin C enthält im Gegensatz zu den meisten bekannten Proteinase-Hemmern keine Disulfid-Brücke, es erweist sich auch für ein Miniprotein als ungewöhnlich stabil gegenüber Denaturierung durch Säure, Lauge oder Wärme und gegenüber proteolytischem Abbau. Die Primärstruktur von Eglin B unterscheidet sich von derjenigen von Eglin C durch Ersatz der Aminosäure 35, Tyrosin, durch Histidin.

Die Egline gehören zu den stärksten heute bekannten Hemmern von humaner und tierischer Granulozyten-Elastase, sowie von humanem Granulozyten-Cathepsin G und von bakteriellen Proteasen vom Typ des Subtilisins. Unkontrollierte bzw. übermässige Freisetzung dieser zellulären Proteasen im Organismus kann einen Entzündungsvorgang verstärken und Gewebeabbau durch unspezifische Proteolyse hervorrufen. Dies insbesondere deshalb, da diese für die intrazelluläre Verdauung zuständigen Enzyme im physiologischen (neutralen bis schwach alkalischen) Milieu optimal wirksam sind und native Gewebesubstanzen (z. B. Elastin) und humorale Faktoren (z. B. Blutgerinnungs- und Komplementfaktoren) rasch zu zerstören und zu inaktivieren vermögen. Auf Grund ihrer bis anhin bekannten Eigenschaften sind die Egline daher für die Anwendung in der medizinischen Therapie (Antiinflammation, Antiphlogistik, septischer Schock, Lungenemphysem, Mucoviscidose etc.) von grossem Interesse.

Im Blutegel werden die Egline nur in sehr geringen Mengen (ca. 16 µg/Egel) gebildet. Die Isolierung und Reinigung der Egline aus Blutegeln gestaltet sich daher sehr zeit- und kostenaufwendig und ist im kommerziellen Massstab nicht durchführbar.

Auf Grund der grossen Fortschritte in der sogenannten rekombinierten DNA-Technologie (oder Gentechnologie) ist es neuerdings möglich, die unterschiedlichsten physiologisch aktiven Polypeptide unter Verwendung dieser Technologie herzustellen.

Der vorliegenden Erfindung liegt die Aufgabe zugrunde, mit Hilfe von gentechnologischen Mitteln Expressionssysteme zur Verfügung zu stellen, die die mikrobielle Herstellung von Eglinen im industriellen Massstab erlaubt. Diese Aufgabe wird in der vorliegenden Erfindung gelöst durch die Bereitstellung von Hybridvektoren, die eine für ein Eglin kodierende DNA-Sequenz enthalten, welche von einer Expressionskontrollsequenz derart reguliert wird, dass ein Eglin in einem mit diesen Hybridvektoren transformierten Wirt exprimiert wird.

**Herstellung von DNA-Sequenzen, die für ein Eglin kodieren**

Unter der allgemeinen Bezeichnung "Egline" werden im Rahmen der vorliegenden Erfindung, wenn nicht spezifischer definiert, Polypeptide mit Proteinaseinhibitor-Aktivität und der Primärstruktur von Eglin B oder C verstanden, die aber auch am Threonin $N^{\alpha}$-acetyliert sein können.

Die Herstellung von DNA-Sequenzen, die für ein Eglin, z. B. Eglin B und insbesondere Eglin C, kodieren, ist dadurch gekennzeichnet, dass man aus genomischer Egel-DNA das Eglin-Strukturgen isoliert, oder aus Eglin-mRNA eine komplementäre doppelsträngige Eglin-DNA (Eglin-ds cDNA) herstellt.

Die Gewinnung von genomischer Eglin-DNA und von Eglin-ds cDNA erfolgt beispielsweise nach an sich bekannten Methoden. So gewinnt man genomische Eglin-DNA beispielsweise aus einer Egel-Genbank, welche das Eglin-Gen enthält, indem man die Egel-DNA-Fragmente in einem Mikroorganismus kloniert und Klone, die Eglin-DNA enthalten, identifiziert, beispielsweise durch Kolonie-Hybridisierung unter Verwendung eines radioaktiv markierten Eglin-DNA spezifischen Oligodesoxynucleotids, welches mindestens 15, vorzugsweise 15 bis 30, Desoxynucleotide enthält. Die so erhaltenen DNA-Fragmente enthalten neben dem Eglin-Gen in der Regel weitere unerwünschte DNA-Bestandteile, welche durch Behandlung mit geeigneten Exo- oder Endonucleasen abgespalten werden können.

Doppelsträngige Eglin-cDNA kann beispielsweise hergestellt werden, indem man aus geeigneten, vorzugsweise zur Eglin-Bildung induzierten Egelzellen mRNA gewinnt, das so erhaltene mRNA-Gemisch in an sich bekannter Weise an Eglin-mRNA anreichert, diese mRNA als Template für die Herstellung von einsträngiger cDNA verwendet, daraus mit Hilfe einer RNA-abhängigen DNA-Polymerase die ds cDNA synthetisiert und diese in einen geeigneten Vektor kloniert. Klone, die Eglin-cDNA enthalten, werden beispielsweise, wie oben beschrieben, durch

Koloniehybridisierung unter Verwendung eines radioaktiv markierten, Eglin-DNA spezifischen Oligodesoxynucleotids identifiziert.

Die auf diese Weise gewonnene genomische Eglin-DNA oder die Eglin-cDNA werden vorzugsweise am 5'- und am 3'-Ende mit chemisch synthetisierten Adapter-Oligodesoxynucleotiden verknüpft, welche die Erkennungssequenz für eine oder verschiedene Restriktionsendonuclease(n) enthalten und damit den Einbau in geeignete Vektoren erleichtern. Zusätzlich muss das Adaptermolekül für das 5'-Ende der Eglin-DNA bzw. -cDNA noch das Translationsstartsignal (ATG) enthalten. Das Translationsstartsignal muss derart angeordnet sein, dass ihm das Codon für die erste Aminosäure des Eglins direkt folgt.

Die chemische Synthese eines Eglin-Gens bietet auf Grund der modernen Synthesemöglichkeiten, insbesondere in zeitlicher Hinsicht, Vorteile, da die Struktur des natürlichen Eglin-Gens nicht bekannt ist.

**Chemische Synthese eines Eglin-Gens**

Die Herstellung eines Strukturgens für ein Eglin ist dadurch gekennzeichnet, dass man Segmente des kodierenden und des komplementären Stranges eines Eglin-Gens chemisch synthetisiert und erhältliche Segmente enzymatisch in ein Strukturgen des Eglins überführt.

Die DNAs enthalten zusätzlich zu den Codons für die Egline Translations-Startsignale und Translations-Stopsignale, die eine Expression in *Saccharomyces cerevisiae* oder *E. coli* ermöglichen, ferner Nukleotidsequenzen an den Enden, die für einen Einbau in einen Vektor geeignet sind.

Die DNA besitzt am 5'-Ende eine durch ein Restriktionsenzym schneidbare Nukleotidsequenz gefolgt vom Translations-Startsignal, Codons für ein Eglin, die gegebenenfalls an einer oder mehreren Stellen das Schneiden durch ein Restriktionsenzym ermöglichen, ein Translations-Stopsignal und am 3'-Ende eine durch ein Restriktionsenzym schneidbare Nukleotidsequenz. Anwendbare Restriktionsenzyme sind beispielsweise EcoRI, BamHI, HpaII, PstI, AvaI oder HindIII.

Eine für ein Eglin kodierende, doppelsträngige DNA besteht aus einer Nukleotidsequenz der Formel I und der komplementären Nukleotidsequenz

| | | | | | | | | | | |
|---|---|---|---|---|---|---|---|---|---|---|
| | Me | B | | | | | | | | |
| $^{5'}(X)_n$ | ATG | D | | | | | | | | |
| Pro | Glu | Val | Val | Gly | Lys | Thr | Val | Asp | Gln | |
| CCX | GAM | GTX | GTX | GGX | AAM | ACX | GTX | GAY | CAM | |
| Ala | Arg | Glu | Tyr | Phe | Thr | Leu | His | Tyr | Pro | |
| GCX | LGN | GAM | TAY | TTY | ACX | YTZ | CAY | TAY | CCX | |
| Gln | Tyr | Asp | Val | W | Phe | Leu | Pro | Glu | Gly | '(I), |
| CAM | TAY | GAY | GTX | YAY | TTY | YTZ | CCX | GAM | GGX | |
| Ser | Pro | Val | Thr | Leu | Asp | Leu | Arg | Tyr | Asn | |
| QRS | CCX | GTX | ACX | YTZ | GAY | YTZ | LGN | TAY | AAY | |
| Arg | Val | Arg | Val | Phe | Tyr | Asn | Pro | Gly | Thr | |
| LGN | GTX | LGN | GTX | TTY | TAY | AAY | CCX | GGX | ACX | |
| Asn | Val | Val | Asn | B' | NON | | | | | |
| AAY | GTX | GTX | AAY | D' | TMK | $(X)_m{}^{3}$ | | | | |

worin die Nukleotidsequenz beginnend mit dem 5'-Ende dargestellt und zum besseren Verständnis die Aminosäuren, für die jedes Triplett kodiert, angegeben sind, und worin D für die N-terminale Aminosäuren des Eglins kodierende Nucleotidsequenz (untere Zeile) und B für die entsprechenden N-terminalen Aminosäuren (obere Zeile)

| | | | | | | | | | |
|---|---|---|---|---|---|---|---|---|---|
| Thr | Glu | Phe | Gly | Ser | Glu | Leu | Lys | Ser | Phe |
| ACX | GAM | TTY | GGX | QRS | GAM | YTZ | AAM | QRS | TTY |

steht und D' für die C-terminale Aminosäuren des Eglins kodierende Nucleotidsequenz (untere Zeile) und B' für die entsprechenden C-terminalen Aminosäuren (obere Zeile)

| | | | | | |
|---|---|---|---|---|---|
| His | Val | Pro | His | Val | Gly |
| CAY | GTX | CCX | CAY | GTX | GGX |

steht, und worin bedeuten

| | |
|---|---|
| A | Desoxyadenosyl, |
| T | Thymidyl, |
| G | Desoxyguanosyl, |
| C | Desoxycytidyl, |
| X | A, T, C oder G, |
| Y | T oder C, |
| Z | A, T, C oder G, wenn Y = C, |
| oder | Z = A oder G, wenn Y = T, |
| Q | T oder A, |
| R | C und S = A, T, C oder G, wenn Q = T, |
| oder | R = G und S = T oder C, wenn Q = A, |
| M | A oder G, |
| L | A oder C, |
| N | A oder G, wenn L = A, |
| oder | N = A, T, C oder G, wenn L = C, |
| K | A oder G, wenn M = A, |
| oder | K = A, wenn M = G, |
| W | Tyr oder His, |

und $(X)_n$ und $(X)_m$ je beliebige Nukleotidsequenzen mit n und m grösser als 3 und kleiner als 100, insbesondere grösser als 5 und kleiner als 12, bedeuten, welche durch ein Restriktionsenzym erkannt und geschnitten werden können.

Die DNA-Sequenz enthält am 5'-Ende eine durch EcoRI spaltbare, in der Mitte eine durch HpaII spaltbare und am 3'-Ende eine durch BamHI spaltbare Nukleotidsequenz.

Die doppelsträngige DNA enthält von *E. coli* bevorzugte Tripletts, die für die Aminosäuren von Eglinen kodieren. Solche Tripletts sind für

| | |
|---|---|
| Glycin (Gly) | GGT |
| Alanin (Ala) | GCT |
| Valin (Val) | GTT |
| Leucin (Leu) | CTG |
| Serin (Ser) | TCT |
| Threonin (Thr) | ACT |
| Phenylalanin (Phe) | TTC |
| Tyrosin (Tyr) | TAC |
| Methionin (Met) | ATG |
| Asparaginsäure (Asp) | GAC |
| Glutaminsäure (Glu) | GAA |
| Lysin (Lys) | AAA |
| Arginin (Arg) | CGT |
| Histidin (His) | CAT |
| Prolin (Pro) | CCG |
| Glutamin (Gln) | CAG |
| Asparagin (Asn) | AAC |

Für Phenylalanin wird auch das Codon TTT und für Prolin CCA oder CCT verwendet, damit neben der Schnittstelle für EcoRI am 5'-Ende, für BamHI am 3'-Ende und einer Schnittstelle für HpaII keine weiteren Schnittstellen für die genannten Restriktionsenzyme enthalten sind. Das Stopsignal (NON) ist das Codon TAG.

Ein Gen für Eglin C in der oben dargestellten Weise ist die DNA der Formel IIa,

```
MetThrGluPheGlySerGluLeuLysSerPheProGluValValGlyLysThrVal
CTGGAATTCATGACTGAATTTGGTTCTGAACTGAAATCTTTCCCAGAAGTTGTTGGTAAAACTGTT
GACCTTAAGTACTGACTTAAACCAAGACTTGACTTTAGAAAGGGTCTTCAACAACCATTTTGACAA
(EcoRI)

AspGlnAlaArgGluTyrPheThrLeuHisTyrProGlnTyrAspValTyrPheLeuProGluGly
GACCAGGCTCGTGAATACTTCACTCTGCATTACCCGCAGTACGACGTTTACTTCCTGCCGGAAGGT
CTGGTCCGAGCACTTATGAAGTGAGACGTAATGGGCGTCATGCTGCAAATGAAGGACGGCCTTCCA
(HpaII)

SerProValThrLeuAspLeuArgTyrAsnArgValArgValPheTyrAsnProGlyThrAsnVal
TCTCCTGTTACTCTGGACCTGCGTTACAACCGTGTTCGTGTTTTCTACAACCCAGGTACTAACGTT
AGAGGACAATGAGACCTGGACGCAATGTTGGCACAAGCACAAAAGATGTTGGGTCCATGATTGCAA
ValAsnHisValProHisValGlyNON
GTTAACCATGTTCCGCATGTTGGTTAGGATCCTG
```

CAATTGGTACAAGGCGTACAACCAATCCTAGGAC (II),
(BamHI)

ein Gen für Eglin B ist die DNA der Formel IIb,

MetThrGluPheGlySerGluLeuLysSerPheProGluValValGlyLysThrVal
CTGGAATTCATGACTGAATTTGGTTCTGAACTGAAATCTTTCCCAGAAGTTGTTGGTAAAACTGTT
GACCTTAAGTACTGACTTAAACCAAGACTTGACTTTAGAAAGGGTCTTCAACAACCATTTTGACAA
(EcoRI)

AspGlnAlaArgGluTyrPheThrLeuHisTyrProGlnTyrAspValHisPheLeuProGluGly
GACCAGGCTCGTGAATACTTCACTCTGCATTACCCGCAGTACGACGTTCATTTCCTGCCGGAAGGT
CTGGTCCGAGCACTTATGAAGTGAGACGTAATGGGCGTCATGCTGCAAGTAAAGGACGGCCTTCCA
(HpaII)

SerProValThrLeuAspLeuArgTyrAsnArgValArgValPheTyrAsnProGlyThrAsnVal
TCTCCTGTTACTCTGGACCTGCGTTACAACCGTGTTCGTGTTTTCTACAACCCAGGTACTAACGTT
AGAGGACAATGAGACCTGGACGCAATGTTGGCACAAGCACAAAAGATGTTGGGTCCATGATTGCAA

ValAsnHisValProHisValGlyNON
GTTAACCATGTTCCGCATGTTGGTTAGGATCCTG (IIb),
CAATTGGTACAAGGCGTACAACCAATCCTAGGAC
(BamHI)

worin A, T, G, C die unter Formel I angegebenen Bedeutungen haben und zum besseren Verständnis die Aminosäuren, für die jedes Triplett kodiert, und die Schnittstellen für die Restriktionsenzyme angegeben sind.

Doppelsträngige DNA-Fragmente von Eglin-Genen können an ihren Enden durch Restriktionsenzyme geschnitten und zu vollständigen Eglin-Genen zusammengesetzt werden. Solche doppelsträngigen DNA-Fragmente von Eglin-Genen haben 30 bis 70 Basenpaare und besitzen beispielsweise die Formel IIIa [$F_1$(C)], die Formel IIIb [$F_1$(B)] und die Formel IV ($F_2$):

MetThrGluPheGlySerGluLeuLysSerPheProGluValValGlyLysThrVal
CTGGAATTCATGACTGAATTTGGTTCTGAACTGAAATCTTTCCCAGAAGTTGTTGGTAAAACTGTT
GACCTTAAGTACTGACTTAAACCAAGACTTGACTTTAGAAAGGGTCTTCAACAACCATTTTGACAA

AspGlnAlaArgGluTyrPheThrLeuHisTyrProGlnTyrAspValTyrPheLeuPro
GACCAGGCTCGTGAATACTTCACTCTGCATTACCCGCAGTACGACGTTTACTTCCTGCCGG
CTGGTCCGAGCACTTATGAAGTGAGACGTAATGGGCGTCATGCTGCAAATGAAGGACGGCC (IIIa)
$F_1$ (C)

MetThrGluPheGlySerGluLeuLysSerPheProGluValValGlyLysThrVal
CTGGAATTCATGACTGAATTTGGTTCTGAACTGAAATCTTTCCCAGAAGTTGTTGGTAAAACTGTT
GACCTTAAGTACTGACTTAAACCAAGACTTGACTTTAGAAAGGGTCTTCAACAACCATTTTGACAA

AspGlnAlaArgGluTyrPheThrLeuHisTyrProGlnTyrAspValHisPheLeuPro
GACCAGGCTCGTGAATACTTCACTCTGCATTACCCGCAGTACGACGTTCATTTCCTGCCGG
CTGGTCCGAGCACTTATGAAGTGAGACGTAATGGGCGTCATGCTGCAAGTAAAGGACGGCC (IIIb)
$F_1$ (B)

ProGluGlySerProValThrLeuAspLeuArgTyrAsnArgValArgValPheTyrAsnProGly
CCGGAAGGTTCTCCTGTTACTCTGGACCTGCGTTACAACCGTGTTCGTGTTTTCTACAACCCAGGT
GGCCTTCCAAGAGGACAATGAGACCTGGACGCAATGTTGGCACAAGCACAAAAGATGTTGGGTCCA

ThrAsnValValAsnHisValProHisValGlyNON
ACTAACGTTGTTAACCATGTTCCGCATGTTGGTTAGGATCCTG (IV)
TGATTGCAACAATTGGTACAAGGCGTACAACCAATCCTAGGAC
$F_2$

Auch einsträngige DNA-Fragmente von Eglin-Genen lassen sich durch chemische und/oder enzymatische Methoden zu Eglin-Genen zusammenfügen. Solche einsträngigen DNA-Fragmente haben 20 bis 70 Nukleotide.

Methoden der Synthese von DNA sind von S.A. Narang (11) zusammenfassend dargestellt worden. Die bekannten Synthesetechniken erlauben die Herstellung von Polynukleotiden mit einer Länge von gegen 20 Basen in guter Ausbeute, hoher Reinheit und in verhältnismässig kurzer Zeit. Geeignet geschützte Nukleotide werden durch die Phosphodiestermethode (12), die noch effizientere Phosphotriestermethode (13) oder Phosphit-Triestermethode (14) miteinander verknüpft. Eine Vereinfachung der Synthese der Oligo- und Polynukleotide wird mit der

Festphasen-Methode ermöglicht, bei der die Nukleotidketten an ein geeignetes Polymer gebunden sind. Itakura et al. (15) verwenden bei der Festphasen-Synthese anstelle einzelner Nukleotide durch Phosphotriester-Methode geknüpfte Trinukleotide, die so in kurzer Zeit und guten Ausbeuten beispielsweise zu einem Polynukleotid mit 31 Basen kondensiert werden können. Die eigentliche doppelsträngige DNA kann aus chemisch hergestellten kurzen Segmenten enzymatisch aufgebaut werden. Khorana et al. (16) verwenden dazu überlappende Polynukleotid-Sequenzen aus beiden DNA-Strängen, die durch Basen-Paarung in richtiger Anordnung zusammengehalten und dann durch das Enzym DNA-Ligase chemisch verknüpft werden. Eine weitere Möglichkeit besteht darin, je eine Polynukleotid-Sequenz aus den beiden DNA-Strängen mit einem kurzen überlappenden Segment in Gegenwart der vier benötigten Desoxynukleosid-Triphosphate mit einer DNA-Polymerase, z. B. DNA-Polymerase I, Klenow-Fragment der Polymerase I, $T_4$ DNA-Polymerase, oder mit AMV (avian myeloblastosis virus) reverse Transcriptase, zu inkubieren. Dabei werden durch Basenpaarung die beiden Polynukleotid-Sequenzen in der richtigen Anordnung zusammengehalten und durch das Enzym mit den benötigten Nukleotiden zu einer vollständigen doppelsträngigen DNA ergänzt (17). Itakura et al. (18) beschreiben, wie basierend auf diesem Prinzip in Gegenwart von DNA-Polymerase I (Klenow-Fragment) aus 4 chemisch synthetisierten Fragmenten einer Länge von 39 bis 42 Basen ein 132 Basen-Paare langes Segment des humanen Leukozyten-Interferon $\alpha_2$-Gens aufgebaut werden kann, wobei 40 % Einsparung an chemischer Synthese gegenüber der Methode, die nur Ligase verwendet, erzielt werden.

Ein Verfahren zur Herstellung von DNAs, welche für Egline kodieren, die für eine Expression in Wirtszellen geeignet sind und deren Enden einen Einbau in Vektoren ermöglichen, ist dadurch gekennzeichnet, dass

a) ein geeignet geschütztes Desoxynukleosid an einen festen Träger gebunden wird,
b) geeignet geschützte Di-, Tri- oder Tetranukleotide nach der Phosphotriester- oder Phosphit-Methode hergestellt werden,
c) ein an den Träger gebundenes Desoxynukleosid oder Oligodesoxynukleotid mit geeignet geschützten Mono- oder (gemäss b) hergestellten) Di-, Tri- oder Tetranukleotiden nach der Phosphotriester- oder der Phosphit-Methode verknüpft wird,
d) nach c) erhältliche, an Träger gebundene Oligodesoxynukleotide mit einer Länge zwischen etwa 20 und etwa 70 Basen vom Träger abgespalten, gegebenenfalls gereinigt, von Schutzgruppen befreit und die freien 5'-terminalen Hydroxygruppen phosphoryliert werden,
e1) 2 Oligodesoxynukleotide einer Länge von je etwa 20 bis etwa 70 Basen aus dem kodierenden und aus dem komplementären Strang und mit mindestens 3, vorzugsweise 8 bis 15, überlappenden Basenpaaren annelliert und mit einer DNA-Polymerase in Gegenwart der vier Desoxynukleosid-Triphosphate zu doppelsträngigen DNA-Segmenten (Fragmenten des Eglin- oder modifizierten Eglin-Gens) ergänzt werden,
und gegebenenfalls 2 doppelsträngige DNA-Segmente mit geeigneten gemäss d) phosphorylierten Enden mit einer Ligase zum Strukturgen des Eglins oder des modifizierten Eglins verknüpft werden, oder 2 erhältliche doppelsträngige DNA-Segmente in geeignete Vektoren subkloniert, anschliessend gemäss d) phosphoryliert und mit einer Ligase zum Strukturgen des Eglins oder modifizierten Eglins verknüpft werden, oder
e2) alternativ je 2 Oligodesoxynukleotide aus dem kodierenden und aus dem komplementären Strang mit einer Länge von z. B. 20 bis 70 Basen und mit je mindestens 3, vorzugsweise 8 bis 15, überlappenden Basenpaaren annelliert, mit einer DNA-Polymerase in Gegenwart der vier Desoxynukleosid-Triphosphate aufgefüllt und mit Ligase zum Strukturgen des Eglins oder des modifizierten Eglins verknüpft werden.

In Schritt a) können eine Vielzahl von festen Trägermaterialien, wie Polystyrol verschiedener Vernetzung und Quellfähigkeit, Polyacrylamide, Polyacrylamid-Copolymere, auf anorganisches Material wie Kieselguhr, Kieselgel oder Alox aufgezogene Polyamide oder funktionalisierte Silane, verwendet werden. Bevorzugt werden als feste Trägermaterialien quervernetzte Polystyrole eingesetzt, die über "Spacer", wie Alkylen-Gruppen mit 2 bis 12 C-Atomen unterbrochen von 1 bis 5 polaren zweiwertigen funktionellen Gruppen, wie Imino, Oxo, Thio, Oxocarbonyl oder Amidocarbonyl, auf an sich bekannte Weise mit der 5'-OH-Gruppe geeignet geschützter Desoxynukleoside verknüpft werden. Besonders bevorzugt ist die Reaktion von in 5'-Stellung und gegebenenfalls im Basenteil geschützten Nukleosiden der Formel V, worin $R^1$ eine durch Säure abspaltbare Schutzgruppe, wie eine Triarylmethylschutzgruppe, beispielsweise eine 4-Methoxytrityl- oder eine 4,4'-Dimethoxytritylgruppe, oder eine Triniederalkylsilylschutzgruppe, beispielsweise eine tert.-Butyldimethylsilylgruppe, und worin B eine gegebenenfalls geschützte Base ausgewählt aus Thymyl, Cytosyl, Adenyl oder Guanyl bedeutet, mit Bernsteinsäureanhydrid, gegebenenfalls in Gegenwart von Basen, wie Pyridin, Triethylamin oder Dimethylaminopyridin, gefolgt von der Reaktion mit aminomethyliertem Polystyrol, das durch 0,5 bis 2 % Divinylbenzol quervernetzt ist, mit Hilfe von den Carbonsäure-Rest aktivierenden Reagentien, vorzugsweise N-Hydroxysuccinimid, oder p-Nitrophenol und wasserentziehenden Mitteln, wie Carbodiimiden, beispielsweise Dicyclohexylcarbodimid (Schema 1).

Die Umsetzung erfolgt in einem inerten nicht-protischen Lösungsmittel, z. B. Pyridin, Tetrahydrofuran, Dioxan, Essigsäureethylester, Chloroform, Methylenchlorid, Dimethylformamid oder Diethylacetamid oder in Mischungen davon, bei Raumtemperatur oder leicht erhöhter oder erniedrigter Temperatur, z. B. in einem Temperaturbereich von ca. −10°C bis ca. 50°C, bevorzugt bei Raumtemperatur, die Umsetzung in Gegenwart des wasserentziehenden Mittels auch bei niedrigerer Temperatur, z. B. bei ca. 0°C.

**Schema 1**

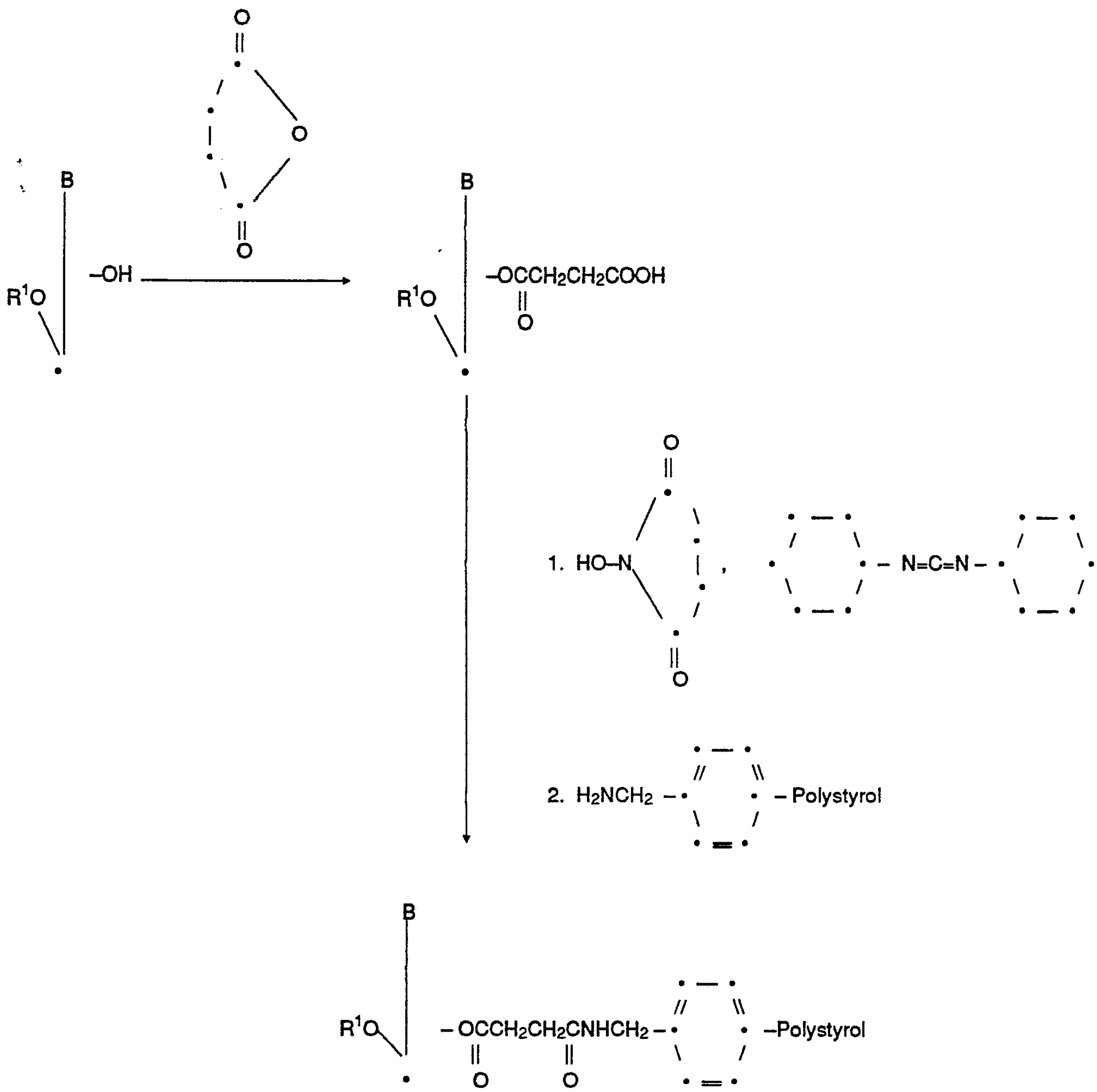


Bei der Herstellung von Di-, Tri- oder Tetranukleotiden gemäss Schritt b) werden in 5'-Stellung und gegebenenfalls im Basenteil geschützte Nukleoside der Formel V, worin $R^1$ und B die oben angegebenen Bedeutungen haben, mit aktivierten Phosphorestern der Formel VII, worin $X^1$ und $X^2$ unabhängig voneinander Hydroxy oder davon abgeleitete Salze, Halogen, Imidazolyl, 1,2,4-Triazol-1-yl, Tetrazolyl oder 1-Benztriazolyloxy und $X^2$ zusätzlich auch 2-Cyanoethyloxy, 2-Trihalogenethyloxy, 2-Arylsulfonylethyloxy, 2-Niederalkylthioethyloxy, 2-Arylthioethyloxy oder 2-(4-Nitrophenyl)-ethyloxy und $R^2$ eine durch Base oder Nukleophile, wie Ammoniumhydroxid, Thiophenolat oder ein Arylaldoximat, abspaltbare Schutzgruppe, wie gegebenenfalls durch Halogen, Nitro und/oder Niederalkyl substituiertes Phenyl, Methyl oder gegebenenfalls durch Nitro substituiertes Benzyl, oder eine durch Metallionen abspaltbare Schutzgruppe, wie 8-Chinolyl oder 5-Chloro-8-chinolyl, bedeutet, gegebenenfalls in Gegenwart von wasserentziehenden Mitteln oder in Gegenwart von Basen umsetzt.

Anschliessend wird eine dermassen gebildete Verbindung der Formel VIII, worin $R^1$, $X^2$ und $R^2$ die oben angegebenen Bedeutungen haben, zuerst gegebenenfalls mit einem 2-substituierten Ethanol umgesetzt, das den Rest $X^2$ in eine Gruppe $OR^3$ umwandelt, worin $R^3$ Cyanoethyl, 2-Trihalogenethyl, 2-Arylsulfonylethyl, 2-Niederalkylthioethyl, 2-Arylthioethyl oder 2-(4-Nitrophenyl)-ethyl bedeutet, und dann die Schutzgruppe $R^1$ abgespalten, und die dermassen hergestellte Verbindung der Formel IX mit einer andern Verbindung der Formel VIII, gegebenenfalls in Gegenwart von wasserentziehenden Mitteln oder in Gegenwart von Basen, zu einem Dinukleotid X umgesetzt (Schema 2). Gegebenenfalls wird eine Verbindung der Formel VIII durch Reaktion mit Basen und Wasser in eine andere Verbindung der Formel VIII, worin $X^2$ Hydroxy oder davon abgeleitete Salze bedeutet, umgewandelt.

Die Umsetzungen erfolgen in einem der oben genannten inerten Lösungsmittel bei Raumtemperatur oder leicht erhöhter oder erniedrigter Temperatur, z. B. bei Raumtemperatur.

Die Abspaltung der Schutzgruppe $R^1$ wird z. B. mit Hilfe von Säuren, wie Mineralsäuren, z. B. Salzsäure oder Schwefelsäure, Carbonsäuren, z. B. Essigsäure, Trichloressigsäure oder Ameisensäure, Sulfonsäuren, z. B. Methan- oder p-Toluolsulfonsäure, oder insbesondere Lewis-Säuren, z. B. Zinkchlorid, Zinkbromid, Aluminiumchlorid, Dialkylaluminiumhalogeniden, z. B. Dibutyl- oder Diethylaluminiumchlorid, oder Bortrifluorid, bei 10°C bis 50°C, insbesondere bei Raumtemperatur, durchgeführt. Bei Verwendung eines Dialkylaluminiumhalogenid erfolgt die Abspaltung in einem lipophilen Lösungsmittel, insbesondere in Toluol, und bei Verwendung einer anderen der genannten Lewis-Säuren in einem Lösungsmittelgemisch, bestehend aus einem Halogenkohlenwasserstoff, z. B. Methylenchlorid, und einem Niederalkanol, z. B. Ethanol oder Isopropanol.

**Schema 2**

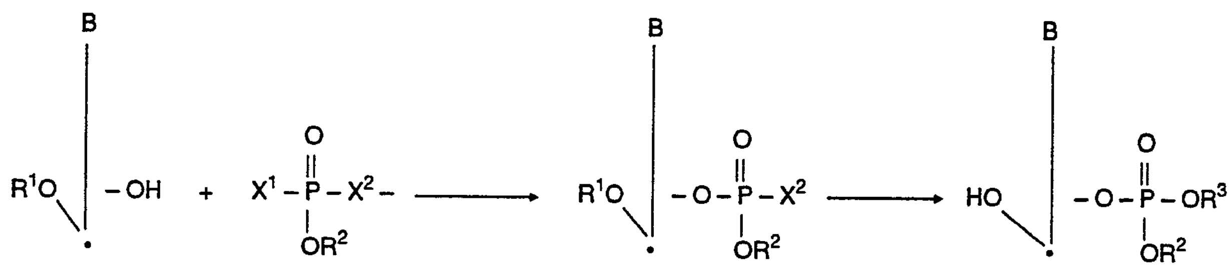

V VII VIII IX

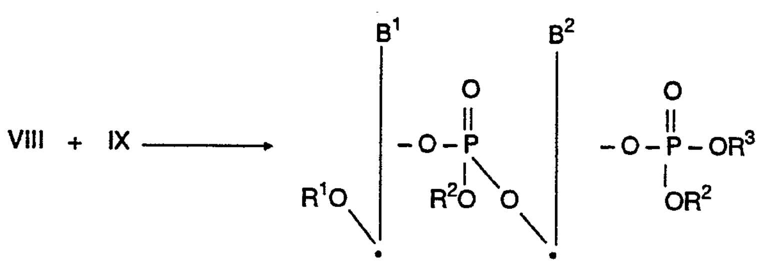

X

Die Herstellung von Dinukleotiden der Formel X umfasst auch die Reaktion von Nukleosiden der Formel V, worin $R^1$ und B die oben angegebenen Bedeutungen haben, mit Phosphiten der Formel VIIA, worin $X^1$ Halogen, insbesondere Chlor, $X^2$ Halogen, insbesondere Chlor, oder Diniederalkylamino, insbesondere Dimethylamino oder Diisopropylamino, oder Morpholino, Piperidino oder Pyrrolidino, bedeuten und $R^2$ die für VII oben angegebene Bedeutung, insbesondere Methyl, hat, gegebenenfalls in Gegenwart einer geeigneten Base. Die erhältlichen Verbindungen der Formel VIIIA werden einerseits mit einem 2-substituierten Ethanol umgesetzt, das den Rest $X^2$ in eine Gruppe $OR^3$ umwandelt, worin $R^3$ die oben angeführten Bedeutungen hat, dann mit einem Oxidationsmittel, beispielsweise Jod in Gegenwart einer Base, zum Phosphat oxidiert und die Schutzgruppe $R^1$ abgespalten, wobei eine Verbindung der Formel IX entsteht, andererseits mit einer Verbindung der Formel IX umgesetzt, dann mit einem Oxidationsmittel, beispielsweise Jod in Gegenwart einer Base, zu einer Verbindung der Formel X oxidiert (Schema 3).

Schema 3

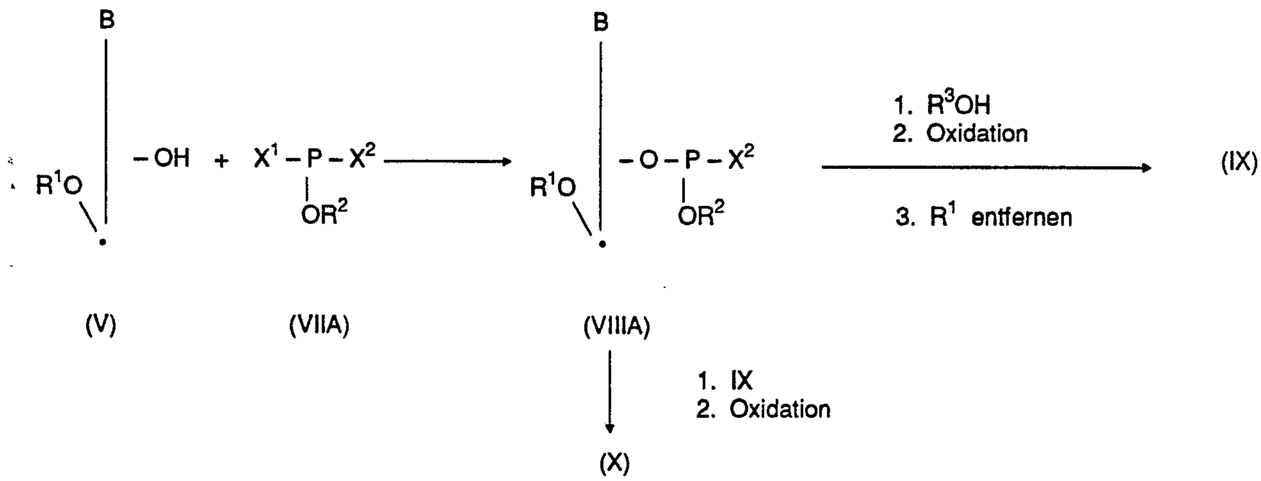


Zur Herstellung von Trinukleotiden wird in Dinukleotiden der Formel X, worin $R^1$, $R^2$ und $R^3$ die oben angegebenen Bedeutungen haben und worin $B^1$ und $B^2$ unabhängig voneinander Thymyl, Cytosyl, Adenyl oder Guanyl bedeuten, die Schutzgruppe $R^1$ abgespalten und die erhaltene Verbindung mit einer Verbindung der Formel VIII, gegebenenfalls in Gegenwart von wasserentziehenden Mitteln oder in Gegenwart von Basen, oder mit einer Verbindung der Formel VIIIA und nachfolgender Oxidation umgesetzt, wobei eine Verbindung der Formel XI entsteht (Schema 4). Die Abspaltung der Schutzgruppe $R^1$ und die Kondensation zu den Trinukleotiden der Formel XI erfolgt in gleicher Weise, wie bei der Herstellung der Dinukleotide der Formel X beschrieben.

Schema 4

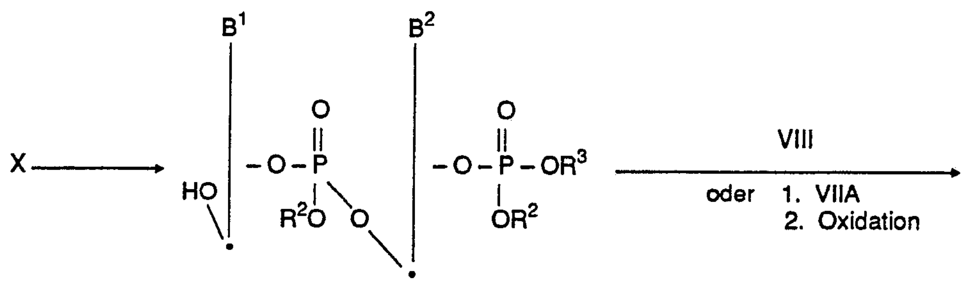


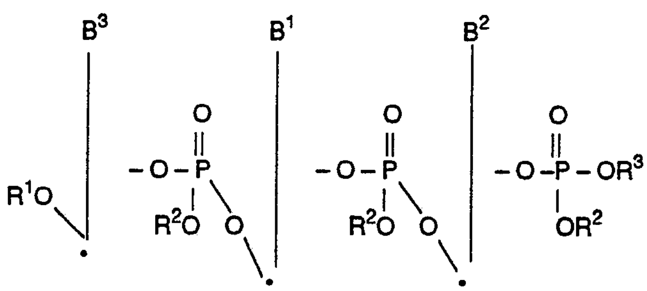

(XI)

Zur Herstellung von Tetranukleotiden werden Trinukleotide der Formel XI so zur Reaktion gebracht, wie oben für Dinukleotide der Formel X beschrieben.

Bevorzugt wird als Schutzgruppe $R^1$ die 4-Methoxytritylgruppe, als Schutzgruppe $R^2$ eine durch Chlor substituierte Phenylgruppe, insbesondere 2-Chlorphenyl, und als Schutzgruppe $R^3$ die 2-Cyanoethylgruppe verwendet. Bevorzugter Rest $X^1$ und $X^2$ in der Verbindung der Formel VII ist der 1-Benztriazolyloxy-Rest.

Trinukleotide der Formel XI werden vorzugsweise hergestellt, indem in Dinukleotiden der Formel X die Schutzgruppe $R^1$ abgespalten und die erhaltene Verbindung mit Verbindungen der Formel VIII, worin $X^2$ Hydrox-

yl oder davon abgeleitete Salze bedeutet, in Gegenwart eines wasserentziehenden Mittels umgesetzt wird (Schema 4). Wasserentziehende Mittel sind beispielsweise 2,4,6-Trimethyl- oder Triisopropylbenzolsulfonyl-chlorid, -imidazolid, -tetrazolid oder -1,2,4-triazolid gegebenenfalls substituiert durch Nitro. Bevorzugtes wasserentziehendes Mittel ist 2,4,6-Trimethylbenzolsulfonyl-3-nitro-1,2,4-triazolid der Formel XII

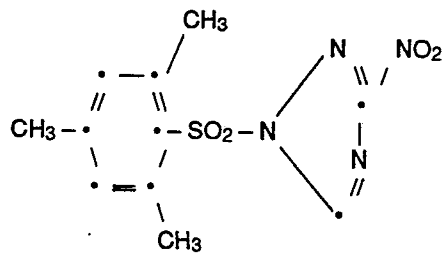

(XII)

Vorzugsweise werden Nukleoside eingesetzt, deren freie Aminogruppe im Basenteil geschützt ist. Bevorzugte Schutzgruppen sind Benzoyl für Adenin, Benzoyl oder 4-Methoxybenzoyl für Cytosin, Isobutyryl oder Diphenylacetyl für Guanin. Thymin wird vorzugsweise ohne Schutzgruppe eingesetzt.

Bei der Herstellung von Oligonukleotiden gemäss Schritt c) wird eine an sich bekannte Apparatur mit halbautomatischem oder vollautomatischem, mikroprozessorgesteuertem Zuführungssystem für Lösungsmittel und Reagentien verwendet. In einer gemäss Schritt a) hergestellten Verbindung der Formel VI wird die Schutzgruppe $R^1$, wie oben beschrieben, abgespalten und anschliessend entweder mit einer Verbindung der Formel VIII, oder mit einer Verbindung der Formel VIIIA, oder mit einer Verbindung der Formel X oder XI, in der vorgängig die Schutzgruppe $R^3$ mit Basen abgespalten worden ist (eine Gruppe 2-Cyanoethyl als $R_3$ beispielsweise mit einem Triniederalkylamin, z. B. Triethylamin, in einem der oben genannten inerten Lösungsmittel oder Lösungsmittelgemische bei 10°C bis 40°C, insbesondere bei Raumtemperatur), gegebenenfalls in Gegenwart eines wasserentziehenden Mittels oder in Gegenwart einer Base umgesetzt. Anstelle eines Dinukleotids der Formel X oder eines Trinukleotids der Formel XI kann auch ein gemäss Schritt b) hergestelltes Tetranukleotid eingesetzt werden. Wird ein Phosphit der Formel VIIIA verwendet, so wird anschliessend mit einem Oxidationsmittel, beispielsweise Jod in Gegenwart einer Base, nachbehandelt. Die dermassen hergestellte Verbindung der Formel XIII, worin $R^1$, $R^2$ und B die oben angegebenen Bedeutungen haben und n eine ganze Zahl von 1 bis 4 ist, wird so oft den für die Verbindung der Formel VI beschriebenen Reaktionsschritten (Abspaltung von $R^1$, Reaktion mit VIII, VIIIA, X, XI oder dem entsprechenden Tetranukleotid, gegebenenfalls mit oxidativer Nachbehandlung) unterworfen, bis eine Verbindung der Formel XIII entsteht, in der n eine beliebige ausgewählte Zahl zwischen etwa 19 und etwa 69 ist.

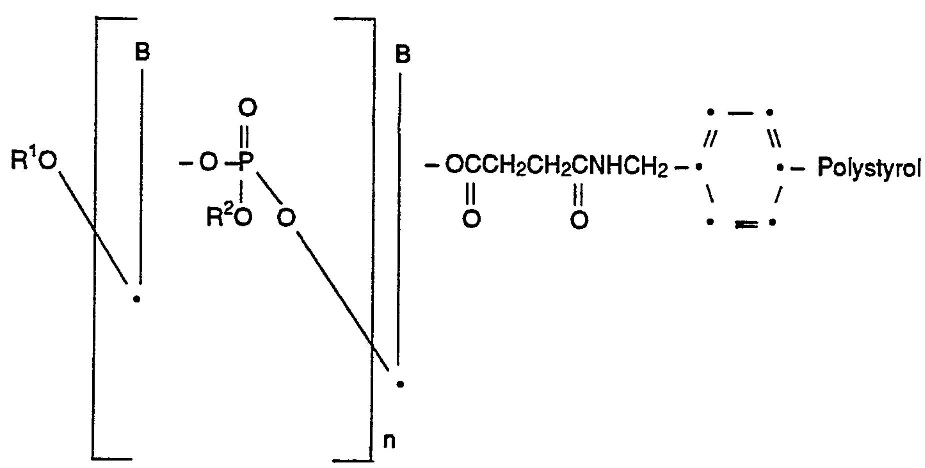

Bevorzugt wird als Schutzgruppe $R^1$ 4-Methoxytrityl verwendet und die Abspaltung mit Zinkbromid in Gegenwart einer CH- oder NH-aciden Verbindung, insbesondere 1,2,4-Triazol oder Tetrazol, durchgeführt. Die Verwendung von z. B. 1,2,4-Triazol bei der Abspaltung der 4-Methoxytrityl-Schutzgruppe ist neu und führt überraschenderweise dazu, dass die Abspaltung schnell, mit hohen Ausbeuten und ohne Nebenreaktionen abläuft. Besonders bevorzugt ist die Verwendung von Zinkbromid und 1,2,4-Triazol in einem molaren Verhältnis zwischen 20 : 1 und 100 : 1 in einem Lösungsmittelgemisch bestehend aus einem aprotischen Lösungsmittel und einem Alkohol, beispielsweise Methylenchlorid und 2-Propanol.

Eine Verbindung der Formel VI oder der Formel XIII, in der die Schutzgruppe abgespalten worden ist, wird mit einem Trinukleotid der Formel XI, in dem die Schutzgruppe $R^3$ abgespalten worden ist, umgesetzt in Gegenwart eines wasserentziehenden Mittels, wie beispielsweise 2,4,6-Trimethyl- oder Triisopropylbenzolsulfonyl-chlorid, -imidazolid, -tetrazolid oder -1,2,4-triazolid gegebenenfalls substituiert durch Nitro. Besonders bevorzugt ist 2,4,6-Trimethylbenzolsulfonyl-3-nitro-1,2,4-triazolid der Formel XII.

Die besonders bevorzugte Kombination, darin bestehend, dass als Schutzgruppe $R^1$ die 4-Methoxytritylgruppe verwendet, zur Abspaltung von $R^1$ Zinkbromid in Gegenwart von 1,2,4-Triazol eingesetzt und als wasserentziehendes Mittel für die Reaktion von entschütztem Oligonukleotid-Polystyrol-Harz der Formel XIII mit einem entschütztem Trinukleotid der Formel XI das Triazolid der Formel XII verwendet wird, ermöglicht es, dass überraschenderweise auch lange Nukleotidketten mit etwa 40 bis etwa 70 Basen in kurzer Zeit, in hohen Ausbeuten und mit hoher Reinheit hergestellt werden können.

Zur Abspaltung der Oligodesoxynukleotide vom Träger und zur Entfernung der Schutzgruppen gemäss Schritt d) werden an sich bekannte Verfahren verwendet. Besonders bevorzugtes Reagens zur Abspaltung vom Träger und zur Entfernung der bevorzugten 2-Chlorphenyl-Schutzgruppe ist ein Arylaldoximat, beispielsweise das 1,1,3,3-Tetramethylguanidinium-2-nitrobenzaldoximat. Die Reaktion erfolgt in einem der oben genannten inerten Lösungsmittel, dem etwas Wasser beigefügt wird, z. B. in 95 %-igem Pyridin, bei Raumtemperatur. Anschliessend wird mit wässrigem Ammoniak bei Raumtemperatur oder erhöhter Temperatur, z. B. bei 20°C bis 70°C, insbesondere bei 50°C, umgesetzt.

Zur Ligation der Oligodesoxynukleotide wird an der 5'-terminalen Hydroxygruppe ein Phosphatrest eingeführt. Die Einführung des Phosphatrestes (Phosphorylierung) erfolgt in an sich bekannter Weise mit Hilfe von $T_4$ Polynucleotid-Kinase in Gegenwart von ATP.

Die hergestellten Oligodesoxynukleotide aus dem kodierenden und aus dem komplementären DNA-Strang enthalten überlappende Sequenzen, bestehend aus mindestens 3, vorzugsweise 8 bis 15 überlappende Basenpaaren. Solche Oligodesoxynukleotid-Paare werden beim Mischen durch Wasserstoffbrückenbindung zusammengehalten. Die überhängenden, einsträngigen Enden dienen gemäss Schritt e1) und e2) als Matrix (Template) für den Aufbau des zweiten (komplementären) Stranges durch eine DNA-Polymerase, z. B. DNA-Polymerase I, Klenow-Fragment der DNA-Polymerase I oder $T_4$ DNA-Polymerase oder mit AMV reverse Transcriptase, in Gegenwart der vier Desoxynukleosid-Triphosphate (dATP, dCTP, dGTP und TTP). Die bei der Komplementierung entstehenden Duplex-DNAs, bei denen es sich insbesondere um Fragmente des Eglin-Gens (Verfahren e1) oder um das komplette Eglin-Gen (Verfahren e2) handelt, besitzen flache Enden.

Die gemäss Verfahrensschritt e1) erhältlichen Fragmente des Eglin-Gens enthalten an den Enden Nukleotidsequenzen, die von Restriktionsendonucleasen erkannt und gespalten werden können. Je nach Wahl der Nukleotidsequenzen und dementsprechend der Restriktionsendonucleasen entstehen bei der Spaltung vollständig basengepaarte (flache) Enden ("blunt ends") oder Enden mit einem überhängenden DNA-Strang ("staggered ends"). Die Restriktions-Erkennungssequenzen werden so gewählt, dass die Ligation der DNA-Fragmente, die mit einer flache Enden bildenden Restriktionsendonuclease behandelt worden sind, bzw. die Basenpaarung der kohäsiven Enden und die anschliessende Ligation von DNA-Fragmenten mit überhängenden DNA-Strängen das vollständige Eglin-Strukturgen ergibt. Die Ligierung zweier doppelsträngiger DNA-Fragmente erfordert eine 5'-terminale Phosphatgruppe am Donor-Fragment und eine freie 3'-terminale Hydroxygruppe am Akzeptor-Fragment. Die anfallenden DNA-Fragmente sind bereits 5'-terminal phosphoryliert und werden in an sich bekannter Weise mit einer Ligase, insbesondere $T_4$ DNA-Ligase, verknüpft.

Bevorzugt werden auf dem beschriebenen Wege zwei Fragmente des Eglin C- oder B-Gens, im Falle des Eglin C-Gens die Fragmente $F_1$ (C) und $F_2$ gemäss Formel IIIa bzw. IV, und im Falle des Eglin B-Gens die Fragmente $F_1$ (B) und $F_2$ gemäss Formel IIIb bzw. IV, hergestellt. Die Fragmente, welche erforderlichenfalls in einem geeigneten Vektor subkloniert werden können, enthalten an den Verknüpfungsenden jeweils die Erkennungssequenz für eine Restriktionsendonuklease, insbesondere HpaII, weswegen nach Spaltung mit besagtem Restriktionsenzym und Ligation der beiden Fragmente die korrekt kodierende Eglin-DNA-Sequenz entsteht. Zusätzlich enthalten das Teilstück 1 vor dem Translationsstartsignal (ATG) und das Teilstück 2 nach dem Translationsstopsignal (z. B. TAG) zusätzlich "terminale" Restriktionsstellen, die den Einbau des Eglin-Gens bzw. der Eglin-Gen-Teilstücke in einen geeigneten Vektor erlauben.

In einer anderen Ausführungsform (Schritt e2) werden je zwei Oligodesoxynukleotide, die alternativ aus dem kodierenden und aus dem komplementären Strang stammen, mittels mindestens 3, vorzugsweise 8 bis 15 komplementären Basen anneliert, mit einer DNA-Polymerase, z. B. eine der oben genannten, aufgefüllt und mit $T_4$ DNA-Ligase zum Eglin-Strukturgen ligiert.

**Herstellung von Expressionsvektoren, welche ein Eglin-Gen enthalten**

Die Expressionsvektoren enthalten eine für ein Eglin kodierende DNA-Sequenz, die von einer Expressionskontrollsequenz derart reguliert wird, dass in einem mit diesen Expressionsvektoren transformierten Wirt Polypeptide mit Eglin-Aktivität exprimiert werden.

Die Expressionsvektoren, die eine für Eglin B oder Eglin C kodierende Sequenz enthalten, werden hergestellt, indem man in eine Vektor-DNA, welche eine Expressionskontrollsequenz enthält, eine für ein Eglin kodierende DNA-Sequenz derart einfügt, dass die Expressionskontrollsequenz besagte DNA-Sequenz reguliert.

Die Wahl eines geeigneten Vektors ergibt sich aus den zur Transformation vorgesehenen Wirten *Saccharomyces cerevisiae* und *E. coli*, insbesondere solchen Stämmen, die über kein Restriktions- oder Modifikationsenzym verfügen, wie z. B. *E. coli* X1776, *E. coli* HB101, *E. coli* W3110, *E. coli* HB101/LM1035, *E. coli* JA221(37)

oder *E. coli* K12 Stamm 294.

Grundsätzlich sind alle Vektoren geeignet, welche die für Eglin B oder C kodierenden DNA-Sequenzen in dem gewählten Wirt replizieren und exprimieren.

Beispiele für Vektoren, die zur Expression eines Eglin-Gens in einem *E. coli*-Stamm geeignet sind, sind Bacteriophagen, z. B. Derivate des Bacteriophagen λ, oder Plasmide, wie insbesondere das Plasmid colE1 und seine Derivate, z. B. pMB9, pSF2124, pBR317 oder pBR322. Bevorzugte Vektoren leiten sich von Plasmid pBR322 ab. Geeignete Vektoren beinhalten ein vollständiges Replicon und ein Markierungsgen, welches die Selektion und Identifizierung der mit den Expressionsplasmiden transformierten Wirte auf Grund eines phänotypischen Merkmals ermöglicht. Geeignete Markierungsgene verleihen dem Wirt beispielsweise Resistenz gegenüber Schwermetallen, Antibiotika und dergleichen. Weiterhin enthalten geeignete Vektoren ausserhalb der Replicon- und Markierungsgen-Regionen Erkennungssequenzen für Restriktionsendonucleasen, so dass an diesen Stellen das Eglin-Gen und gegebenenfalls die Expressions-Kontrollsequenz eingefügt werden können. Der bevorzugte Vektor, das Plasmid pBR322, enthält ein intaktes Replicon, gegen Tetracyclin und Ampicillin Resistenz verleihende Markierungsgene ($tet^R$ und $amp^R$ und eine Reihe von nur einmal vorkommenden Erkennungssequenzen für Restriktionsendonucleasen, z. B. PstI (schneidet im $amp^R$-Gen, das $tet^R$-Gen bleibt intakt), BamHI, HindIII, SalI (schneiden alle im $tet^R$-Gen, das $amp^R$-Gen bleibt intakt), NruI und EcoRI.

Mehrere Expressionskontrollsequenzen können zur Regulation der Genexpression eingesetzt werden. Insbesondere werden Expressionskontrollsequenzen stark exprimierter Gene des zu transformierenden Wirtes verwendet. Im Falle von pBR322 als Hybridvektor und *E. coli* als Wirtsmikroorganismus sind beispielsweise die Expressionskontrollsequenzen (welche unter anderem den Promotor und die ribosomale Bindungsstelle enthalten) des Lactoseoperons, Tryptophanoperons, Arabinoseoperons und dergleichen, des β-Lactamasegens, die entsprechenden Sequenzen des Phage λN-Gens oder des Phage fd-Schichtproteingens und andere geeignet. Während der Promotor des β-Lactamasegens (β-lac-Gen) bereits im Plasmid pBR322 enthalten ist, müssen die übrigen Expressionskontrollsequenzen in das Plasmid eingeführt werden. Eine bevorzugte Expressionskontrollsequenz ist diejenige des Tryptophanoperons (trp po).

Zur Replikation und Expression in *Saccharomyces cerevisiae* geeignete Vektoren enthalten einen Replikationsstart und einen selektiven genetischen Marker für *Saccharomyces cerevisiae*. Hybridvektoren, die einen Replikationsstart von *Saccharomyces cerevisiae* enthalten, z. B. das chromosomale autonom replizierende Segment (ars), bleiben nach der Transformation innerhalb der Hefezelle extrachromosomal erhalten und werden bei der Mitose autonom repliziert. Ferner können Hybridvektoren, die der *Saccharomyces cerevisiae* -2μ-Plasmid-DNA homologe Sequenzen enthalten, verwendet werden. Solche Hybridvektoren werden durch Rekombination innerhalb der Zelle bereits vorhandenen 2μ-Plasmiden einverleibt oder replizieren autonom. 2μ-Sequenzen sind besonders für Plasmide mit grosser Transformationshäufigkeit geeignet und gestatten eine hohe Kopienzahl. Geeignete Markierungsgene für *Saccharomyces cerevisiae* sind vor allem solche, die dem Wirt antibiotische Resistenz verleihen oder, im Fall von auxotrophen Mutanten, Gene, die Wirtsdefekte komplementieren. Entsprechende Gene verleihen z. B. Resistenz gegen das Antibiotikum Cycloheximid oder sorgen für Prototrophie in einer auxotrophen Mutante, z. B. das *URA3*-, *LEU2*-, *HIS3*- oder vor allem das *TRP1*-Gen. Geeignete Hybridvektoren für *Saccharomyces cerevisiae* enthalten weiterhin einen Replikationsstart und ein Markierungsgen für *E. coli*, damit die Konstruktion und die Klonierung der Hybridvektoren und ihrer Vorstufen in *E. coli* erfolgen kann. Für die Expression in *Saccharomyces cerevisiae* geeignete Expressionskontrollsequenzen sind beispielsweise diejenigen des *TRP1*-, *ADHI*-, *ADHII*-, *PHO3*- oder *PHO5*-Gens, ferner im glykolytischen Abbau involvierte Promoter, z. B. der *PGK*- und der *GAPDH*-Promoter.

Um eine effektive Expression zu erreichen, muss das Strukturgen richtig (in "Phase") mit der Expressionskontrollsequenz angeordnet sein. Es ist vorteilhaft, die Expressionskontrollsequenz in den Bereich zwischen dem Haupt-mRNA-Start und dem ATG der Genkodiersequenz, welche natürlich mit der Expressionskontrollsequenz verknüpft ist (z. B. die β-lac-Kodiersequenz bei Verwendung des β-lac-Promotors), mit dem Eglin-Gen, welches vorzugsweise sein eigenes Translationsstartsignal (ATG) und Translationsstopsignal (z. B. TAG) mitbringt, zu verknüpfen. Dadurch wird eine effektive Transkription und Translation gewährleistet.

Beispielsweise wird ein Vektor, insbesondere pBR322, mit einer Restriktionsendonuclease geschnitten und, gegebenenfalls nach Modifikation des so gebildeten linearisierten Vektors, eine mit entsprechenden Restriktionsenden versehene Expressionskontrollsequenz eingeführt. Die Expressionskontrollsequenz enthält am 3'-Ende (in Translationsrichtung) die Erkennungssequenz einer Restriktionsendonuclease, sodass der die Expressionskontrollsequenz bereits enthaltende Vektor mit besagtem Restriktionsenzym verdaut und das mit passenden Enden versehene Eglin-Strukturgen eingesetzt werden kann. Dabei entsteht ein Gemisch von zwei Hybridplasmiden, welche das Gen in richtiger bzw. in falscher Orientierung enthalten. Vorteilhaft ist es, den die Expressionskontrollsequenz bereits enthaltenden Vektor noch mit einer zweiten Restriktionsendonuclease innerhalb der Vektor-DNA zu spalten und in das entstandene Vektor-Fragment das mit richtigen Enden versehene Strukturgen einzusetzen. Alle Operationen am Vektor erfolgen vorzugsweise in einer Weise, dass die Funktion des Replicons und zumindest eines Markierungsgens nicht beeinträchtigt wird.

Bevorzugt wird ein von pBR322 abgeleiteter Vektor, welcher eine Expressionskontrollsequenz, insbesondere diejenige vom Tryptophan-Operon (trp po), enthält, die am 3'-Ende (zwischen dem Haupt-mRNA-Start und dem ersten ATG) die Erkennungssequenz für eine, vorzugsweise kohäsive Enden bildende, Restriktionsendonuclease, z. B. EcoRI, trägt, mit der genannten Restriktionsendonuclease und im Vektor-DNA-Teil mit einer zweiten Restriktionsendonuclease, welche flache oder bevorzugt kohäsive Enden bildet, z. B. BamHI, verdaut, wonach der so linearisierte Vektor mit dem entsprechende Enden aufweisenden Eglin-Gen (z. B. mit einem EcoRI-Ende vor

dem ATG-Start und einem BamHI-Ende nach dem Translationsstop-Codon) verknüpft. Die Verknüpfung erfolgt in bekannter Weise durch Paarung der komplementären (kohäsiven) Enden und Ligierung, z. B. mit $T_4$-DNA-Ligase.

Das über den mRNA-Weg, aus genomischer DNA oder synthetisch gewonnene, mit entsprechenden kohäsiven (insbesondere EcoRI- und BamHI-) Enden versehene Eglin-Gen kann vor dem Einbringen in ein Expressionsplasmid auch in einen Vektor, z. B. pBR322, kloniert werden, um grössere Mengen an Strukturgen, beispielsweise für die Sequenzanalyse, zu gewinnen. Die Isolierung der Klone, welche das Hybridplasmid enthalten, wird beispielsweise mit einer Eglin-spezifischen, radioaktiv-markierten Oligodesoxynucleotid-Probe (siehe oben) durchgeführt. Die Charakterisierung des Eglin-Gens erfolgt beispielsweise nach dem Verfahren von Maxam und Gilbert (3).

Bevorzugt werden zwei Teilstücke eines Eglin-Gens, beispielsweise zwei Teilstücke des Eglin C-Gens, synthetisiert. Teilstück 1, welches den 1. Teil des Gens umfasst, enthält vor dem ATG und am Ende jeweils die Erkennungssequenz für kohäsive Enden bildende Restriktionsendonucleasen, z. B. vor dem ATG EcoRI und am Ende HpaII. Teilstück 2, welches den hinteren Teil des Gens umfasst, besitzt entsprechende Erkennungssequenzen, am Anfang z. B. HpaII und nach dem Translationsstopsignal (z. B. TAG) BamHI. Die Teilstücke werden an den äusseren Erkennungssequenzen gespalten (Teilstück 1 z. B. mit EcoRI und Teilstück 2 entsprechend mit BamHI) und in einen entsprechend zugeschnittenen Vektor (z. B. pBR322) subkloniert. Die Identifizierung der Klone, welche die Teilstücke enthalten, und die Charakterisierung der Teilstücke erfolgt wie oben beschrieben. Die Teilstücke werden dann mit den entsprechenden Restriktionsendonucleasen aus den Hybridvektoren herausgeschnitten (Teilstück 1 z. B. mit EcoRI und HpaII und Teilstück 2 z. B. mit HpaII und BamHI) und über ihre kohäsiven Enden, insbesondere HpaII-Enden, ligiert, wobei das komplette Eglin-Gen entsteht, das wie angegeben in eine Vektor-DNA eingefügt wird.

**Transformation der Wirtszellen**

Wirte, die mit einem Expressionsplasmid, das eine von einer Expressionskontrollsequenz regulierte, für ein Eglin kodierende DNA-Sequenz enthält, transformiert werden können sind *Saccharomyces cerevisiae* und *Escherichia coli*. Die Transformation mit den Expressionsplasmiden erfolgt beispielsweise wie in der Literatur beschrieben nach (4) für *S. cerevisiae* und nach (6) für *E. coli*. Die Isolierung des transformierten Wirtes erfolgt vorteilhaft aus einem selektiven Nährmedium, dem das Biocid zugesetzt wird, gegen welches das im Expressionsplasmid enthaltene Markierungs-Gen Resistenz verleiht. Wenn, wie bevorzugt, die Expressionsplasmide das $amp^R$-Gen enthalten, wird dem Nährmedium demgemäss Ampicillin zugesetzt. Zellen, welche das Expressionsplasmid nicht enthalten, werden in einem solchen Medium abgetötet.

**Kultivierung des transformierten Wirtes und Gewinnung von Eglinen**

Der transformierte Wirt kann zur Herstellung von Eglinen verwendet werden. Das Verfahren zur Herstellung von Eglinen ist dadurch gekennzeichnet, dass der transformierte Wirt kultiviert und das Produkt aus den Wirtszellen freigesetzt und isoliert wird.

Überraschenderweise wurde jetzt gefunden, dass die transformierten Wirte *E. coli* und *Saccharomyces cerevisiae* Gemische von Polypeptiden mit Eglin-Aktivität produzieren. Aus den Gemischen lassen sich natürliche Egline, Methionyl-egline und N-terminal acetylierte Egline isolieren. Transformierte Stämme von *E. coli* produzieren insbesondere Polypeptide mit Eglin-Aktivität, welche sich von den natürlichen Eglinen B und C durch einen N-Acetylrest an der N-terminalen Aminosäure Threonin unterscheiden. Die Produktion derartiger Polypeptide mittels gentechnologischer Methoden ist bisher noch nicht beobachtet worden. So wird selbst α-Thymosin, welches natürlich N-terminal acetyliert ist, von entsprechenden genetisch modifizierten Wirten in nicht-acetylierter Form exprimiert (35).

Die Produktion N-terminal acetylierter Egline ist von grossem Vorteil, weil solche Verbindungen eine erhöhte Stabilität gegenüber den in den Wirtszellen vorhandenen Aminopeptidasen besitzen, wodurch der (partielle) proteolytische Abbau ausgehend vom N-Terminus verhindert und infolgedessen die Ausbeute erhöht wird. Weiterhin wird dadurch das Reinigungsverfahren erheblich vereinfacht, weil die gewünschten Produkte nicht mit durch proteolytischen Abbau entstandenen Fragmenten verunreinigt sind.

Die Erfindung betrifft daher ein Verfahren zur Herstellung von Eglin-Verbindungen der Formel

VGluPheGlySerGluLeuLysSerPheProGluValValGlyLysThrValAspGlnAlaArgGlu
TyrPheThrLeuHisTyrProGlnTyrAspValWPheLeuProGluGlySerProValThrLeuAsp (XIV'),
LeuArgTyrAsnArgValArgValPheTyrAsnProGlyThrAsnValValAsnHisValProHis
ValGly

worin V für N-Acetyl-Thr steht und W Tyr oder His bedeutet, und von Salzen solcher Verbindungen, dadurch gekennzeichnet, dass ein mit einem Expressionsplasmid, welches eine von einer Expressionskontrollsequenz regulierte, für ein Eglin kodierende DNA-Sequenz enthält, transformierter *E. coli*- oder *Saccharomyces cerevisiae*-Stamm in einem flüssigen Nährmedium, welches assimilierbare Kohlenstoff- und Stickstoffquellen enthält, kultiviert und das Eglin aus den

Mikroorganismen-Zellen freigesetzt und isoliert wird, und, falls gewünscht, ein erhaltenes Salz in das freie Polypeptid oder ein erhaltenes Polypeptid in ein Salz desselben überführt wird.

In den Verbindungen der Formel XIV' hat W bevorzugt die Bedeutung Tyr (Eglin C-Verbindungen).

Die Erfindung betrifft speziell ein Verfahren zur Herstellung von $N^{\alpha}$-Acetyl-eglin C.

Für die Kultivierung der transformierten Wirte können verschiedene Kohlenstoffquellen verwendet werden. Beispiele bevorzugter Kohlenstoffquellen sind assimilierbare Kohlenhydrate, wie Glucose, Maltose, Mannit oder Lactose, oder ein Acetat, das entweder allein oder in geeigneten Gemischen verwendet werden kann. Geeignete Stickstoffquellen sind beispielsweise Aminosäuren, wie Casaminosäuren, Peptide und Proteine und ihre Abbauprodukte, wie Trypton, Pepton oder Fleischextrakte; weiterhin Hefeextrakte, Malzextrakt, wie auch Ammoniumsalze, z. B. Ammoniumchlorid, -sulfat oder -nitrat, die entweder allein oder in geeigneten Mischungen verwendet werden können. Anorganische Salze, die ebenfalls verwendet werden können, sind z. B. Sulfate, Chloride, Phosphate und Carbonate von Natrium, Kalium, Magnesium und Calcium.

Weiterhin enthält das Medium z. B. wachstumsfördernde Substanzen, wie Spurenelemente, z. B. Eisen, Zink, Mangan und dergleichen, und vorzugsweise Substanzen, die einen Selektionsdruck ausüben und das Wachstum von Zellen, die das Expressionsplasmid verloren haben, verhindern. So wird dem Medium beispielsweise Ampicillin zugesetzt, wenn das Expressionsplasmid ein $amp^R$-Gen enthält. Ein solcher Zusatz von antibiotisch wirksamen Substanzen bewirkt auch, dass kontaminierende, Antibiotika-empfindliche Mikroorganismen abgetötet werden.

Die Kultivierung erfolgt nach an sich bekannten Verfahren. Die Kultivierungsbedingungen, wie Temperatur, pH-Wert des Mediums und Fermentationszeit, werden so ausgewählt, dass maximale Eglin-Titer erhalten werden. So wird ein *E. coli*-Stamm bevorzugt unter aeroben Bedingungen in submerser Kultur unter Schütteln oder Rühren bei einer Temperatur von etwas 20 bis 40°C, vorzugsweise etwa 30°C, und einem pH-Wert von 4 bis 9, vorzugsweise bei pH 7, während etwa 4 bis 20 h, vorzugsweise 8 bis 12 h, kultiviert. Dabei sammelt sich das Expressionsprodukt (Eglin) intrazellulär an.

Wenn die Zelldichte einen ausreichenden Wert erreicht hat, wird die Kultivierung abgebrochen und das Eglin aus den Zellen des Wirtes freigesetzt. Zu diesem Zweck werden die Zellen zerstört, z. B. durch Behandlung mit einem Detergens, wie SDS oder Triton, oder mit Lysozym oder einem gleichartig wirkenden Enzym lysiert. Alternativ oder zusätzlich kann man mechanische Kräfte, wie Scherkräfte (z. B. X-Presse, French-Presse, Dyno-Mill) oder Schütteln mit Glasperlen oder Aluminiumoxid, oder abwechselndes Einfrieren, z. B. in flüssigem Stickstoff, und Auftauen, z. B. auf 30° bis 40°C, sowie Ultraschall zum Brechen der Zellen anwenden. Die resultierende Mischung, die Proteine, Nucleinsäuren und andere Zellbestandteile enthält, wird nach der Zentrifugation in an sich bekannter Weise an Proteinen, inklusive Eglin, angereichert. So wird z. B. der grösste Teil der Nicht-Protein-Bestandteile durch Polyäthylenimin-Behandlung abgetrennt und die Proteine einschliesslich Eglin z. B. durch Sättigen der Lösung mit Ammoniumsulfat oder mit anderen Salzen ausgefüllt. Bakterielle Proteine können auch mittels Ansäuern mit Essigsäure (z. B. 0,1 %, pH 4 – 5) ausgefällt werden. Eine weitere Anreicherung an Eglin kann durch Extraktion des essigsauren Überstandes mit n-Butanol erreicht werden. Weitere Reinigungsschritte umfassen beispielsweise Gelelektrophorese, chromatographische Verfahren, wie Ionenaustauschchromatographie, Grössen-Exklusionschromatographie, HPLC, Umkehrphasen HPLC und dergleichen, Auftrennung der Mischungsbestandteile nach Molekülgrössen mittels einer geeigneten Sephadex-Säule, Dialyse, Affinitätschromatographie, z. B. Antikörper-, insbesondere monoklonale Antikörper-Affinitätschromatographie oder Affinitätschromatographie an einer Anhydrochymotrypsin-Säule, und weitere, insbesondere aus der Literatur, bekannte Verfahren.

Beispielsweise umfasst die Isolierung des exprimierten Eglins die folgenden Stufen:
Abtrennung der Zellen aus der Kulturlösung mittels Zentrifugation;
Herstellung eines Rohextrakts durch Zerstören der Zellen, z. B. durch Behandlung mit einem lysierenden Enzym und/oder abwechselndem Einfrieren und Wiederauftauen;
Abzentrifugieren der unlöslichen Bestandteile;
Ausfällen der DNA durch Zugabe von Polyäthylenimin;
Fällung der Proteine einschliesslich Eglin durch Ammoniumsulfat;
Affinitätschromatographie des gelösten Niederschlags an einer monoklonalen Anti-Eglin-Antikörper-Säule oder einer Anhydro-chymotrypsin-Säule;
Entsalzung der so gewonnen Lösung mittels Dialyse oder Chromatographie an Sephadex G25.

Alternativ können nach Abtrennen der DNA die bateriellen Proteine mittels 0,1 %-iger Essigsäure ausgefällt werden, aus dem sauren Überstand das Eglin mit n-Butanol extrahiert oder der saure Überstand direkt der Ionenaustausch-Chromatographie (z. B. an Carboxymethylcellulose) unterworfen werden. Weitere Reinigungsschritte schliessen Gelfiltration an Sephadex G50 (oder G75), und Umkehrphasen-HPLC ein. Entsalzung erfolgt wieder an Sephadex G25.

Zum Nachweis der Eglin-Aktivität kann der Test mit Anti-Eglin-Antikörpern (z. B. aus Kaninchen erhältliche, oder aus Hybridomazellen erhältliche monoklonale Antikörper) oder die Hemmung der Proteasen Humanleukozyten-Elastase (HLE) oder Cathepsin G (Cat G) (1) durch Eglin herangezogen werden.

Die Erfindung betrifft bevorzugt Eglin-Verbindungen der Formel

VGluPheGlySerGluLeuLysSerPheProGluValValGlyLysThrValAspGlnAlaArgGlu
TyrPheThrLeuHisTyrProGlnTyrAspValWPheLeuProGluGlySerProValThrLeuAsp (XIV'),
LeuArgTyrAsnArgValArgValPheTyrAsnProGlyThrAsnValValAsnHisValProHis
ValGly

worin V für N-Acetyl-Thr steht und W Tyr oder His ist, und Salze von solchen Verbindungen.

Die Erfindung betrifft insbesondere $N^{\alpha}$-Acetyl-eglin C und Salze davon.

Die erfindungsgemäss herstellbaren Verbindungen bzw. die neuen Verbindungen der Formel XIV' können nicht nur in freier Form, sondern auch in Form ihrer Salze, insbesondere ihrer pharmazeutisch annehmbaren Salze, vorliegen. Da sie mehrere Aminosäurereste mit freien Aminogruppen bzw. Guanidinogruppen enthalten, können die erfindungsgemässen Verbindungen z. B. in Form von Säureadditionssalzen vorliegen. Als Säureadditionssalze kommen insbesondere physiologisch verträgliche Salze mit üblichen, therapeutisch anwendbaren Säuren in Betracht; als anorganische Säuren sind die Halogenwasserstoffsäuren (wie die Chlorwasserstoffsäure), aber auch Schwefelsäure und Phosphor- bzw. Pyrophosphorsäure zu nennen; als organische Säuren sind in erster Linie Sulfonsäuren (wie die Benzol- oder p-Toluolsulfonsäure oder Niederalkansulfonsäuren, wie Methansulfonsäure) sowie Carbonsäuren, wie Essigsäure, Milchsäure, Palmitin- und Stearinsäure, Äpfelsäure, Weinsäure, Ascorbinsäure und Citronensäure, geeignet. Da die Eglin-Verbindungen auch Aminosäurereste mit freien Carboxylgruppen enthalten, die dem ganzen Peptid sauren Charakter verleihen, können sie auch als Metallsalz, insbesondere als Alkalimetall- oder Erdalkalimetallsalz, z. B. Natrium-, Kalium-, Calcium- oder Magnesiumsalz, oder auch als Ammoniumsalz, abgeleitet von Ammoniak oder einer physiologisch verträglichen, organischen stickstoffhaltigen Base, vorliegen. Da sie aber zugleich freie Carboxylgruppen und freie Amino (und Amidino)-Gruppen enthalten, können sie auch als inneres Salz vorliegen.

Je nach Arbeitsweise erhält man die erfindungsgemässen Verbindungen in freier Form oder in Form von Säureadditionssalzen, inneren Salzen oder Salzen mit Basen. Aus den Säureadditionssalzen können in an sich bekannter Weise die freien Verbindungen gewonnen werden. Von letzteren wiederum lassen sich durch Umsetzen mit Säuren oder Basen, z. B. mit solchen, die die obengenannten Salze bilden, und Eindampfen oder Lyophilisieren therapeutisch annehmbare Säureadditionssalze oder Metallsalze gewinnen. Die inneren Salze können durch Einstellen des pH auf einen geeigneten Neutralpunkt gewonnen werden.

**Pharmazeutische Präparate**

Die gemäss der vorliegenden Erfindung erhältlichen neuen Egline ($N^{\alpha}$-Acetyl-eglin B und -eglin C) weisen wertvolle pharmakologische Eigenschaften auf und können, wie die aus Blutegeln extrahierten Egline (vgl. DE-OS-2 808 396), prophylaktisch oder insbesondere therapeutisch angewendet werden.

Die erfindungsgemässen neuen Eglin-Verbindungen $N^{\alpha}$-Acetyl-eglin B und $N^{\alpha}$-Acetyl-eglin C zeichnen sich durch eine sehr starke und spezifische Hemmung von Humanleukozyten-Elastase (HLE), Leukozyten-Cathepsin G (H.Cat.G) und Chymotrypsin aus. In der folgenden Tabelle sind die Assoziations-Geschwindigkeitskonstanten ($k_{ass}$) und die Gleichgewichts-Konstanten ($K_i$) der gebildeten Enzym-Inhibitor-Komplexe für die Reaktionen von $N^{\alpha}$-Acetyl-eglin C und zweier natürlich vorkommender Protease-Inhibitoren, $\alpha_1$-Proteinase-Inhibitor ($\alpha_1$PI, früher $\alpha_1$-Antitrypsin genannt) und $\alpha_2$-Makroglobulin ($\alpha_2$M), mit HLE und H.Cat.G zusammengestellt:

**Tabelle**

Kinetische Parameter für die Wechselwirkung ausgewählter Proteinasen mit den Inhibitoren $N^{\alpha}$-Acetyl-eglin C, $\alpha_1$PI und $\alpha_2$M

| Proteine | Inhibitor | $k_{ass}[M^{-1} \cdot sec^{-1}]$ | $K_i$[M] |
|---|---|---|---|
| HLE | $\alpha_1$PI | $1{,}5 \cdot 10^7$ | irreversibel |
| | $\alpha_2$M | $1{,}0 \cdot 10^7$ | irreversibel |
| | $N^{\alpha}$-Acetyl-eglin C | $1{,}4 \cdot 10^7$ | $8 \cdot 10^{-11}$ |
| H.Cat.G | $\alpha_1$PI | $1{,}0 \cdot 10^6$ | irreversibel |
| | $\alpha_2$M | $3{,}5 \cdot 10^6$ | irreversibel |
| | $N^{\alpha}$-Acetyl-eglin C | $2{,}0 \cdot 10^6$ | $5 \cdot 10^{-11}$ |

**Bedingungen**

Die Bestimmung der Assoziations-Geschwindigkeitskonstanten erfolgte nach Bieth et al. (36). Die $K_i$-Werte für die Wechselwirkung von $N^{\alpha}$-Acetyl-eglin C mit HLE und H.Cat.G. wurden aus "steady state" Reaktionsgeschwindigkeiten unter der Annahme errechnet, dass diese Wechselwirkungen reversibel sind. Alle Werte wurden bei 37°C und pH 7,4 bestimmt.

Die Daten zeigen, dass die Assoziations-Geschwindigkeitskonstanten für die Reaktion von $N^{\alpha}$-Acetyl-eglin C sowie der natürlichen Inhibitoren $\alpha_1$PI und $\alpha_2$M mit HLE bzw. H.Cat.G. in der gleichen Grössenordnung liegen. Die hohe Stabilität der $N^{\alpha}$-Acetyl-eglin-Enzym-Komplexe ($K_i$-Werte!), die erwiesene, äusserst geringe Toxizität der Egline, sowie deren Spezifität (signifikante Wechselwirkungen mit anderen Säugetier-Proteinasen, insbesondere

mit solchen der Blutgerinnungs-, Fibrinolyse- und Komplement-Systeme, werden nicht beobachtet), ihre auf Grund der N-terminalen Acetylgruppe erhöhte Stabilität gegenüber proteolytischem Abbau durch Aminopeptidasen und die im Vergleich zu den körpereigenen Faktoren $\alpha_1$PI und $\alpha_2$M leichte Zugänglichkeit grösserer Mengen mit Hilfe des erfindungsgemässen Verfahrens empfehlen diese Verbindungen für die pharmakologische Evaluierung bei Krankheitsbildern, die durch von HLE verursachte Gewebezerstörungen charakterisiert sind.

Die Wirksamkeit der erfindungsgemässen Verbindungen erweist sich beispielsweise im experimentellen Emphysem-Modell. Eine Stunde vor Emphysem-Induktion durch intratracheale Applikation von 0,3 mg HLE im Hamster wurden 0,5 mg bzw. 2 mg $N^{\alpha}$-Acetyl-eglin C (in je 8 Tieren) ebenfalls intratracheal appliziert. Bei nicht geschützten (nicht mit $N^{\alpha}$-Acetyl-eglin C vorbehandelten) Tieren zeigten die nach zwei Monaten durchgeführten Lungenfunktiontests und histologischen Untersuchungen schwere Lungenobstruktionen und Emphysema. Im Gegensatz dazu wiesen alle mit $N^{\alpha}$-Acetyl-eglin C vorbehandelten Tiere normale Lungenfunktionen auf. Die histologische Untersuchung der Lungen ergab lediglich bei zwei der acht Tiere aus der Niedrigdosis-Gruppe (0,5 mg $N^{\alpha}$-Acetyl-eglin C) geringfügige, lokale emphysematische Veränderungen; die übrigen Tiere zeigten keine Veränderungen, wodurch die Schutzwirkung des intratracheal applizierten $N^{\alpha}$-Acetyl-eglin C und gleichzeitig dessen geringe Toxizität bewiesen sind.

Die erfindungsgemässen neuen Eglin-Verbindungen können demgemäss zur Prophylaxe und für die therapeutische Behandlung von Lungenerkrankungen, z. B. durch Leukocyten-Elastase hervorgerufenen Lungenerkrankungen, wie Lungenemphysem und ARDS ("acute respiratory distress syndrome"), Mucoviscidose, ferner bei septischem Schock sowie als Antiphlogistika und Antiinflammatorika verwendet werden. Die Verwendung der erfindungsgemässen neuen Eglin-Verbindungen und ihrer pharmazeutisch annehmbaren Salze bei der prophylaktischen und therapeutischen Behandlung der genannten Krankheitsbilder ist ebenfalls Gegenstand der vorliegenden Erfindung.

Die Erfindung betrifft ebenfalls pharmazeutische Zusammensetzungen, welche wenigstens eine der erfindungsgemässen Verbindungen oder deren pharmazeutisch annehmbaren Salze enthalten.

Diese Zusammensetzungen können insbesondere bei den oben angegebenen Indikationen Verwendung finden, wenn sie z. B. parenteral (wie intravenös oder intrapulmonal) oder topisch verabreicht werden. Die Dosierung hängt in erster Linie von der spezifischen Verarbeitungsform und vom Zweck der Therapie bzw. Prophylaxe ab.

Die Verabreichung erfolgt durch intravenöse Injektion oder intrapulmonal, durch Inhalation, z. B. mit einem Bird-Gerät. Dementsprechend enthalten pharmazeutische Präparate zur parenteralen Verabreichung in Einzeldosis-Form in Abhängigkeit von der Applikationsart pro Dosis etwa 10 bis 50 mg der erfindungsgemässen Verbindungen. Neben dem Wirkstoff enthalten diese pharmazeutischen Zusammensetzungen üblicherweise noch Natriumchlorid, Mannit oder Sorbit zur Einstellung der Isotonie. Sie können in gefriergetrockneter oder gelöster Form vorliegen, wobei Lösungen mit Vorteil ein antibakteriell wirkendes Konservierungsmittel, z. B. 0,2 bis 0,3 % 4-Hydroxybenzoesäure-methylester oder -ethylester, enthalten können.

Ein Präparat für die topische Anwendung kann als wässrige Lösung, Lotion oder Gelee, ölige Lösung oder Suspension, oder fetthaltige oder insbesondere Emulsions-Salbe vorliegen. Ein Präparat in Form einer wässrigen Lösung erhält man beispielsweise dadurch, dass man die erfindungsgemässen Wirkstoffe oder ein therapeutisch annehmbares Salz davon in einer wässrigen Pufferlösung von pH 4 bis 7,5 löst und gewünschtenfalls einen weiteren Wirkstoff, z. B. ein Antiinflammatorikum, und/oder ein polymeres Haftmittel, z. B. Polyvinylpyrrolidon, und/oder ein Konservierungsmittel zufügt. Die Konzentration des Wirkstoffs beträgt etwa 0,1 bis etwa 5 mg, vorzugsweise 0,25 bis 1,0 mg, in 10 ml einer Lösung bzw. 10 g eines Gelees.

Eine ölige Applikationsform für die topische Verarbreichung erhält man beispielsweise durch Suspendieren der erfindungsgemässen Wirkstoffe oder eines therapeutisch annehmbaren Salzes davon in einem Öl, gegebenenfalls unter Zusatz von Quellmitteln, wie Aluminiumstearat, und/oder grenzflächenaktiven Mitteln (Tensiden), deren HLB-Wert ("hydrophilic-lipophilic-balance") unter 10 liegt, wie Fettsäuremonoester mehrwertiger Alkohole, z. B. Glycerinmonostearat, Sorbitanmonolaurat, Sorbitanmonostearat oder Sorbitanmonooleat. Eine fetthaltige Salbe erhält man z. B. durch Suspendieren der erfindungsgemässen Wirkstoffe oder deren Salze in einer streichbaren Fettgrundlage, gegebenenfalls unter Zusatz eines Tensids vom HLB-Wert unter 10. Eine Emulsionssalbe erhält man durch Verreihen einer wässrigen Lösung der erfindungsgemässen Wirkstoffe oder deren Salze in einer weichen, streichbaren Fettunterlage unter Zusatz eines Tensids, dessen HLB-Wert unter 10 liegt. Alle diese topischen Applikationsformen können auch Konservierungsmittel enthalten. Die Konzentration des Wirkstoffes beträgt etwa 0,1 bis etwa 5 mg, vorzugsweise 0,25 bis 1,0 mg, in etwa 10 g der Grundmasse.

Inhalationspräparate für die Behandlung der Atemwege durch intrapulmonale Verabreichung sind z. B. Aerosole oder Sprays, welche den pharmakologischen Wirkstoff in Form von Tropfen einer Lösung oder Suspension verteilen können. Präparate, in welchen der pharmakologische Wirkstoff in Lösung vorliegt, enthalten ausser diesem ein geeignetes Treibmittel, ferner, falls notwendig, ein zusätzliches Lösungsmittel und/oder einen Stabilisator. Anstelle des Treibgases kann auch Druckluft verwendet werden, wobei diese mittels einer geeigneten Verdichtungs- und Entspannungsvorrichtung nach Bedarf erzeugt werden kann.

Besonders geeignet für die Verabreichung sind Bird-Beatmungsgeräte, welche in der Medizin eingeführt und bekannt sind; dabei wird eine Lösung des Wirkstoffs ins Gerät gegeben und mit leichtem Überdruck vernebelt und in die Lunge des beatmeten Patienten gebracht.

Je nach Alter, individuellem Zustand und Art der Erkrankung beträgt bei einem Warmblüter (Mensch oder Tier) von etwa 70 kg Gewicht die Dosierung bei intrapulmonaler Verabreichung etwa 10 bis etwa 30 mg pro Inhalation (ein- bis zweimal täglich) und bei intravenöser Verabreichung, z. B. auch durch Dauerinfusion, bei

etwa 10 bis etwa 1000 mg pro Tag.

Therapeutisch wirksame Sputum- und Plasmakonzentrationen, welche mittels immunologischer Verfahren, wie ELISA, bestimmt werden können, liegen zwischen 10 und 100 µg/ml (ca. 1 bis 10 µMol/l).

Insbesondere betrifft die Erfindung das in den Beispielen beschriebene Verfahren zur Herstellung von Eglinen mit Hilfe der transformierten Mikroorganismen *E. coli* und *Saccharomyces cerevisiae*, sowie die in den Beispielen genannten neuen Eglin-Verbindungen.

Einige Ausführungsformen der vorliegenden Erfindung, die im nachfolgenden Experimentellen Teil beschrieben sind, werden anhand der begleitenden Zeichnungen veranschaulicht.

- In Figur 1 ist die Synthese der Teilstücke $F_1$ (C) und $F_2$ des Eglin C-Gens schematisch dargestellt.
- Figur 2 zeigt die Herstellung des Plasmids pML 87, des Klonierungsvektors für das Teilstück $F_1$ (C) des Eglin C-Gens.
- Figur 3 zeigt dementsprechend die Herstellung des Plasmids pML136, des Klonierungsvektors für das Teilstück $F_2$ des Eglin C- bzw. Eglin B-Gens.
- In Figur 4 ist die Konstruktion des Klonierungsvektors pML141, welcher die $F_1$ (C)-$F_2$-DNA enthält, erläutert.
- In Figur 5 ist die Herstellung des Vektors pHRi148, welcher den trp-Promoter enthält, schematisch dargestellt.
- Figur 6 zeigt schematisch die Herstellung des Expressionsplasmids pML147, welches das Eglin C-Gen [$F_1$ (C)-$F_2$-DNA] unter der Kontrolle des trp-Promoters enthält.

Die folgenden Beispielen dienen zur Illustration der Erfindung und sollen sie in keiner Weise einschränken.

**Experimenteller Teil**

Die in den Beispielen verwendeten Abkürzungen haben die folgende Bedeutung:

| | |
|---|---|
| TNE | Lösung, die 100 mM NaCl, 50 mM Tris · HCl pH 7,5 und 5 mM EDTA enthält, |
| SDS | Natriumdodecylsulfat |
| EDTA | Ethylendiamintetraessigsäure |
| DTT | 1,4-Dithiothreitol (1,4-Dimercapto-2,3-butandiol) |
| BSA | Rinder-Serumalbumin |
| EtBr | Ethidiumbromid |
| Tris | Tris-(hydroxymethyl)-aminomethan |
| Tris · HCl | Monohydrochlorid von Tris |

**Beispiel 1**

**Herstellung des geschützten Nucleosid-Polystyrol-Harzes**

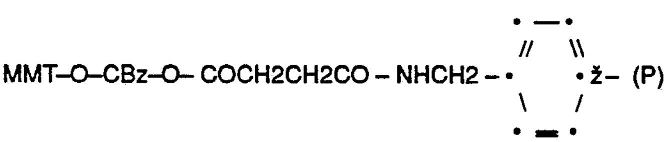

3,1 g (5 mMol) 5'-(4-Methoxytrityl)-N-benzoyl-deoxycytidin in 20 ml abs. Pyridin werden mit 750 mg Bernsteinsäureanhydrid und 910 mg 4-Dimethylaminopyridin versetzt und 16 Stunden bei Raumtemperatur belassen. Nach Einengen der Pyridin-Lösung wird in 200 ml Essigsäureethylester aufgenommen, zweimal mit je 200 ml 0,1 M Phosphatpuffer unter Zusatz von 10 ml gesättigter Kochsalz-Lösung ausgeschüttelt, nochmals mit gesättigter Kochsalzlösung gewaschen, getrocknet, eingeengt und Hexan zugetropft. Das ausgefallene Produkt wird abgetrennt und zweimal mit Äther zerrieben, dann in 300 ml Essigsäureethylester gelöst und bei 0°C mit 180 ml 0,1 M Kaliumhydrogensulfat von pH 2,5 ausgeschüttelt. Nach zweimaligem Waschen mit Wasser wird die Essigesterlösung mit Natriumsulfat getrocknet, filtriert, mit 0,5 ml Pyridin versetzt, eingeengt und tropfenweise mit Hexan verdünnt. Das ausgefallene Bernsteinsäurederivat wird abfiltriert.

1,17 g dieser Verbindung werden zusammen mit 190 mg N-Hydroxysuccinimid in 4 ml Essigsäureethylester und 2 ml Dimethylformamid gelöst und bei 0°C mit 370 mg N,N'-Dicyclohexylcarbodiimid versetzt. Nach Stehen über Nacht im Kühlschrank wird der ausgefallene N,N'-Dicyclohexylharnstoff abfiltriert, das Filtrat mit Essigsäureethylester verdünnt, mit kaltem 0,1 M Natriumhydrogencarbonat und Wasser extrahiert, getrocknet und im Vakuum zur Trockne eingedampft. Der Rückstand wird mit Essigsäureethylester an Kieselgel chromatographiert. DC: $R_f$ 0,58 in Dichlormethan-Methanol (9 : 1).

88 mg dieses N-Succinimidoyl-bernsteinsäureesters werden mit 1 g Aminomethyl-polystyrol (Amingehalt 110 µMol/g) in 2 ml Dichlormethan und 4 ml Dimethylformamid 20 Stunden gerührt. Das Polymer-Harz wird abfiltriert und mit Dimethylformamid, Methanol, Dichlormethan und Methanol ausgewaschen. Nach dem Trocknen werden

die nicht umgesetzten Aminogruppen acetyliert, indem das Harz in 6 ml Pyridin mit 1 ml Essigsäureanhydrid und 100 mg 4-Dimethylaminopyridin 30 min. gerührt wird. Das Polymer-Harz wird mit Dichlormethan, Dimethylformamid, Methanol und Dichlormethan ausgewaschen und bis zur Gewichtskonstanz getrocknet. Die spektroskopische Methoxytrityl (MMT)-Bestimmung ergibt eine Beladung von 85 µMol/g.

**Beispiel 2**

Analog Beispiel 1 werden folgende geschützte Nucleosid-Polystyrol-Harze hergestellt:

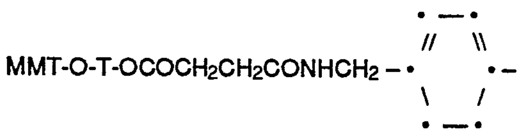
aus 5'-(4-Methoxytrityl)-thymidin, Beladung 92 µMol/g.

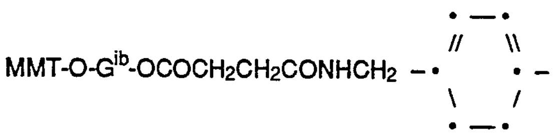
aus 5'-(4-Methoxytrityl)-N-isobutyryl-deoxyguanosin,

Beladung 75 µMol/g.

**Beispiel 3**

**Synthese des Trinukleotids**

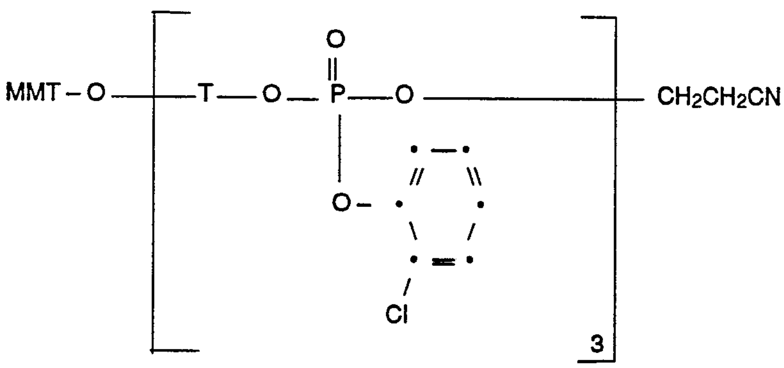

a) Synthese des Dinukleotids:

7,73 g (15 mMol) 5'-(4-Methoxytrityl)-thymidin (MMT-O-T-OH) werden zweimal mit abs. Pyridin eingedampft. Der Rückstand wird in 20 ml abs. Tetrahydrofuran gelöst und zu 80 ml einer 0,2 M Lösung von 2-Chlorphenyl-di-(1-benzotriazolyl)-phosphat in Tetrahydrofuran unter Rühren und Feuchtigkeitsausschluss getropft und das Reaktionsgemisch 1 Stunde bei Raumtemperatur gerührt. Die erhaltene Lösung des 2-Chlorphenyl-1-benzotriazolyl-5'-(4-methoxytrityl)-thymidin-3'-phosphats wird in drei Teile geteilt.

α) Hydrolyse zu Triethylammonium-2-chlorphenyl-5'-(4-methoxytrityl)-thymidin-3'-phosphat:

Zu einem Drittel der obigen Lösung von 2-Chlorphenyl-1-benzotriazolyl-5'-(4-methoxytrityl)-thymidin-3'-phosphat werden unter Kühlung 100 ml 0,5 M Triethylammoniumbicarbonat gegeben. Nach 15 min. wird mit Dichlormethan extrahiert. Die Dichlormethan-Lösung wird mit Wasser gewaschen, eingeengt und tropfenweise mit Petrolether versetzt. Die erhaltene Fällung wird abgenutscht, mit Ether-Petrolether 1 : 1 ausgewaschen und im Vakuum getrocknet. DC: $R_f$ 0,35 in Dichlormethan-Methanol-Wasser (75 : 22 : 3).

β) Veresterung zu 2-Cyanoethyl-2-chlorphenyl-5'-(4-methoxytrityl)-thymidin-3'-phosphat und Abspaltung der 4-Methoxytrityl-Schutzgruppe:

Zu einem Drittel der Lösung von 2-Chlorphenyl-1-benzotriazolyl-5'-(4-methoxytrityl)-thymidin-phosphat werden 1,3 ml 2-Cyanoethanol und 2 ml Pyridin gegeben. Das Gemisch wird über Nacht bei Raumtemperatur belassen. Die Lösungsmittel werden im Vakuum abdestilliert und der Rückstand in Essigsäureethylester gelöst und mehrmals mit 0,1 M Phosphatpuffer pH 7 und Wasser ausgeschüttelt. Die organische Phase wird getrocknet, eingeengt und in Hexan eingetropft. Der Niederschlag wird abfiltriert, in 50 ml Dichlormethan-Methanol 7 : 3 gelöst und bei 0°C mit einer Lösung von 3,8 g p-Toluolsulfonsäuremonohydrat in 75 ml Dichlormethan-Methanol 7 : 3 versetzt. Nach 2 Stunden wird die Reaktionslösung mit Dichlormethan verdünnt und mit einer kalten Natriumhydrogencarbonat-Lösung ausgeschüttelt. Die organische Phase wird eingeengt und mit Hexan versetzt. Das ausgefallene 2-Cyanoethyl-2-chlorphenyl-thymidin-3'-phosphat wird an Kieselgel mit Dichlormethan-Methanol 96 : 4 chromatographiert. DC: $R_f$ von 0,45 in Dichlormethan-Methanol (9 : 1).

λ) Kondensation zum 5'-(4-Methoxytrityl)-3'-cyanoethyl)-bis-thymidin-dinukleotid:

2,2 g 2-Cyanoethyl-2-chlorphenyl-thymidin-3'-phosphat werden zweimal durch Eindampfen mit abs. Pyridin entwässert, in 20 ml abs. Tetrahydrofuran gelöst und zum restlichen Drittel der Lösung von 2-Chlorphenyl-1-benzotriazolyl-5'-(4-methoxytrityl)-thymidin-3'-phosphat gegeben. Nach 18 Stunden bei Raumtemperatur werden zur Reaktionslösung unter Eiskühlung 10 ml Wasser und 200 ml Essigsäureethylester zugegeben. Die organische Phase wird mehrmals mit Natriumhydrogencarbonat und Wasser gewaschen, über Natriumsulfat getrocknet und auf ein kleines Volumen eingeengt. Das im Phosphat-Teil und am 5'- bzw. 3'-Ende geschützte Dinukleotid wird durch Eintropfen in Ether-Hexan 1 : 1 gefällt. DC: $R_f$ 0,48 in Dichlormethan-Methanol (9 : 1).

b) Synthese des Trinukleotids:

1,17 g (1 mMol) des oben beschriebenen vollgeschützten Dinukleotids werden in 30 ml Dichlormethan-Methanol 7 : 3 gelöst und unter Eiskühlung mit einer Lösung von 1,9 g p-Toluolsulfonsäuremonohydrat in 20 ml Dichlormethan-Methanol 7 : 3 versetzt. Nach 2 Stunden wird eiskalte Natriumhydrogencarbonat-Lösung zugegeben und mit Dichlormethan extrahiert. Die organische Phase wird getrocknet, eingeengt und in Hexan eingetropft. Das ausgefallene rohe Dinukleotid mit freier 5'-Hydroxygruppe wird an Kieselgel mit einem Gradienten von 2 – 8 % Methanol in Dichlormethan chromatographiert. DC: $R_f$ 0,33 in Dichlormethan-Methanol (9 : 1).

850 mg dieses 5'-Hydroxy-dinukleotids und 1,06 g Triethylammonium-2-chlorphenyl-5'-(4-methoxytrityl)-thymidin-3'-phosphat [vgl. Abschnitt a)α)] werden zweimal mit Pyridin eingedampft, dann in 10 ml abs. Pyridin gelöst und mit 560 mg Mesitylensulfonyl-3-nitro-1,2,4-triazolid (MSNT) versetzt. Nach 2 Stunden werden 2 ml eiskaltes Wasser zugegeben und nach einer weiteren Stunde mit Dichlormethan extrahiert. Die organische Phase wird mit gesättigtem Natriumhydrogencarbonat-Lösung und Wasser gewaschen, getrocknet, eingeengt und mit Ether versetzt. Das ausgefallene Trinukleotid wird durch Chromatographie an Kieselgel gereinigt. $R_f$ 0,45 in Dichlormethan-Methanol (9 : 1).

**Beispiel 4**

Analog Beispiel 3 werden folgende geschützte Trinukleotide der allgemeinen Formel

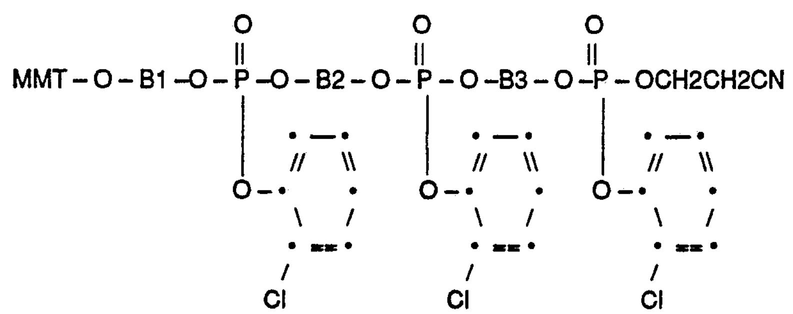

abgekürzt $B^1B^2B^3$ ergestellt. Für die Nukleoside $B^1$, $B^2$, $B^3$ werden folgende Abkürzungen verwendet:

A = N-Benzoyl-deoxyadenosin
C = N-Benzoyl-deoxycytidin
G = N-Isobutyryl-deoxyguanosin
T = Thymidin

| Verbindung | $R_f$[a)] | Verbindung | $R_f$[a)] |
|---|---|---|---|
| TTT | 0,45 | ATG | 0,48 |
| TTC | 0,55 | ACT | 0,53 |
| TCT | 0,46 | ACC | 0,48 |
| TAC | 0,56 | AAT | 0,49 |
| TAA | 0,53 | AAC | 0,46 |
| TAG | 0,60 | AAA | 0,51 |
| TGT | 0,42 | AGT | 0,45 |
| TGG | 0,43 | AGA | 0,49 |
| CTG | 0,46 | GTT | 0,45 |
| CCT | 0,45 | GCT | 0,55 |
| CCG | 0,47 | GCA | 0,49 |
| CAT | 0,55 | GCG | 0,48 |
| CAA | 0,52 | GAT | 0,44 |
| CAG | 0,44 | GAC | 0,48 |
| CGT | 0,49 | GAA | 0,50 |
| GGA | 0,44 | GGT | 0,46 |

a) Dünnschichtchromatogramm auf Kieselgel in Dichlormethan-Methanol 9 : 1.

**Beispiel 5**

**Synthese des DNA-Fragments einer Länge von 61 Basen von Base Nr. 172 bis Base Nr. 232 des komplementären DNA-Stranges (172/61 Komplementär)**

a) Abspaltung der 2-Cyanethylschutzgruppe der Trinukleotide:

15 µMol der Trinukleotide aus Beispiel 3 oder 4 werden unter Feuchtigkeitsausschluss in 60 µl Pyridin-Acetonitril-Triethylamin 1 : 1 : 1 gelöst. Nach 1 Stunde bei Raumtemperatur werden 0,7 ml peroxidfreier Ether zugetropft und der Niederschlag abzentrifugiert. Das rohe Triethylammoniumsalz wird in 50 µl Pyridin gelöst und nochmals mit 0,5 ml Ether ausgefällt, abzentrifugiert und 15 Stunden am Hochvakuum getrocknet.

b) Kupplung der teilgeschützten Trinukleotide mit der an Polystyrol-Harz gebundenen Oligonukleotidkette:

Alle Operationen werden unter Feuchtigkeitsausschluss in einem Reaktionsgefäss mit 280 µl Inhalt und mikroprozessorgesteuerter Lösungsmittel- und Reagens-Zugabe durchgeführt. 17,6 mg (1,5 µMol) des Cytidin-Polystyrol-Harzes (Beispiel 1) werden im Reaktionsgefäss vorgelegt und folgenden Operationen unterworfen:

1. Methylenchlorid, 2 ml/min., 5 min.
2. Methylenchlorid-Isopropanol (85 : 15), 2 ml/min., 2 min.
3. Zinkbromid 1 M und 1,2,4-Triazol 0,02 M in Methylenchlorid-Isopropanol (7 : 3), 1 ml/min., 2 – 3,5 min.
4. Methylenchlorid-Isopropanol (85 : 15), 2 ml/min., 3 min.
5. Triethylammoniumacetat 0,5 M in DMF, 2 ml/min., 10 min.
6. Molekularsieb-getrocknetes Pyridin, 2 ml/min., 5 min.
7. Tetrahydrofuran (Peroxid-frei, Molekularsieb-getrocknet), 2 ml/min., 5 min.
8. Stickstoffstrom, 10 min.
9. Injektion 15 µMol Trinukleotid AAA (Trimethylammoniumsalz aus Abschnitt a)) und 13,3 mg (45 µMol) Mesitylensulfonyl-3-nitro-1,2,4-triazolid (MSNT) gelöst in 160 µl Pyridin
10. 40°C, 30 min.
11. Pyridin, 2 ml/min., 5 min.
12. Acetanhydrid 5 % und 4-Dimethylaminopyridin 2,5 % in Pyridin, 2 ml/min., 5 min.
13. Pyridin, 2 ml/min., 5 min.
14. Pyridin-Isopropanol (1 : 1), 2 ml/min., 3 min.

Alle 14 Operationen werden 19 mal wiederholt, wobei bei der 9. Operation anstelle von AAA jeweils folgende Trinukleotide in Form ihrer Triethylammoniumsalze (Abschnitt a)) eingesetzt werden: AGA, TGT, GGT, CTG, TAC, TAG, CGT, CAA, TAA, GGT, CAT, GAA, GCG, CAT, CAA, AAC, CCT, GAT, CAG. Die mittlere Kupplungsausbeute beträgt 96 %.

Das Endprodukt hat folgende Struktur:

MMT-CAGGATCCTAACCAACATGCGGAACATGGTTAACAACGTTAGTACCTGGGTTGTAGAAAAC-Polystyrol

c) Abspaltung des DNA-Fragments vom Träger und Abspaltung der Schutzgruppen:

40,2 mg (ca. 0,66 µMol) DNA-Syntheseharz/172/61 komplementär werden mit 66 mg (0,40 mMol) o-Nitrobenzaldoxim und 50 µl (0,40 mMol) 1,1,3,3-Tetramethylguanidin in 400 µl 95 %-igem Pyridin während 3 Stunden bei 50°C und während 12 Stunden bei Raumtemperatur gehalten. Nach dem Abblasen des Pyridins mit Stickstoff wird der Rückstand mit 1,6 ml wässerigem Ammoniak (33 %) versetzt und im geschlossenen Gefäss während 24 Stunden bei 50°C gehalten.

Die abgetrennte flüssige Phase wird im Vakuum vom Ammoniak befreit und 3 mal mit je 3 ml peroxidfreiem Diethylether gewaschen. Nach dem Abtrennen der niedermolekularen Bestandteile an einer Biogel P6 Säule (100 – 200 mesh, 3 x 66 cm, 0,01 Molar Trimethylammoniumhydrogencarbonat pH 7,5, 1,5 ml/min.) werden 285 ODs (260 nm) DNA isoliert.

Insgesamt 60 ODs werden an einer HPLC-Säule aufgetrennt (PRP-1/Hamilton, 250 x 4,6 mm). Gradient (Lösung A: 0,05 M Triethylammoniumacetat pH 7,0; Lösung B: Lösung A : Acetonitril 1 : 1): 30 % B in A → 60 % B in A in 20 min. bei 50°C und 2 ml/min. Der lipophile Hauptpeak (Retentionszeit ca. 14 min.) wird gesammelt, an einer DE52-Cellulose (Whatman) Säule aufkonzentriert, eluiert und mit Ethanol gefällt. Zur Abspaltung der 4-Methoxytrityl-Schutzgruppe wird der Niederschlag in 50 µl Essigsäure/$H_2O$ (4 : 1) gelöst und 45 min. bei Raumtemperatur gehalten. Das Reaktionsprodukt wird lyophilisiert, mit Ethanol gefällt und zur Reinigung auf einem 8 % Polyacrylamidgel (7 M Harnstoff) elektrophoretisch aufgetrennt. Die der erwarteten DNA-Grösse entsprechende Bande wird ausgeschnitten und das Produkt elektroeluiert, an DE52-Cellulose aufkonzentriert und die DNA der Struktur

5'-CAGGATCCTAACCAACATGCGGAACATGGTTAACAACGTTAGTACCTGGGTTGTAGAAAAC-3'

mit Ethanol gefällt.

**Beispiel 6**

Analog Beispiel 5 werden folgende DNA-Fragmente (5'-3') hergestellt:

1/40
CTGGAATTCATGACTGAATTTGGTTCTGAACTGAAATCTT

30/37 komplementär
AACAGTTTTACCAACAACTTCTGGGAAAGATTTCAGT

67/34
GACCAGGCTCGTGAATACTTCACTCTGCATTACC

91/37 komplementär (C)
CCGGCAGGAAGTAAACGTCGTACTGCGGGTAATGCAG

91/37 komplementär (B)
CCGGCAGGAAATGAACGTCGTACTGCGGGTAATGCAG

124/61
CCGGAAGGTTCTCCTGTTACTCTGGACCTGCGTTACAACCGTGTTCGTGTTTTCTACAACC

Ferner werden die folgenden verkürzten Fragmente hergestellt:

1/40 (Δ 12) (C')
CTGGAATTCATGTCTGAACTGAAATCTT

und 1/40 (Δ 18) (C")
CTGGAATTCATGCTGAAATCTT

**Beispiel 7**
**Phosphorylierung der Fragmente 30/37, 67/34, 124/61 und 172/61**

Die Phosphorylierung und die radioaktive Markierung an den 5'-Enden erfolgen mit [$\lambda$-$^{32}$P]ATP und $T_4$ Polynukleotid-Kinase (Boehringer) wie beschrieben (19).

**Beispiel 8**

**Polymerisation zu Duplex III (Teilstück $F_2$ des Eglin C und Eglin B-Gens)**

Je 50 pMol Fragment 124/61/kinasiert und Fragment 172/61/kinasiert werden in 24 µl Wasser gelöst, die Lösung während 3 min. auf 90°C erwärmt und innerhalb von 5 min. auf 12°C abgekühlt. Nach Zugabe von 4 µl Endo-R Puffer (0,1 Molar Tris · HCl pH 7,5, 66 mM $MgCl_2$, 66 mM β-Mercaptoethanol, 0,6 M NaCl), 10 µl Deoxynucleosidtriphosphat-Gemisch (dATP, dCTP, dGTP, TTP, je $2 \cdot 10^{-3}$ Molar, mit $NH_3$ auf pH 7,0 eingestellt) und 2 µl (10 Einheiten) DNA-Polymerase I, Klenow-Fragment (Boehringer) wird während 30 min. bei 12°C inkubiert. Die Reaktion wird durch 3 minütiges Erhitzen auf 90°C gestoppt und das Gemisch bis zur Weiterverarbeitung bei −80°C aufbewahrt.

In analoger Weise werden die Fragmente 1/40 und 30/37, 67/34 und 91/37 (C) bzw. 67/34 und 91/37 (B) zu den Duplexen I, II (C) bzw. II (B) polymerisiert.

Die Duplexe I – III haben die folgende Strukturen:

Duplex I
CTGGAATTCATGACTGAATTTGGTTCTGAACTGAAATCTTTCCCAGAAGTTGTTGGTAAAACTGTT
GACCTTAAGTACTGACTTAAACCAAGACTTGACTTTAGAAAGGGTCTTCAACAACCATTTTGACAA

Duplex II (C)
GACCAGGCTCGTGAATACTTCACTCTGCATTACCCGCAGTACGACGTTTACTTCCTGCCGG
CTGGTCCGAGCACTTATGAAGTGAGACGTAATGGGCGTCATGCTGCAAATGAAGGACGGCC

Duplex II (B)
GACCAGGCTCGTGAATACTTCACTCTGCATTACCCGCAGTACGACGTTCATTTCCTGCCGG
CTGGTCCGAGCACTTATGAAGTGAGACGTAATGGGCGTCATGCTGCAAGTAAAGGACGGCC

Duplex III (Teilstück $F_2$ des Eglin C- und Eglin B-Gens)
CCGGAAGGTTCTCCTGTTACTCTGGACCTGCGTTACAACCGTGTTCGTGTTTTCTACAACCCAGGTAC
GGCCTTCCAAGAGGACAATGAGACCTGGACGCAATGTTGGCACAAGCACAAAAGATGTTGGGTCCATG

TAACGTTGTTAACCATGTTCCGCATGTTGGTTAGGATCCTG
ATTGCAACAATTGGTACAAGGCGTACAACCAATCCTAGGAC

**Beispiel 9**

**Ligation von Duplex I mit Duplex II (C), Herstellung des Teilstücks $F_1$ (C) des Eglin C-Gens**

Je 60 pMol Duplex I und Duplex II (C) (vgl. Beispiel 8; nur an den A und G 5'-Enden kinasiert) werden in 54 µl Ligase-Puffer (66 mM Tris · HCl pH 7,5, 6,6 mM $MgCl_2$, 10 mM Dithiothreitol, 5 mM ATP) gelöst, mit 6 µl (= 6 Einheiten) $T_4$-DNA-Ligase (Boehringer) versetzt und während 21 Stunden bei 20°C inkubiert. Die Reaktion wird durch 5 minütiges Erhitzen auf 70°C gestoppt und die DNA nach Phenol/Chloroform-Extraktion durch Ethanolfällung isoliert.

Nach Auftrennung des Gemisches an einem 8 % Polyacrylamidgel (nativ) durch Elektrophorese werden die Ligationsprodukte mit 122 – 132 Basenpaaren elektroeluiert, an einer DE52-Cellulose-Säule aufkonzentriert und nach Elution durch Ethanolfällung isoliert.

Das Teilstück $F_1$ (C) des Eglin C-Gens hat die folgende Struktur:

CTGGAATTCATGACTGAATTTGGTTCTGAACTGAAATCTTTCCCAGAAGTTGTTGGTAAAACTGTT
GACCTTAAGTACTGACTTAAACCAAGACTTGACTTTAGAAAGGGTCTTCAACAACCATTTTGACAA

GACCAGGCTCGTGAATACTTCACTCTGCATTACCCGCAGTACGACGTTTACTTCCTGCCGG
CTGGTCCGAGCACTTATGAAGTGAGACGTAATGGGCGTCATGCTGCAAATGAAGGACGGCC

**Beispiel 10**

**Ligation von Duplex I mit Duplex II (B), Herstellung des Teilstücks $F_1$ (B) des Eglin B-Gens**

In analoger Weise, wie in Beispiel 9 beschrieben, werden je 60 pMol Duplex I und Duplex II (B) miteinander ligiert.

Das Teilstück $F_1$ (B) des Eglin (B)-Gens hat die folgende Struktur:

CTGGAATTCATGACTGAATTTGGTTCTGAACTGAAATCTTTCCCAGAAGTTGTTGGTAAAACTGTT
GACCTTAAGTACTGACTTAAACCAAGACTTGACTTTAGAAAGGGTCTTCAACAACCATTTTGACAA

GACCAGGCTCGTGAATACTTCACTCTGCATTACCCGCAGTACGACGTTCATTTCCTGCCGG
CTGGTCCGAGCACTTATGAAGTGAGACGTAATGGGCGTCATGCTGCAAGTAAAGGACGGCC

**Beispiel 11**

**Herstellung des Plasmids pML87, enthaltend die $F_1$ (C)-DNA des Eglin C-Gens (Fig. 2)**

a) Herstellung des linearisierten Vektors pBR322/EcoRI/BalI.

30 µg pBR322 Plasmid-DNA werden mit 5 Einheiten BalI Restriktionsendonuclease (Biolabs) in 200 ml einer Lösung aus 100 µg/ml Gelatine für 5 Stunden bei 37°C verdaut. Anschliessend wird diese Lösung auf 100 mM Tris · HCl (pH 7,5), 50 mM NaCl eingestellt und die DNA mit 30 Einheiten EcoRI-Restriktionsendonuclease (Biolabs) für 2 Stunden bei 37°C verdaut. Dann wird die Lösung auf TNE eingestellt, mit 1 Volumen Phenol und Chloroform extrahiert und die verdaute DNA mit 2 Volumen Alkohol bei –20°C über Nacht ausgefüllt.

Der aus der pBR322 DNA herausgeschnittene Vektor (pBR322/EcoRI/BalI, 2916 Basenpaare) wird durch Dichtegradientenzentrifugation in Sucrose (5 – 23 %) in 50 mM Tris · HCl (pH 8) und 1 mM EDTA vom kleinen DNA-Fragment (1445 Basenpaare) abgetrennt. Die Zentrifugation wird bei 36.000 upm in einem TST 41 Rotor (Kontron AG) für 16 Stunden bei 15°C durchgeführt. Anschliessend werden von der zentrifugierten Lösung Fraktionen à 0,2 ml mit einem ISCO Gradienten-Sammler gewonnen. Diejenigen Fraktionen, welche das grosse DNA-Fragment (2916 Basenpaare) enthalten, werden vereinigt und die DNA mit Alkohol ausgefällt. Der Niederschlag wird in 100 µl 10 mM Tris · HCl (pH 8), 0,1 mM EDTA gelöst und bis zu seiner Verwendung als Klonier-Vektor bei –20°C aufgehoben. 5,1 µg (= 10,5 pMol Enden) DNA werden erhalten.

b) Herstellung der $F_1$ (C)-DNA/EcoRI

16 ng (= 0,84 pMol Enden) der chemisch synthetisierten $F_1$ (C)-DNA (siehe Beispiel 9) werden mit 5 Einheiten EcoRI Restriktionsendonuclease (Biolabs) in 50 µl 100 mM Tris · HCl (pH 7,5), 50 mM NaCl und 100 µg/ml Gelatine für 1 Stunde bei 37°C verdaut. Danach werden der Lösung 0,5 µg (= 1 pMol Enden) des linearisierten Vektors pBR322/EcoRI/BalI (Beispiel 11a) zugesetzt. Anschliessend wird das Enzym durch Erhitzen auf 65°C nach 10 min inaktiviert, die Lösung auf TNE eingestellt und mit Phenol/Chloroform extrahiert. Die DNA wird mit Alkohol präzipitiert. Die ausgefällte DNA wird unter Alkohol bei –20°C bis zur Weiterverarbeitung gelagert.

c) Ligierung der pBR322/EcoRI/BalI Vektor-DNA mit der $F_1$ (C)-DNA/EcoRI und Konstruktion des Plasmids pML87

Der in Beispiel 11b) erhaltene DNA-Niederschlag, welcher die beiden genannten DNA-Bruchstücke enthält, wird in 30 µl einer Lösung von 50 mM Tris · HCl (pH 7,8), 10 mM $MgCl_2$, 10 mM DTT, 0,5 mM ATP, 100 µg/ml Gelatine gelöst und mit 25 Einheiten/µl $T_4$ DNA-Ligase (Biolabs) bei 15°C für 16 Stunden behandelt. Auf diese Weise entsteht in der Lösung das rekombinierte Plasmid pML87, welches die $F_1$ (C)-DNA enthält.

d) Transformation von E. coli HB101 mit dem Plasmid pML87

Die für die Transformation notwendigen mit Calcium vorbehandelten *E. coli* HB101 Zellen werden, wie von Mandel et. al. (6) beschrieben, hergestellt.

Die unter c) erhaltene Lösung, welche das rekombinierte Plasmid pML87 enthält, wird für 10 min. auf 65°C erhitzt, um die $T_4$-DNA Ligase zu inaktivieren und anschliessend auf 37°C abgekühlt. 10 µl dieses Reaktionsgemisches werden zu 150 µl Calcium-behandelten *E. coli* HB101-Zellen in 10 mM $MgCl_2$ und 10 mM Tris · HCl (pH 7,5) in einem Gesamtvolumen von 200 µl gegeben.

Danach wird diese Mischung für 30 min. in Eis gekühlt, 2 min. auf 42°C erwärmt und dann 50 min. in 1 ml L-Medium (vgl. Beispiel 21) bei 37°C stehen lassen. Darauf wird die Mischung in Aliquoten à 0,2 ml auf 5 Agarplatten (McConkey-Agar, Difco), welche 60 µg/ml Ampicillin (Serva) enthalten, ausgestrichen. Die Agarplatten werden hernach bei 37°C 16 – 18 Stunden gehalten. 470 Ampicillin-resistente Kolonien des transformierten *E. coli* HB101 werden erhalten.

e) Screening der Kolonien, die $F_1$ (C)-DNA enthalten

470 transformierte Kolonien (Beispiel 11d) werden auf Nitrozellulosefilter B85 (Schleicher und Schüll) abgedrückt. Nach Grunstein und Hogness (24) werden die Kolonien lysiert und ihre denaturierte DNA auf dem Filter fixiert. Anschliessend erfolgt eine Vorhybridisierung der Filter in 20 ml (pro Filter) 4 x SET [= Lösung von 30 mM Tris · HCl (pH 8), 150 mM NaCl, 1 mM EDTA], 0,1 % (g/v) Ficoll 400 (Pharmacia), 0,5 % SDS, 50 µg/ml denaturierte Kalbsthymus-DNA für 4 Stunden bei 64°C. Danach werden die Nitrozellulosefilter in 20 ml (pro Filter) 5 x SET (g/v) Ficoll 400, 0,2 % SDS und 50 µg/ml denaturierter Kalbsthymus-DNA für 16 Stunden bei 64°C mit der $^{32}$p-radioaktiv markierten Probe (ca. $10^3$ – $10^4$ Cerencov cpm pro Filter) behandelt. Als Probe wird das Oligonucleotid 91/37 komplementär (C) (vgl. Beispiel 6) verwendet.

Anschliessend werden die Filter in 2 x SET, 0,2 % SDS bei Raumtemperatur zweimal gewaschen, danach

zweimal in 2 x SET, 0,5 % SDS bei 60°C (zuerst 30 min., dann 60 min. lang). Dann werden die Filter zwischen 3 MM Papier (Whatman) getrocknet und bei –80°C auf einen Röntgen-Film (Fuji) mit einer Verstärkerfolie (Intensifying screen, Ilford) für 1 – 2 Tage gelegt.

Das erhaltene Autoradiogramm zeigt 71 positive Kolonien (Klone), die zur Weiterarbeit verwendet werden können, eine davon erhält die Bezeichnung pML87.

**Beispiel 12**

**Herstellung des Plasmids pML90, enthaltend die $F_1$ (B)-DNA des Eglin B-Gens**

In analoger Weise, wie in Beispiel 11b) beschrieben, werden 16 µg der chemisch synthetisierten $F_1$ (B)-DNA mit 5 Einheiten EcoRI-Restriktionsendonuclease verdaut und mit dem linearisierten Vektor pBR322/EcoRI/BalI gemischt. Das Enzym wird inaktiviert und die DNA mit Alkohol präzipitiert. Der DNA-Niederschlag wird gemäss Beispiel 11c) mit $T_4$ DNA-Ligase behandelt, wobei ein Plasmid entsteht, welches die $F_1$ (B)-DNA enthält.

Die rekombinierte Plasmide enthaltende Lösung wird entsprechend Beispiel 11d) zur Transformation von Calcium-behandelten *E. coli* HB101-Zellen verwendet. 310 Ampicillin-resistente Kolonien des transformierten *E. coli* HB101 werden erhalten.

Die 310 Kolonien werden, in Analogie zu Beispiel 11e), auf das Vorhandensein von $F_1$ (B)-DNA geprüft, wobei als Probe das Oligonucleotid 91/37 komplementär (B) verwendet wird. Im erhaltenen Autoradiogramm sind 55 positive, zur Weiterarbeit verwendbare Klone erkennbar. Einer davon erhielt die Bezeichnung pML90.

**Beispiel 13**

**Herstellung des Plasmids pML136, welches die $F_2$-DNA enthält (Fig. 3)**

a) <u>Herstellung des linearisierten Vektors pBR322/BamHI/NruI</u>

15 µg pBR322 Plasmid-DNA werden mit 30 Einheiten BamHI Restriktionsendonuclease 30 min. bei 37°C in einer Lösung von 100 mM NaCl, 6 mM Tris · HCl (pH 7,9), 6 mM $MgCl_2$ und 100 µg/ml Gelatine verdaut. Anschliessend werden der Lösung 15 Einheiten NruI Restriktionsendonuclease zugefügt und 2 Stunden bei 37°C verdaut.

Das Reaktionsgemisch wird für 10 min. auf 70°C erwärmt, um die Enzyme zu inaktivieren. Danach werden die beiden DNA-Fragmente durch Gelelektrophorese an 1 %-er niedrigschmelzender Agarose in Tris-Acetat EDTA Puffer pH 8 voneinander getrennt. Nach Anfärben der DNA im Agarosegel mit EtBr wird die Stelle des Gels, welche die DNA-Bande des pBR322/BamHI/NruI Vektors (= 3766 Basenpaare) enthält, aus dem Gel ausgeschnitten und bei 65°C 10 min. verflüssigt. Anschliessend werden zu dem verflüssigten Agarosegelstück 2 Volumen 100 mM Tris · HCl (pH 8,7) gegeben und die Mischung auf 37°C abgekühlt. Diese DNA-Mischung wird mit 0,5 Einheiten alkalischer Phosphatase aus Kälberdarm (Boehringer) für 30 min. bei 37°C verdaut. Durch Erhitzen der Lösung auf 65°C für 60 min. wird das Enzym inaktiviert.

Zu dieser phosphatasebehandelten DNA-Lösung werden 20 Volumina TNE hinzugefügt und die DNA nach Müller et al. (23) durch DE-52 Chromatographie gereinigt, mit Phenol/Chloroform extrahiert und die DNA bei –20°C über Nacht mit Alkohol ausgefällt. Der DNA-Niederschlag wird in 50 µl 0,01 M Tris · HCl (pH 8), 0,1 mM EDTA gelöst und bis zur Verwendung bei –20°C aufbewahrt. 1,5 µg (= 2,4 pMol Enden) DNA werden erhalten.

b) <u>Herstellung der $F_2$-DNA/BamHI</u>

1,6 µg (= 90 pMol Enden) der chemisch synthetisierten $F_2$-DNA (Beispiel 8) werden mit 16 Einheiten BamHI Restriktionsendonuclease (Biolabs) in 20 µl 150 mM NaCl, 6 mM Tris · HCl (pH 7,9), 6 mM $MgCl_2$ und 100 µg/ml Gelatine für 30 min. bei 37°C verdaut. Anschliessend werden der Lösung 60 ng (= 96 nMol Enden) des linearisierten Vektors pBR322/BamHI/NruI (Beispiel 13a) zugesetzt, die ganze Lösung auf TNE eingestellt und mit Phenol/Chloroform extrahiert und die DNA mit 2 Volumen Alkohol ausgefällt. Die ausgefällte DNA wird bis zur Weiterverarbeitung unter Alkohol bei –20°C gelagert.

c) <u>Ligierung der pBR322/BamHI/NruI Vektor-DNA mit der $F_2$-DNA/BamHI und Konstruktion des Plasmids pML136</u>

Der unter Beispiel 13b) erhaltene DNA-Niederschlag, der die beiden genannten DNA-Bruchstücke enthält, wird in 20 µl einer Lösung von 50 mM Tris · HCl (pH 7,8), 10 mM $MgCl_2$, 10 mM DTT, 0,5 mM ATP, 100 µg/ml Gelatine gelöst und mit 15 Einheiten/µl $T_4$ DNA-Ligase (Biolabs) bei 15°C für 3 Stunden behandelt. Auf diese Weise entsteht in der Lösung das rekombinierte Plasmid pML136, welches die $F_2$-DNA enthält.

d) <u>Transformation von E. coli HB101 mit dem Plasmid pML136</u>

Die Transformation der mit Calcium behandelten *E. coli* HB101 Zellen erfolgt wie in Beispiel 11d) beschrieben. Es

werden 10 µl des in Beispiel 13c) erhaltenen Reaktionsgemisches verwendet. 65 Ampicillin-resistente Kolonien werden erhalten.

e) Screening der Kolonien, die $F_2$-DNA enthalten

65 transformierte Kolonien (Beispiel 13d) werden auf $F_2$-DNA geprüft, wie in Beispiel 11e) beschrieben. Als radioaktive Probe wird das Oligonucleotid 172/61 komplementär (vgl. Beispiel 5) verwendet. Im Autoradiogramm werden 2 positive Kolonien erhalten, eine davon erhält die Bezeichnung pML136.

**Beispiel 14**

**Charakterisierung der Klone pML87, pML90 und pML136**

Die DNAs der rekombinierten Plasmide pML87, pML90 und pML136 werden nach Ish-Horowitz isoliert (25). Die Nucleotidsequenzen des $F_1$ (C)-DNA-, $F_1$ (B)-DNA- und $F_2$-DNA-Inserts werden nach Maxam und Gilbert bestimmt (3). Zu diesem Zweck werden je 10 µg Plasmid-DNA von pML87 und pML90 mit EcoRI-Restriktionsendonuclease und 10 µg Plasmid-DNA von pML136 mit BamHI-Restriktionsendonuclease gespalten und die linearisierten DNAs durch Gelelution aus Agarosegel isoliert [vgl. Beispiele 11a) bzw. 13a)]. Anschliessend werden die isolierten DNAs mit alkalischer Phosphatase verdaut und über DE-52 chromatographiert (vgl. Beispiel 13a). Danach werden die DNAs am 5'-Ende mit [$\lambda$-$^{32}$P]ATP (spezifische Aktivität > 5000 Ci/mmol, Amersham) und $T_4$-Polynucleotid-Kinase (P-L-Biochemicals) radioaktiv markiert.

Die radioaktiv markierten DNAs werden dann mit einer zweiten Restriktionsendonuclease (PvuII) gespalten. Die entstandenen DNA-Fragmente werden durch Gelelution aus Agarose isoliert. Im Falle von pML87 und pML90 wird dann vom PvuII-EcoRI* Fragment (ca. 2190 Basenpaare) die Nucleotidsequenz der $F_1$ (C)- bzw. $F_1$ (B)-DNA, im Fall vom pML136 die der $F_2$-DNA im PvuII-BamHI*-Fragment (ca. 1815 Basenpaare) bestimmt.
(* gibt das DNA-Ende an, das radioaktiv markiert ist).

Die Nucleotidsequenzen, welche für die $F_1$ (C)-DNA, $F_1$ (B)-DNA und $F_2$-DNA bestimmt werden, sind identisch mit den in den Beispielen 8 – 10 dargestellten.

**Beispiel 15**

**Herstellung des Plasmids pML141, enthaltend die $F_1$ (C)-$F_2$-DNA (Fig. 4)**

a) Herstellung des linearisierten Vektors pBR322/EcoRI/BamHI

10 µg pBR322 Plasmid-DNA werden mit je 10 Einheiten EcoRI- und BamHI-Restriktionsendonuclease (Biolabs) in 100 µl einer Lösung von 50 mM Tris · HCl (pH 7,5), 50 mM NaCl, 6 mM $MgCl_2$ und 100 µg/ml Gelatine für 1 Stunde bei 37°C verdaut. Anschliessend wird diese Lösung auf TNE eingestellt, mit 1 Volumen Phenol und Chloroform extrahiert und die DNA mit 2 Volumen Alkohol bei –20°C über Nacht ausgefällt.

Der aus der pBR322-DNA herausgeschnittene Vektor (pBR322/EcoRI/Ball, 3986 Basenpaare) wird durch Dichtegradientenzentrifugation in Sucrose (5 – 23 %) in 50 mM Tris · HCl (pH 8) und 1 mM EDTA vom kleineren DNA-Fragment (376 Basenpaare) abgetrennt. Die Zentrifugation wird bei 30.000 upm in einem TST 41 Rotor (Kontron AG) für 15 Stunden bei 15°C durchgeführt. Anschliessend werden von der zentrifugierten Lösung Fraktionen à 0,2 ml mit einem ISCO Gradienten-Sammler gewonnen. Diejenigen Fraktionen, die das grosse DNA-Bruchstück (3986 Basenpaare) enthalten werden vereinigt und die DNA mit Alkohol ausgefällt. Der Niederschlag wird in 100 µl 50 mM Tris · HCl (pH 8) mit 0,3 Einheiten alkalischer Phosphatase aus Kälberdarm (Boehringer) für 30 min. bei 37°C verdaut. Durch Erhitzen der Lösung auf 65°C für 1 Stunde wird das Enzym inaktiviert. Anschliessend wird die Lösung mit Phenol/$CHCl_3$ extrahiert und die DNA über Nacht bei –20°C mit Alkohol ausgefällt. Der Niederschlag wird in 50 µl 10 mM Tris · HCl (pH 8), 0,1 mM EDTA gelöst und bis zu seiner Verwendung als Klonier-Vektor bei –20°C aufbewahrt. 3,75 µg DNA (= 5,7 pMol Enden) werden erhalten.

b) Herstellung der $F_1$ (C)-DNA/EcoRI/HpaII und der $F_2$-DNA/BamHI/HpaII

I. Herstellung der $F_1$ (C)-DNA/EcoRI/HpaII

10 µg Plasmid DNA vom pML87 werden zuerst mit 20 Einheiten HpaII Restriktionsendonuclease in 100 µl einer Lösung von 10 mM Tris · HCl (pH 7,4), 6 mM KCl, 10 mM $MgCl_2$, 1 mM DTT und 100 µg/ml Gelatine verdaut. Es folgt eine Phenol/Chloroform Extraktion der Lösung und Ausfällung der entstandenen DNA-Fragmente mit Alkohol bei –20°C.

Das DNA-Fragmentgemisch wird anschliessend durch Elektrophorese an einem 6 % Polyacrylamidgel in Tris-acetat-EDTA Puffer pH 8 aufgetrennt. Das grösste DNA-Fragment (= 586 Basenpaare) wird durch Gelelution isoliert und anschliessend mit EcoRI-Restriktionsendonuclease gespalten (vgl. Beispiel 11a). Das entstandene DNA-Fragmentgemisch wird abermals einer Elektrophorese an 8 % Polyacrylamid unterworfen. Daraus werden 40 ng von $F_1$ (C)-DNA/EcoRI/HpaII (127 Basenpaare) isoliert.

II) Herstellung der $F_2$-DNA/BamHI/HpaII

20 µg Plasmid DNA vom pML136 werden mit 20 Einheiten BamHI Restriktionsendonuclease gespalten. Ein Aliquot (1 µg) dieser linearisierte Plasmid DNA/BamHI wird durch Gelelution aus einem Agarosegel isoliert (vgl. Beispiel 13a) und mit [$\lambda$-$^{32}$P]ATP radioaktiv markiert (vgl. Beispiel 14). Die Hauptmenge der Plasmid DNA/BamHI wird dann mit dieser radioaktiv markierten DNA vermischt, mit PvuII Restriktionsendonuclease verdaut und das PvuII-BamHI* DNA Fragment (1203 Basenpaare) nach Gelelektrophorese an 1 % Agarose isoliert. 14 µg des PvuI-BamHI* Fragments werden mit HpaII Restriktionsendonuclease verdaut (siehe oben), anschliessend das DNA-Gemisch durch 8 % Polyacrylamidgel-elektrophorese aufgetrennt und 150 ng der $F_2$-DNA/BamHI*/HpaII (109 Basenpaare) durch Gelelution isoliert.

c) Ligation der $F_1$ (C)-DNA mit der $F_2$-DNA und Konstruktion des Plasmids pML141

10 ng (= 473 nMol Enden) $F_1$ (C)-DNA/EcoRI/HpaII und 9 ng (= 495 nMol Enden) $F_2$-DNA/BamHI/HpaII werden in einem Volumen von 20 µl mit $T_4$-DNA Ligase, wie bereits unter Beispiel 13c) beschrieben, behandelt. Anschliessend wird die Mischung mit Phenol/Chloroform extrahiert und die DNA mit Alkohol präzipitiert. Der DNA-Niederschlag wird dann wie in Beispiel 13a) beschrieben gelöst und mit EcoRI- und BamHI-Restriktionsendonuclease verdaut. Die Lösung wird dann auf TNE eingestellt und 30 ng (= 50 nMol Enden) der Vektor-DNA pBR322/EcoRI/BamHI (vgl. Beispiel 15a) zugegeben. Anschliessend wird die Lösung abermals mit Phenol/Chloroform extrahiert und die DNA mit Alkohol ausgefällt. Die präzipitierte DNA-Mischung wird wie in Beispiel 13c) angegeben mit $T_4$-DNA Ligase (Biolabs) behandelt. Auf diese Weise entstehen in der Lösung rekombinierte Plasmide, welche die $F_1$ (C)-$F_2$-DNA (Eglin C-Gen) als Insert enthalten.

d) Transformation von E. coli HB101 mit dem Plasmid pML141

Die Transformation der mit Calcium behandelten *E. coli* H8101 Zellen erfolgt wie in Beispiel 11d) beschrieben. Es werden 10 µl des in Beispiel 15c) erhaltenen Reaktionsgemisches verwendet. 2586 Ampicillinresistente Kolonien werden erhalten.

e) Screening der Kolonien, die $F_1$ (C)-$F_2$-DNA enthalten

18 transformierte Kolonien (Beispiel 15d) werden auf ihren $F_1$ (C)-$F_2$-DNA-Gehalt geprüft, wie in Beispiel 11e) beschrieben. Als radioaktive Probe wird eine Mischung der in den Beispielen 5 und 6 beschriebenen Oligonucleotide verwendet. Im Autoradiogramm werden 13 positive Kolonien erhalten, vier davon erhalten die Bezeichnung pML141, pML143, pML144, pML145.

**Beispiel 16**

**Herstellung des Plasmids pML160, enthaltend die $F_1$ (B)-$F_2$-DNA**

a. Herstellung der $F_1$ (B)-DNA/EcoRI/HpaII

In analoger Weise, wie für die $F_1$ (C)-DNA/EcoRI/HpaII beschrieben (Beispiel 15bI), werden 10 µg Plasmid-DNA von pML90 zuerst mit HpaII und danach mit EcoRI gespalten. Das Fragmentgemisch wird, wie beschrieben, durch PAGE gereinigt.

b. Ligation der $F_1$ (B)-DNA mit der $F_2$-DNA und Konstruktion eines rekombinierten Plasmids

Die Ligation erfolgt, wie in Beispiel 15c beschrieben, ausgehend von 10 µg $F_1$ (B)-DNA/EcoRI/HpaII (s.o.) und 9 µg $F_2$-DNA/BamHI/HpaII (Beispiel 15bII). Die gebildete $F_1$ (B)-$F_2$-DNA/EcoRI/BamHI wird, wie beschrieben, mit 30 µg der Vektor-DNA pBR322/EcoRI/BamHI (vgl. Beispiel 15a) ligiert.

Die entstandene, rekombinierte Plasmide enthaltende Lösung wird zur Transformation von mit Calcium-behandelten *E. coli* HB101-Zellen verwendet. 15 transformierte Klone werden auf ihren $F_1$ (B)-$F_2$-DNA-Gehalt geprüft, wie in Beispiel 11e) beschrieben. Als radioaktive Probe wird wieder eine Mischung der in den Beispielen 5 und 6 beschriebenen Oligonucleotide benutzt. In Autoradiogramm werden 6 positive Kolonien erhalten, eine davon erhält die Bezeichnung pML160.

**Beispiel 17**

**Charakterisierung der Klone pML141 und pML160**

Je 10 µg der Plasmid-DNAs von pML141 und pML160 werden jeweils mit EcoRI- bzw. BamHI-Restriktionsendonuclease verdaut (vgl. Beispiel 11a bzw. 13a). Die Charakterisierung von pML141 und pML160 erfolgt wie bereits in Beispiel 14 beschrieben.

Die für die $F_1$ (C)-$F_2$-DNA und $F_1$ (B)-$F_2$-DNA ermittelten Nucleotid-Sequenzen sind mit denjenigen der oben

dargestellten synthetischen Eglin C- und Eglin B-Gene identisch.

**Beispiel 18**

**Herstellung des Expressionsplasmids pML147**

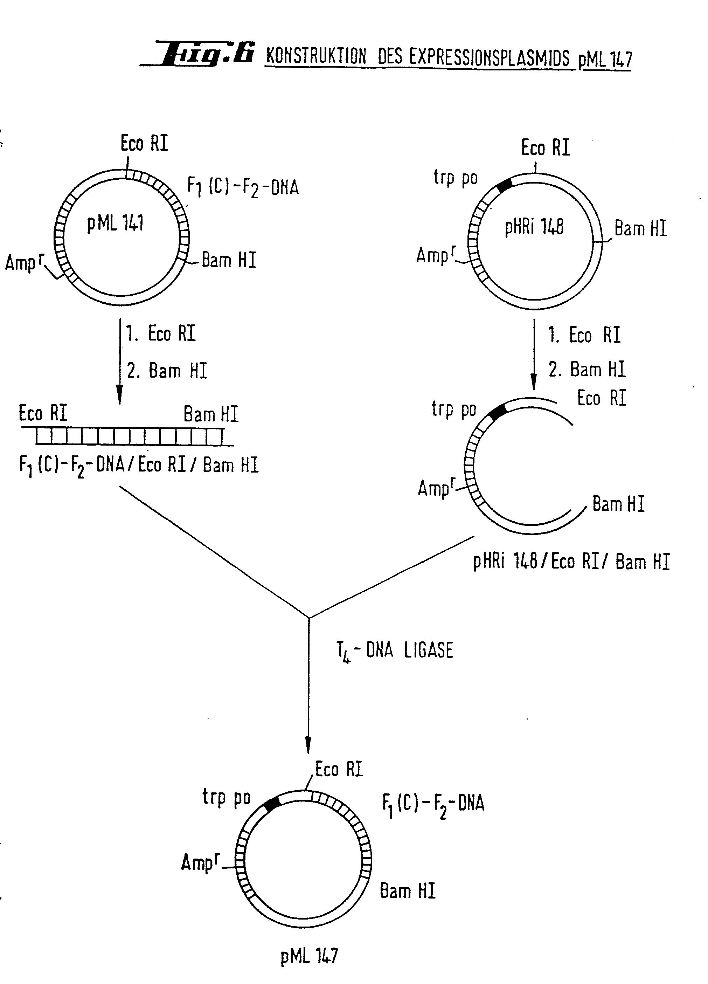

a) Konstruktion des linearisierten Vektors pHRi148/EcoRI/BamHI, welcher den trp-Promotor-Operator enthält (Fig. 5 und Fig. 6)

**A. Konstruktion des Plasmids p159**

10 µg Plasmid $pBRH_{trp}$ (21) werden mit 50 Einheiten EcoRI (Biolabs) gespalten während 60 min. bei 37°C, und das Verdauungsgemisch wird nach Phenolextraktion durch einen Saccharose-Dichtegradienten (5 – 23 %) in 50 mM Tris · HCl (pH 8,0), 1 mM EDTA in einem TST41 (Kontron AG) Rotor fraktioniert. Die Zentifugation dauert 14 Stunden bei 40 000 upm und 15°C. 0,3 ml Fraktionen werden mit einem ISCO-Gradientkollektor bei 1 ml/min. gesammelt. Die Fraktionen, welche das kleinere Fragment enthalten, werden vereinigt, die Lösung wird auf TNE eingestellt und mit 2 Volumen Ethanol bei –20°C gefällt. Die DNA wird nach Zentrifugation in einer Eppendorf-Zentrifuge in 100 µl 10 mM Tris · HCl pH 7,5, 0,5 mM EDTA gelöst. 5 µg dieses DNA-Fragmentes werden mit 5 Einheiten BglII (Biolabs) 60 min. bei 37°C gespalten. Das Reaktionsgemisch wird mit Phenol und Chloroform extrahiert und die DNA wird mit 2 Volumina Ethanol bei –80°C während 10 min. inkubiert und die DNA durch Zentrifugation gesammelt und erneut in 50 µl 50 mM Tris · HCl (pH 8,0) gelöst. 2 µl von dieser Lösung werden entnommen (0,2 µg DNA) und bei einer DNA-Konzentration von 10 ng/µl in 50 mM Tris · HCl (pH 8,0) mit 1 Einheit intestinaler alkalischer Kälber-Phosphatase (Boehringer) während 30 min. bei 37°C inkubiert. Das Enzym wird durch Erhitzen der Lösung während 60 min. bei 65°C inaktiviert. 0,04 µg DNA wird entnommen und 5'-terminal mit 10 µCi [$\lambda$-$^{32}P$]-ATP (> 5000 Ci/mmol, Amersham) und 5 Einheiten $T_4$ Polynucleotid-Kinase (P-L Biochemicals) in 20 µl Reaktionsvolumen in 50 mM Tris · HCl (pH 9,5), 10 mM $MgCl_2$, und 5 mM DTT, während 30 min. bei 37°C inkubiert. Die radioaktive Probe wird mit der nicht-markierten Probe (siehe oben) gemischt und die DNA-Fragmente werden durch einen 5 – 23 % Saccharose-Dichtegradienten in 50 mM Tris · HCl (pH 8,0), 1 mM EDTA in einem TST60 Rotor fraktioniert. Die Zentrifugation erfolgt während 5 Stunden bei 60 000 upm und 15°C. Es werden 0,2 ml Fraktionen gesammelt. Die Radioaktivität jeder Fraktion wird durch Messung der Cerencov-Strahlung bestimmt und die Fragmente damit identifiziert. Die gewünschten Fraktionen, welche das kleine DNA-Fragment enthalten, werden vereinigt, die DNA mit 2 Volumina Ethanol gefällt und nach Zentrifugation wieder in 20 µl 10 mM Tris · HCl pH 7,5, 0,5 mM EDTA gelöst.

Das $^{32}P$-markierte EcoRI-BglII DNA-Fragment wird mit 0,2 Einheiten TaqI (Biolabs) in 50 µl Volumen während 10 min. bei 37°C partiell gespalten. Das Reaktionsgemisch wird auf 0,2 % SDS, 10 % Glycerin, 10 mM EDTA, 0,05 % Bromphenol-Blau eingestellt und die DNA-Fragmente auf einem 6 % Polyacrylamidgel in Tris-Borat-EDTA (22) aufgetrennt. Auf dem Autoradiogramm wird die das gewünschte EcoRI-TaqI enthaltende Bande (das grösste Teilfragment) identifiziert. Dieses Fragment (L, siehe Fig. 5) wird aus dem Gel extrahiert und gereinigt (23) und in 10 µl 10 mM Tris · HCl pH 7,5, 1 mM EDTA gelöst.

Als Akzeptor-Plasmid wird pBR322, welches mit ClaI und EcoRI gespalten ist, verwendet: 2 µg pBR322 werden mit 4 Einheiten ClaI (Biolabs) in 20 µl Reaktionsvolumen während 60 min. bei 37°C verdaut. Das Protein wird mit Phenol extrahiert und die DNA anschliessend mit 2 Volumina Ethanol bei –80°C während 10 min. gefällt. Die DNA wird durch Zentrifugation gesammelt und dann mit 10 Einheiten EcoRI (Biolabs) während 30 min. bei 37°C in 20 µl Reaktionsvolumen verdaut. Anschliessend wird die Lösung mit 2 Volumina 0,1 M Tris · HCl (pH 8,7) versetzt und mit 1 Einheit alkalischer Kälber-Phosphatase (Boehringer) während 30 min. bei 37°C inkubiert. Die Phosphatase wird anschliessend durch Inkubation bei 65°C während 60 min. inaktiviert.

100 ng des Akzeptor-Plasmids werden mit 5 µl Fragment L-DNA in 15 µl Reaktionsvolumen in 10 mM $MgCl_2$, 20 mM Tris · HCl (pH 7,8), 10 mM DTT, 0,5 mM ATP mit 30 Einheiten pro µl Reaktionsvolumen $T_4$ DNA-Ligase (Biolabs) während 2 Stunden inkubiert.

5 µl dieser Lösung werden zu einem Gemisch gegeben, das 150 ml mit Calciumchlorid behandelte *E. coli* HB101-Zellen (6) in 10 mM $MgCl_2$, 10 mM $CaCl_2$ und 10 mM Tris · HCl (pH 7,5) in einem Gesamtvolumen von 200 µl enthält. Das Gemisch wird 20 min. in Eis gekühlt, 1 min. auf 42°C erhitzt und 10 min. bei 20°C inkubiert. 1 ml Trypton-Medium [Trypton-Medium enthält 10 g Bacto-Trypton (Difco); 1 g Hefeextrakt (Difco); 1 g Glucose; 8 g NaCl und 294 mg $CaCl_2 \cdot 2H_2O$ in 1 l destilliertem Wasser] wird zugesetzt und das Gemisch 30 min. bei 37°C unter Schütteln bei 300 U/min. inkubiert. Die Mischung wird auf zwei Agarplatten (McConkey Agar, Difco; 0,6 ml/Platte), supplementiert mit 50 µg/ml Ampicillin (Sigma), plattiert. Die Platten werden 12 bis 17 Stunden bei 37°C inkubiert.

Die Plasmid-DNA von 10 verschiedenen Kolonien wird wie folgt isoliert:
Die Kolonien werden zur Inokulierung von 10 ml Trypton-Medium, supplementiert mit 50 µg/ml Ampicillin, wie oben, in einem 25 ml Erlenmeyerkolben verwendet. Die Kulturen werden 15 bis 18 Stunden bei 37°C und 300 U/min. geschüttelt. Die Zellen werden durch Zentrifugieren (Sorval, HS-4 Rotor, 10 min. bei 4000 U/min., 4°C) geerntet. Man erhält etwa 0,1 g Zellen, und diese werden in 1 ml 50 mM Tris · HCl (pH 8,0) resuspendiert. 0,25 ml Lysozymlösung [10 mg/ml in 50 mM Tris · HCl (pH 8,0); Lysozym wird von Sigma vertrieben] werden zugesetzt und nach 10 minütiger Inkubation bei 0°C werden 0,15 ml 0,5 mM EDTA (pH 7,5) zugegeben. Nach weiteren 10 min. bei 0°C werden 60 µl 2 %-iges Triton X-100 (Merck) zugegeben. Nach 30 min. bei 0°C wird

die Probe 30 min. bei 15'000 U/min. und 4°C in einem Sorval SA-600 Rotor zentrifugiert. Der Überstand wird mit 1 Vol. Phenol (gesättigt in TNE) deproteinisiert. Die Phasen werden durch Zentrifugieren (Sorval HB-4 Rotor) während 10 min. bei 5000 U/min. und 4°C getrennt. Die obere Phase wird zweimal mit 1 Vol. Chloroform extrahiert. Pankreatische RNAse A (Sigma; 10 mg/ml in TNE 10 min. bei 85°C vorerhitzt) wird bis zu einer Endkonzentration von 25 µg/ml zugegeben und das Gemisch 40 min. bei 37°C inkubiert. Die Lösung wird dann auf 1 M NaCl und 10 % Polyethylenglykol 6000 (Fluka, 20 min. bei 120°C im Autoklaven behandelt) eingestellt und 2 Stunden bei –10°C inkubiert. Das Präzipitat wird in einem Sorval HB-4 Rotor (20 min. bei 10 000 U/min., 0°C) gesammelt und erneut in 100 µl TNE gelöst. Die DNA-Lösung wird mit 1 Vol. Phenol extrahiert und die DNA wird mit 2 Vol. Ethanol 10 min. bei –80°C präzipitiert. Das Präzipitat wird durch Zentrifugieren in einer Eppendorf-Zentrifuge gesammelt und die DNA erneut in 20 µl 10 mM Tris · HCl (pH 7,5) und 0,5 mM EDTA gelöst. Aus einer 10 ml Kultur gewinnt man 8 bis 10 µg Plasmid-DNA.

Die Plasmid-DNAs werden nach Verdauung mit den folgenden Restriktionsenzymen analysiert:

Je 0,5 µg Plasmid-DNA wird mit HpaI (Biolabs) sowie mit HpaI (Biolabs) und EcoRI (Biolabs) mit ClaI (Biolabs) nach Standard-Vorschriften, gemäss Angaben des Enzym-Herstellers, gespalten. Die DNAs werden auf einem 1 % Agarose-Gel in 40 mM Tris · Acetat (pH 7,8) 1 mM EDTA und 0,5 µg/ml Ethidiumbromid fraktioniert. Die gewünschten Plasmide enthalten eine HpaI-Stelle und ergeben nach der 3-fachen Verdauung neben dem grossen DNA-Fragment 2 kleinere Fragmente, welche grösser sind als das kleine EcoRI-ClaI-Fragment von pBR322. Eines dieser Plasmide wird mit p159 bezeichnet (siehe Fig. 5).

**B. Konstruktion des Plasmids pHRi145**

2 µg p159-DNA werden mit 10 Einheiten EcoRI (Biolabs) während 30 min. bei 37°C verdaut. Die DNA wird mit Phenol extrahiert, mit Ethanol gefällt und nach Zentrifugation in 10 µl 10 mM Tris · HCl (pH 7,5), 0,5 mM EDTA gelöst. Die mit EcoRI verdaute DNA wird weiterhin mit 5 Einheiten DNA-Polymerase (Klenow-Fragment) (Boehringer) in 10 mM $MgCl_2$, 10 mM ß-Mercaptoethanol, 50 mM NaCl, 0,1 mM dATP (P&L Biochemicals). 0,1 mM dTTP (P&L Biochemicals) während 15 min. bei 12°C behandelt. Die Polymerase wird anschliessend durch Inkubation bei 85°C während 5 min. inaktiviert. Das Reaktionsgemisch wird in 20 mM Tris · HCl (pH 7,8), 10 mM $MgCl_2$, 10 mM DTT, 0,5 mM ATP (Sigma) auf das 10-fache verdünnt und mit 30 Einheiten $T_4$ DNA-Ligase pro µl Reaktionsgemisch während 1 Stunde bei 15°C inkubiert.

50 ng der DNA werden in *E. coli* transformiert (wie oben beschrieben) und auf McConkey-Agarplatten supplementiert mit 50 µg/ml Ampicillin, ausplattiert.

Die Plasmid-DNAs von 10 verschiedenen Kolonien werden, wie oben beschrieben, isoliert. Die Plasmid-DNAs werden analysiert durch Verdauung mit EcoRI. Die gewünschten Plasmide sind EcoRI-resistent. Die Analyse erfolgt wie oben beschrieben. Eines der gewünschten Plasmide wird als HRi145 bezeichnet (Fig. 5).

**C. Konstruktion des Plasmids pHRi148**

2 µg pHRi145-DNA werden mit 5 Einheiten ClaI (Boehringer) während 60 min. bei 37°C behandelt, dann mittels Phenol-Extraktion deproteiniert. Die DNA wird mit Ethanol gefällt und dann in 20 µl 10 mM Tris · HCl (pH 7,5), 0,5 mM EDTA gelöst. Die überhängenden Enden werden, wie oben beschrieben, mit DNA Polymerase I (Klenow-Fragment) aufgefüllt, mit der Änderung, dass dATP und dTTP ersetzt werden durch dCTP (P&L Biochemicals) und dGTP (P&L Biochemicals). Die Polymerase wird inaktiviert durch Inkubation bei 85°C während 5 min. Das Reaktionsgemisch wird mit 2 Volumina 0,1 M Tris · HCl (pH 8,7) versetzt und mit 0,5 Einheiten Kälber-Phosphatase (Boehringer) während 30 min. bei 37°C inkubiert. Das Reaktionsgemisch wird deproteiniert durch Phenol-Extraktion. Die DNA wird mit Ethanol gefällt und in 8 µl 10 mM Tris · HCl (pH 7,5), 0,5 mM EDTA gelöst.

Ein chemisch synthetisierter DNA-Linker der Formel

5'-GAATTCCATGGTACCATGGAATTC-3'

wird am 5'-Ende phosphoryliert, indem 8 pMol des Linkers mit 5 µCi [$\lambda$-$^{32}$P]-ATP (5500 Ci · $mmol^{-1}$ Amersham) in 8 µl Reaktionsvolumen, welches 0,1 mM rATP (Sigma), 50 mM Tris · HCl (pH 9,5), 10 mM $MgCl_2$, 5 mM DTT und 2 Einheiten $T_4$ Polynucleotid-Kinase (P&L Biochemicals) enthält, während 30 min. bei 37°C inkubiert werden. Die Reaktion wird gestoppt durch Einfrieren bei –80°C.

Der radioaktiv markierte Linker wird anschliessend mit 1 µg ClaI und Phosphatase behandelt und mit pHRi145-DNA (siehe oben) in einem 20 µl Reaktionsvolumen ligiert, welches 0,5 mM rATP (Sigma), 10 mM DTT (Calbiochem), 20 mM Tris · HCl (pH 7,8), 1 mM $MgCl_2$ und 800 Einheiten $T_4$ DNA-Ligase (Biolabs) enthält. Die Inkubation erfolgt bei 15°C während 2 Stunden. Die Ligase wird inaktiviert durch Inkubation bei 85°C während 10 min. Anschliessend werden 2 Volumina Wasser hinzugegeben, die Kochsalz-Konzentration auf 10 mM eingestellt und 20 Einheiten KpnI (Biolabs) hinzugegeben während 30 min. bei 37°C. Nach der Extraktion mit Phenol und Chloroform wird die Mischung durch ein 0,9 % niedrigschmelzendes Agarose-Gel (Biorad) in 40 mM Tris · Acetat (pH 7,8), 1 mM EDTA und 0,5 µg/ml Ethidiumbromid fraktioniert. Die durch UV-Bestrahlung

sichtbare Bande, welche die gleiche Mobilität wie eine Marker-DNA gleicher Grösse aufzeigt, wird mit einem Skalpell herausgeschnitten. Das Gelstück wird 5 min. bei 65°C geschmolzen und dann auf 37°C abgekühlt. Man erhält ein Volumen von ca. 20 µl. 5 µl dieser Lösung werden entnommen und mit 400 Einheiten $T_4$-Ligase (Biolabs) in 10 µl Reaktionsvolumen, welches auf 0,5 mM ATP, 10 mM DTT, 10 mM $MgCl_2$, 20 mM Tris · HCl (pH 7,8) eingestellt wird, während 12 Stunden bei 15°C inkubiert. 1/10 Volumen einer Lösung mit 100 mM Tris · HCl (pH 7,5), 100 mM $CaCl_2$ und 100 mM $MgCl_2$ werden zum Ligase-Gemisch (solifidiziert bei 15°C) hinzugegeben und bei 65°C während 5 min. inkubiert. Die Lösung wird dann verwendet, um mit Calcium behandelte *E. coli* HB101-Zellen, wie oben beschrieben, zu transformieren. Es wird auf McConkey-Agarplatten, supplementiert mit 50 µg/ml Ampicillin, ausplattiert.

Die Plasmid-DNAs von 10 verschiedenen Kolonien werden isoliert, wie oben beschrieben, und die DNA wird der folgenden Restriktionsenzymanalyse unterworfen: Je 0,5 µg Plasmid DNA wird nacheinander mit KpnI (Biolabs), NcoI (Biolabs) und EcoRI (Biolabs) nach den Vorschriften des Enzym-Herstellers gespalten. Die Spaltprodukte werden auf 1 % Agarose-Gelen in 40 mM Tris · Acetat (pH 7,8), 1 mM EDTA, 0,5 µg/ml Ethidiumbromid fraktioniert. Alle Plasmide zeigen je eine dieser Enzymspaltstellen, wie gewünscht. Eines wird als HRi148 bezeichnet.

Das Plasmid HRi148 enthält einen Tryptophan-Promoter-Operator und eine ribosomale Bindungsstelle bis und mit ATG. Eglin C als auch andere heterologe Gene können direkt über die einmalig im Plasmid vorhandenen EcoRI, NcoI, KpnI-Stellen angekoppelt werden. Desweiteren ermöglicht diese Konstruktion das direkte Koppeln und Exprimieren von heterologen Genen, ohne dass das für die Initiation der Translation notwendige ATG auf dem entsprechenden Gen vorhanden sein muss. Dies kann leicht erreicht werden durch Spaltung mit NcoI und Auffüllen der überhängenden Enden mit DNA-Polymerase I, wie beschrieben, oder durch Spalten mit KpnI und Entfernen der überhängenden Enden durch Nuklease $S_1$. Das Plasmid HRi148 ist somit ein breit anwendbares Expressionsplasmid.

**D. Herstellung des linearisierten Vektors pHRi148/EcoRI/BamHI**

5 µg Plasmid-DNA von pHRi148 werden mit den Restriktionsendonucleasen EcoRI und BamHI, wie in Beispiel 15a beschrieben, verdaut. Der herausgeschnittene Vektor pHRi148/EcoRI/BamHI wird mittels Dichtegradientenzentrifugation (vgl. Beispiel 15a) isoliert.

b) Herstellung der $F_1$ (C)-$F_2$-DNA/EcoRI/BamHI (Fig. 6)

5 µg Plasmid-DNA von pML141 werden mit EcoRI- und BamHI-Restriktionsendonuclease wie in den Beispielen 11a) und 13a) beschrieben verdaut. Nach Phenol/Chloroform-Extraktion und Alkoholfällung wird die $F_1$ (C)-$F_2$-DNA/EcoRI/BamHI vom Plasmid (pBR322/EcoRI/BamHI) durch Gelelektrophorese an 1 %-iger niedrigschmelzender Agarose (Biorad) (Beispiel 13a) abgetrennt und mit EtBr sichtbar gemacht. Anschliessend wird die Stelle des Gels, welche die DNA Bande der $F_1$ (C)-$F_2$-DNA (= 236 Basenpaare) enthält, aus dem Gel geschnitten und bei 65°C 10 min. verflüssigt.

c) Ligierung der pHRi148/EcoRI/BamHI Vektor-DNA mit der $F_1$ (C)-$F_2$-DNA/EcoRI/BamHI und Konstruktion des Plasmids pML147 (Fig. 6)

100 ng (ca. 100 nMol Enden) Plasmid DNA pHRi148/EcoRI/BamHI und 28 ng (713 nMol Enden) der $F_1$ (C)-$F_2$-DNA/EcoRI/BamHI (gelöst in 10 µl des in Beispiel 18b) erhaltenen flüssigen Gels) werden bei 37°C in einem Volumen von 20 µl miteinander gemischt und wie in Beispiel 13c) beschrieben, mit $T_4$-DNA Ligase bei 15°C für 16 Stunden behandelt. Auf diese Weise entsteht in dieser Mischung das Expressionsplasmid pML147, welches das Eglin C-Gen ($F_1$ (C)-$F_2$-DNA) enthält.

d) Transformation von E. coli HB101 mit dem Plasmid pML147

10 µl des das Plasmid pML147 enthaltenden Gemisches (Beispiel 18c) werden für 10 min. bei 65°C verflüssigt und für die Transformation der Calcium-behandelten *E. coli* HB101 Zellen verwendet. Ca. 6000 Ampicillin-resistente Kolonien werden erhalten.

e) Screening der Kolonien, die $F_1$ (C)-$F_2$-DNA enthalten

Transformierte Kolonien (Beispiel 18d) werden auf das Vorhandensein von $F_1$ (C)-$F_2$-DNA geprüft, wie in Beispiel 15e) angegeben.

Es werden sieben positive Kolonien erhalten, welche die Bezeichnung pML147 – pML153 erhalten.

**Beispiel 19**

**Herstellung des Expressionsplasmids pML 199**

a. Herstellung der $F_1$ (B)-$F_2$-DNA/EcoRI/BamHI

Analog Beispiel 18b) werden 5 µg Plasmid-DNA von pML160 mit den Restriktionsendonucleasen EcoRI und BamHI verdaut. Die $F_1$ (B)-$F_2$-DNA/EcoRI/BamHI wird, wie beschrieben, mittels Gelelektrophorese abgetrennt.

b. Ligation der pHRi148/EcoRI/BamHI-Vektor-DNA mit der $F_1$ (B)-$F_2$-DNA/EcoRI/BamHI und Konstruktion von rekombinierten Plasmiden

100 µg Plasmid-DNA pHRi148/EcoRI/BamHI (vgl. Beispiel 18aD) wird mit 28 µg $F_1$ (B)-$F_2$-DNA/EcoRI/BamHI gemäss Beispiel 18c) ligiert. Die entstandene Lösung, welche rekombinierte Plasmide enthält, wird benutzt, um Calcium-behandelte *E. coli* HB101-Zellen zu transformieren. Transformierte Kolonien werden auf das Vorhandensein von $F_1$ (B)-$F_2$-DNA geprüft, wie in Beispiel 15e) beschrieben.

Es werden sechs positive Kolonien erhalten, welche die Bezeichnung pML199 – 204 erhalten.

**Beispiel 20**

**Charakterisierung der Klone pML147 und pML199**

Die Charakterisierung der $F_1$ (C)-$F_2$- bzw. $F_1$ (B)-$F_2$-DNA-Sequenzen in den rekombinierten Plasmiden pML147 und pML199 erfolgt durch Sequenzierung der $F_1$ (C)-$F_2$- bzw. $F_1$ (B)-$F_2$-DNA nach Maxam und Gilbert (3), wie in Beispiel 17 beschrieben. 10 µg Plasmid-DNA werden geprüft. Die Nucleotidsequenz der $F_1$ (C)-$F_2$-DNA ist mit der für das synthetische Eglin C-Gen beschriebenen, diejenige der $F_1$ (B)-$F_2$-DNA mit der für das synthetische Eglin B-Gen beschriebenen identisch.

**Beispiel 21**

**Synthese von Polypeptiden mit Eglin-Aktivität durch *E. coli*-Zellen, die Plasmide mit rekombinierten Eglin-Genen enthalten**

a. Synthese von Polypeptiden mit Eglin C-Aktivität

Jeder der 7 Klone, die das rekombinierte Eglin C-Gen enthalten, nämlich

*E. coli* HB101 pML147
*E. coli* HB101 pML148
*E. coli* HB101 pML149
*E. coli* HB101 pML150
*E. coli* HB101 pML151
*E. coli* HB101 pML152
*E. coli* HB101 pML153

wird auf die Bildung von Eglin C-Aktivität getestet.

Zu diesem Zweck werden die oben genannten Klone in 5 ml L-Medium über Nacht (16 Stunden) bei 37°C und 250 upm kultiviert. L-Medium ist wie folgt zusammengesetzt:

| | |
|---|---|
| Bacto Tryptone | 10 g |
| Bacto Hefe-Extrakt | 5 g |
| NaCl | 5 g |
| Glucose | 5 g |
| Ampicillin | 0,1 g |

1 ml dieser Übernachtkultur wird am nächsten Tag in 25 ml M9-Medium übertragen. M9-Medium ist wie folgt zusammengesetzt:

| | |
|---|---|
| $Na_2HPO_4 \cdot 7H_2O$ | 13,25 g |
| $KH_2PO_4$ | 3,0 g |
| NaCl | 0,5 g |
| $NH_4Cl$ | 1,0 g |
| $CaCl_2 \cdot 2H_2O$ | 0,015 g |
| $MgSO_4 \cdot 7H_2O$ | 0,25 g |
| Casaminosäuren | 2,5 g |
| Vitamin $B_1$ | 0,0099 g |
| Glucose | 5,0 g |
| Ampicillin | 0,1 g |

Es wird bei 37°C und 250 upm solange kultiviert, bis die Bakteriensuspension eine optische Dichte ($OD_{623}$) von ca. 0,9 – 1,0 erreicht hat. Anschliessend erntet man die Zellen (5 ml der wachsenden Kultur) und resuspendiert die Bakterien in 0,5 ml einer Lösung von 50 mM Tris · HCl (pH 8) und 30 mM NaCl. Danach wird die Suspension auf 1 mg/ml Lysozym (Boehringer) eingestellt und 30 min. in Eis gestellt. Durch abwechselndes Einfrieren der Suspension in flüssigem Stickstoff und Auftauen bei 37°C werden die Bakterien zerstört. Dieser Vorgang wird 5 mal wiederholt, anschliessend wird die Mischung 30 min. bei 16 000 upm bei 4°C zentrifugiert. Die Überstände werden auf Eglin C-Aktivität untersucht, indem die Hemmung der Humanleukocyten-Elastase (1) gemessen wird.

Folgende Aktivitäten werden erhalten:

| Bakterienextrakt | Eglin C-Aktivität µg/ml Kultur |
|---|---|
| *E. coli* HB101 pML147 | 3,3 |
| *E. coli* HB101 pML148 | 3,3 |
| *E. coli* HB101 pML149 | 3,4 |
| *E. coli* HB101 pML150 | 3,3 |
| *E. coli* HB101 pML151 | 3,3 |
| *E. coli* HB101 pML152 | 3,5 |
| *E. coli* HB101 pML153 | 3,3 |

b. Synthese von Polypeptiden mit Eglin B-Aktivität

In analoger Weise, wie in Beispiel 21a) beschrieben, werden jeder der 6 Klone, die das rekombinierte Eglin B-Gen enthalten, nämlich

*E. coli* HB101 pML199
*E. coli* HB101 pML200
*E. coli* HB101 pML201
*E. coli* HB101 pML202
*E. coli* HB101 pML203
*E. coli* HB101 pML204

auf die Bildung von Eglin B-Aktivität getestet.

Die genannten Klone werden, wie beschrieben, in L-Medium kultiviert und dann auf M9-Medium übertragen. Nach Erreichen einer optischen Dichte ($OD_{623}$) von ca. 0,9 – 1,0 werden die Zellen geerntet, lysiert und durch abwechselndes Einfrieren und Auftauen zerstört. Die Mischungen werden zentrifugiert, und die Überstände durch Messung der Hemmung der Humanleukocyten-Elastase (1) auf Eglin B-Aktivität geprüft.

Folgende Aktivitäten werden erhalten:

| Bakterienextrakt | Eglin B-Aktivität µg/ml Kultur |
|---|---|
| *E. coli* HB101 pML199 | 3,2 |
| *E. coli* HB101 pML200 | 3,1 |
| *E. coli* HB101 pML201 | 3,8 |
| *E. coli* HB101 pML202 | 3,5 |
| *E. coli* HB101 pML203 | 3,3 |
| *E. coli* HB101 pML204 | 3,3 |

**Beispiel 22**

**Kultivierung des Stammes E. coli HB101 pML147**

20 ml L-Medium (siehe Beispiel 21) werden mit den *E. coli* HB101 pML147-Zellen von einer gut gewachsenen Agarplatte beimpft und im Schüttelkolben auf einem Rundschüttler bei 150 upm bei 37°C für 12 Stunden geschüttelt. 5 ml dieser Vorkultur werden in 120 ml M9 Nährmedium übertragen. Diese Kultur wird bei 250 upm und 37°C geschüttelt. Nach ca. 8 – 10 Stunden hat die Kultur den höchsten Titer an Polypeptiden mit Eglin C-Aktivität erreicht und wird geerntet.

**Beispiel 23**

**Nachweis der Eglin C-Aktivität**

Ca. 5 – 10 µl einer Probe, welche Polypeptide mit Eglin C-Aktivität enthält (vgl. Beispiele 21 und 22), werden auf 1 $cm^2$ Nitrocellulosepapier (NZ) (BIORAD) aufgetropft und 30 min. bei Raumtemperatur getrocknet. Danach wird das NZ für 1 Stunde bei 37°C in einer Lösung von 3 % Serumalbumin in 0,01 M Tris · HCl (pH 8) und 0,9 % NaCl inkubiert.

Anschliessend wird das NZ in einer Lösung von 0,01 M Tris · HCl (pH 8) und 0,9 % NaCl für 30 min. gewaschen. Dabei wird die Lösung 5-mal gewechselt. Danach wird das gewaschene NZ für 2 Stunden bei 25°C in einer Lösung von 3 % Serumalbumin in 0,01 M Tris · HCl (pH 8) und 0,9 % NaCl, die 2 µg/ml Antikörper (aus Kaninchen hergestellte, oder monoclonale Antikörper) gegen Eglin C enthält, behandelt. Danach wird das NZ, wie oben beschrieben gewaschen.

Anschliessend wird das NZ für 2 – 3 Stunden bei 25°C mit einer Lösung von 3 % Serumalbumin in 0,01 M Tris · HCl (pH 8) und 0,9 % NaCl, die 0,2 µCi/ml $^{125}$I-Protein A (sp. Aktivität 89,8 µCi/mg) (NEN) enthält, behandelt. Danach wird abermals das NZ, wie oben beschrieben, gewaschen, getrocknet und in einem γ-Zähler (Multi Gamma, 1260 gamma counter, LKB, Wallace) die gebundene Radioaktivität bestimmt, welche ein Mass für das auf dem NZ vorhandene Polypeptid mit Eglin C-Aktivität ist.

In einem alternativen Verfahren wird obige Probe einer SDS-Polyacrylamidgel-Elektrophorese (PAGE) unterworfen [vgl. (7)]. Das PAGE-Elektropherogramm wird durch Elektro-Blotting auf NZ übertragen. Danach wird die NZ wie oben beschrieben behandelt und/oder über Nacht zusammen mit einem Röntgenfilm (Fuji) autoradiographiert. Stellen auf dem NZ, welche Polypeptide mit Eglin C-Aktivität enthalten, erscheinen auf dem Film als schwarze Flecken.

**Beispiel 24**

**Isolierung und Reinigung von $N^{\alpha}$-Acetyl-eglin C mit Hilfe einer monoklonalen Antikörper-Säule**

a. Herstellung der Polypeptidlösung für die monoklonale Antikörpersäule

150 ml Kulturbrühe (erhalten gemäss Beispiel 22) werden auf 4°C abgekühlt und die Zellen durch Zentrifugation (5000 upm, 15 min., Sorvall RC 3B) abgetrennt. Der klare Überstand enthält keine Eglin C-Aktivität.

Anschliessend werden die Zellen in 12 ml Lyse Puffer (50 mM Tris · HCl pH 8, 30 mM NaCl) suspendiert. Dieser Mischung werden 15 mg Lysozym (Boehringer) zugesetzt und diese dann 30 min. bei 4°C aufbewahrt. Anschliessend werden die Zellen durch 4 maliges Einfrieren in flüssigem Stickstoff und anschliessendem Auftauen bei 37°C zerstört.

Danach wird 30 min. bei 16 000 upm, 4°C zentrifugiert. Der Überstand enthält die $N^{\alpha}$-Acetyl-eglin C-Aktivität. In dem Überstand (15 ml) werden dann 7,7 g festes Ammoniumsulfat aufgelöst. Die trübe Mischung wird bei 4°C für 30 min. stehengelassen und dann zentrifugiert (s. oben). Das nasse Sediment wird in 1 ml 0,05 mM Tris · HCl pH 8 Puffer gelöst und man erhält die gewünschte Polypeptidlösung.

b. Reinigung von $N^{\alpha}$-Acetyl-eglin C auf einer monoklonalen Antikörper-Säule

Die monoklonale Antikörper-Säule 1K-F299-22-10 (Bettvolumen 0,8 ml, siehe unten) ist mit 0,05 M Tris · HCl (pH 8) äquilibriert. 0,5 ml-Portionen der oben erhaltenen Polypeptid-Lösung werden bei 4°C auf die Säule mit einer Fliessgeschwindigkeit von 7 ml/Stunde aufgetragen. Gewaschen wird dann mit 10 ml 0,05 M Tris · HCl pH 8. Die ersten Fraktionen enthalten die nicht adsorbierten Polypeptide, welche verworfen werden. Anschliessend wird die Säule mit 5 ml 5 M Natriumthiocyanat (Merck) in 0,05 M Tris · HCl (pH 8) gewaschen und die erhaltenen Fraktionen auf $N^{\alpha}$-Acetyl-eglin C-Aktivität mit dem HLE Test (1) geprüft. Die Fraktionen, welche die Polypeptide enthalten, werden durch Messung der $OD_{280nm}$ bestimmt. Fraktionen 19 und 20 enthalten die $N^{\alpha}$-Acetyl-eglin C-Aktivität; sie werden bei –20°C aufbewahrt oder bis zur Weiterverarbeitung im Eisbad. Die $N^{\alpha}$-Acetyl-eglin C-Aktivität beträgt in Fraktion 19 61 µg/ml und in Fraktion 20 49 µg/ml. Danach werden die Fraktionen dialysiert oder über Sephadex-G25 (Pharmacia) entsalzt. Die SDS-Polyacrylamidgel-Elektrophorese (7) ergibt ein Molekulargewicht für $N^{\alpha}$-Acetyl-eglin C von ca. 8100 Dalton.

In analoger Weise kann $N^{\alpha}$-Acetyl-eglin B mittels der monoklonalen Antikörper-Säule 1K-F299-22-10 gereinigt werden.

c. Herstellung der monoklonalen Antikörper-Säule 1K-F299-22-10

A) Immunisierung von Mäusen

Reines natürliches Eglin C (6 mg) in lyophilisierter Form wird in wenig 0,1 % Essigsäure gelöst und dann mit phosphatgepufferter Kochsalzlösung ergänzt und der pH wird auf 7,2 eingestellt, so dass die Endkonzentration 2 mg/ml beträgt. Portionen dieser Antigen-Lösung werden mit gleichen Mengen komplettem Freund's Adjuvans, inkomplettem Freund's Adjuvans oder phosphatgepufferter Salzlösung gemischt und emulgiert.

Weibliche Balb/c Mäuse (8 – 14 Wochen alt, erhalten von der Tierfarm Sisseln, Schweiz) werden durch Injektion einer solchen Emulsion, enthaltend 100 µg Eglin, in die Fusspfoten immunisiert. Während den folgenden sechs Wochen werden jede Woche weitere 100 µg Eglin emulgiert wie zuvor, aber in inkomplettem Freund's Adjuvans subcutan und schliesslich 200 µg Eglin in phosphatgepufferter Salzlösung intravenös injiziert. Vier Tage später wird die Milz für die Fusion entnommen.

B) Herstellung der Hybridoma und Antikörper-Test

Die Herstellung der Hybridomazellen erfolgt durch Verschmelzung der erhaltenen Milzzellen mit der Myeloma-Zellinie SP 2/0. Dabei werden $10^8$ Milzzellen und $10^7$ Myelomazellen eingesetzt. Die Fusion wird wie beschrieben (9, 26) durchgeführt.

Die Bestimmung der anti-Eglin C-Aktivität in den Hybridoma-Überständen erfolgt mit Hilfe eines kompetitiven Radioimmunoassays [RIA, (10)].

Zu diesem Zwecke wird Eglin C mit radioaktivem $^{125}$Jod nach der üblichen Chloramin T-Methode markiert (30 000 cpm). Durch Inkubieren über Nacht wird ein polyklonaler Kaninchen anti-Eglin C-Antikörper in den Vertiefungen einer Polystyrol-Mikrotiterplatte fixiert. Etwa 50 – 70 % des radioaktiven Eglins C werden an diesen Festphasen-Antikörper gebunden. Von 45 erhaltenen Hybridoma-Kulturen inhibierten 32 Überstände diese Bindung zu mehr als 50 % signifikant. Zwei der stark inhibierenden Überstände, bzw. ihre Hybridoma-Zellen, werden als 299S18 und 299S22 bezeichnet und zur weiteren Charakterisierung ausgewählt. Sie werden zunächst durch die begrenzende Verdünnungsmethode kloniert, wobei 299S18 vier und 299S22 neun positive Klone ergibt, von denen die Klone 299S18-20, 299S22-1 und 299S22-10 ausgewählt und näher charakterisiert werden. Die genannten Hybridoma-Zellinien produzieren monoklonale Antikörper (mit der gleichen Bezeichnung) vom Subtyp $Ig_1$kappa.

C) Isolierung und Reinigung der Anti-Eglin C-Antikörper aus Aszites

Balb/c Mäuse werden intraperitoneal mit 0,4 ml Pristan (Carl Roth) vorbehandelt. Nach einer Woche werden 2 bis 5 x $10^6$ klonierte Hybridoma-Zellen intraperitoneal injiziert. Aszitische Flüssigkeit wird von jeder Maus wiederholt genommen und bei –80°C gefroren. Die gesammelte Flüssigkeit wird aufgetaut und 30 min. bei 4°C mit 16 000 upm zentrifugiert. Das Fett wird abgesaugt und zum verbleibenden Debri-freien Überstand langsam unter Rühren bei 0°C 0,9 Volumenäquivalente einer gesättigten Ammoniumsulfatlösung zugetropft. Die erhaltene rohe Immunoglobulin-Fraktion wird unter Verwendung von 0,1 mM Tris · HCl (pH 8,2) durch Sephacryl G 200 (Pharmacia), gemäss den Angaben des Herstellers, gegeben. Aktive Fraktionen werden vereinigt und mit Amicon XM50-Filter (Amicon) konzentriert. Man erhält auf diese Weise die monoklonalen anti-Eglin C-Antikörper 299S18-20, 299S22-1 und 299S22-10.

D) Herstellung der Antikörper-Säule 1K-F299-22-10

Affi-Gel 10 (Bio-Rad) wird nach den Angaben des Herstellers mit kaltem destilliertem Wasser und Kupplungspuffer pH 8,0 (0,1 M $NaHCO_3$-Lösung) gewaschen. Eine 50 %-ige Suspension des Gels in Kupplungspuffer (1 ml) wird in ein Plastikrohr übertragen, mit der gleichen Menge gereinigter Antikörperlösung (19 mg monoklonale anti-Eglin-C-Antikörper 299S22-10) vermischt und 4 Stunden bei Zimmertemperatur rotiert. Danach wird das Gel mit Kupplungspuffer gewaschen. Zur Blockierung der noch freien aktiven Stellen wird das Gel mit 0,1 ml 1 M Ethanolamin-HCl (pH 8,0) pro ml Gel während 2 Stunden bei Raumtemperatur behandelt, dann mit phosphatgepufferter Salzlösung, enthaltend 10 mM Natriumazid pro ml Gel gewaschen und darin bei 4°C gehalten. Der Kupplungsgrad wird durch Messung der Extinktion bei 280 nm bestimmt und beträgt 15 bis 30 mg Antikörper pro ml Gel. 0,8 ml des entstandenen Immunogels werden verwendet, um die monoklonale Antikörpersäule 1K-F299-22-10 herzustellen.

**Beispiel 25**

**Isolierung und Reinigung von $N^{\alpha}$-Acetyl-eglin C mit Hilfe einer Anhydrochymotrypsin-Säule**

a Herstellung der Polypeptidlösung für die Anhydrochymotrypsin-Säule

150 ml Kulturbrühe (erhalten gemäss Beispiel 22) werden auf 4°C abgekühlt und die Zellen durch Zentrifugation (5000 upm, 15 min., Sorvall RC 3B) abgetrennt. Der klare Überstand enthält keine Eglin C-Aktivität.

Anschliessend werden die Zellen in 12 ml Lyse Puffer (50 mM Tris · HCl pH 8, 30 mM NaCl) suspendiert. Dieser Mischung werden 15 mg Lysozym (Boehringer) zugesetzt und diese dann 30 min. bei 4°C aufbewahrt. Anschliessend werden die Zellen durch 4 maliges Einfrieren in flüssigem Stickstoff und anschliessendem Auftauen bei 37°C zerstört. Danach wird 30 min. bei 16 000 upm 4°C zentrifugiert. Der Überstand enthält die $N^{\alpha}$-Acetyl-eglin C-Aktivität. In dem Überstand (15 ml) werden dann 7,7 g festes Ammoniumsulfat aufgelöst. Die trübe Mischung wird bei 4°C für 30 min. stehengelassen und dann zentrifugiert (s. oben). Das nasse Sediment wird in 1 ml 0,05 mM Tris · HCl pH 8 Puffer gelöst und man erhält die gewünschte Polypeptidlösung.

b. Reinigung von $N^{\alpha}$-Acetyl-eglin C auf einer Anhydrochymotrypsin (AnCht)-Säule

Die AnCht-Säule (Bettvolumen 4 ml) ist mit 0,05 M Tris-HCl pH 8 äquilibriert. 2,5 ml-Portionen der oben erhaltenen Polypeptid-Lösung werden bei 4°C auf die Säule mit einer Fliessgeschwindigkeit von 7 ml/Stunde aufgetragen. Gewaschen wird dann mit 25 ml 0,05 M Tris · HCl (pH 8). Die ersten Fraktionen enthalten die nicht adsorbierten Polypeptide, welche verworfen werden. Anschliessend wird die Säule mit 10 ml 5 M Natriumthiocyanat (Merck) in 0,05 M Tris · HCl (pH 8) gewaschen und die erhaltenen Fraktionen auf $N^{\alpha}$-Acetyl-eglin C-Aktivität mit dem HLE Test (1) geprüft. Die Fraktionen, welche die Polypeptide enthalten, werden durch Messung der $OD_{280nm}$ bestimmt. Fraktionen 30 und 31 enthalten die $N^{\alpha}$-Acetyl-eglin C-Aktivität; sie werden bei –20°C aufbewahrt oder bis zur Weiterverarbeitung im Eisbad. Die $N^{\alpha}$-Acetyl-eglin C-Aktivität beträgt in Fraktion 30 30 µg/ml und in Fraktion 31 64 µg/ml. Danach werden die Fraktionen dialysiert oder über Sephadex-G25 (Pharmacia) entsalzt. Die SDS-Polyacrylamidgel-Elektrophorese (7) ergibt ein Molekulargewicht für $N^{\alpha}$-Acetyl-eglin C von ca. 8100 Dalton.

c. Herstellung der Anhydrochymotrypsin-Säule

A. Herstellung von Anhydrochymotrypsin (AnCht)

Die Herstellung von AnCht erfolgt wie von Ako et al. (27) beschrieben:

500 mg Chymotrypsin (Merck) werden in 50 ml 0,1 M Tris-HCl (pH 8)-Puffer, der 0,1 M NaCl, 0,12 M $CaCl_2$ und 13 % (v/v) Methanol enthält, gelöst. Zu dieser Lösung werden siebenmal 0,1 ml Aliquote von Phenylmethylsulfonylfluorid (PMSF) (Fluka, Lösung von 7 mg/ml in Aceton) unter Rühren zugegeben und jeweils die Abnahme der Chymotrypsinaktivität bestimmt (28). Nachdem die Chymotrypsinaktivität unter 1 % gesunken ist, wird die Lösung gegen 1 mM HCl über Nacht bei 4°C dialysiert (3 x 10 Liter) und anschliessend lyophilisiert.

Das entstandene Phenylmethylsulfonyl-Chymotrypsin (PMS-Cht) wird in 100 ml eiskalter 0,1 M KOH gelöst, 1 Stunde in Eis stehen lassen und anschliessend mit 6 N HCl auf pH 3 eingestellt. Die erhaltene Lösung wird über Nacht bei 4°C gegen 1 mM HCl dialysiert (3 x 10 Liter) und anschliessend lyophilisiert. AnCht fällt als weisses Pulver an (120 mg).

B. Herstellung der AnCht-Säule

Affi-Gel 10 (Bio Rad) wird nach Angaben des Herstellers mit kaltem destilliertem Wasser und Kupplungspuffer pH 8,5 (0,1 M $NaHCO_3$/$Na_2CO_3$-Lösung) gewaschen. Eine 50 %-ige Suspension der Gels in Kupplungspuffer (4 ml) wird in ein Plastikrohr übertragen, mit der gleichen Menge Anhydrochymotrypsinlösung (120 mg in 4 ml Kupplungspuffer) vermischt und über Nacht bei 4°C rotiert. Danach wird das Gel mit Kupplungspuffer gewaschen. Zur Blockierung der noch freien aktiven Stellen wird das Gel mit 0,1 ml 1 M Äthanolamin-HCl (pH 8,0) pro ml Gel während 3 Stunden bei 4°C behandelt dann mit phosphatgepufferter Salzlösung, enthaltend 10 mM Natriumazid pro ml Gel gewaschen und darin bei 4°C gehalten. Der Kupplungsgrad wird durch Messung der Extinktion bei 280 nm bestimmt und beträgt 15 bis 30 mg AnCht pro ml Gel.
4 ml des entstandenen AnCht-Gels werden verwendet, um die Affinitätssäule herzustellen.
In gleicher Weise kann auch $N^{\alpha}$-Acetyl-eglin B gereinigt werden.

**Beispiel 26**

**Alternative Reinigungsverfahren für $N^{\alpha}$-Acetyl-eglin C**

Alternativ oder zusätzlich zu den vorstehenden Reinigungsverfahren (vgl. Beispiele 24 und 25) können folgende Reinigungsschritte verwendet werden:

a. Butanol-Extraktion des Lysats

Die nach der Lyse durch viermaliges Einfrieren und Auftauen zerstörten Zellen (vgl. Beispiel 24a) werden mit Essigsäure (Endkonzentration 0,1 %; pH 4,5) versetzt. Die ausfallenden bakteriellen Proteine werden mittels Zentrifugation abgetrennt. $N^{\alpha}$-Acetyl-eglin C verbleibt im Überstand.

Das Zweiphasengemisch n-Butanol-Eisessig-Wasser 5 : 1 : 4 (25 ml) wird kräftig vorgemischt. Man lässt es dann 2 Stunden bei Zimmertemperatur äquilibrieren, wobei sich das Gemisch in zwei Phasen trennt. 0,5 ml der 0,1 % Essigsäure-Lysat-Probe (s.o.) werden mit 250 µl Unterphase verdünnt und $N^{\alpha}$-Acetyl-eglin C mit 750 µl Oberphase extrahiert (5 min. Vortex, Fa. Bender Hobein). Anschliessend trennt man die Phasen durch 60 min. Zentrifugation (5400 upm) bei Raumtemperatur. (Hettich Tischzentrifuge EBA 3S). Die Probe wird mit einem Gerät der Fa. Savant (Speed Vac Concentrator) am Hochvakuum zur Trockene eingedampft. Der Nachweis von $N^{\alpha}$-Acetyl-eglin C gelingt mittels HLE-Test, RP-HPLC und SDS-Gelelektrophorese.

b. Gelfiltration an Sephadex G50®

31 mg des so erhaltenen Materials werden in 600 µl 30 % Essigsäure suspendiert, 5 min bei Raumtemperatur bei 5 000 upm zentrifugiert und der klare Überstand wird auf die Sephadex-Säule G50® fine (Pharmacia) aufgetragen (Säulendimension: 1,5 cm x 30 cm; Detektion: LKB8300 Uvicord II; 254 nm, Transmission 500 mV; Fluss: 0,4 ml/min).

Eluiert wird mit 50 ml 2 % Essigsäure. $N^{\alpha}$-Acetyl-eglin C ist in den Fraktionen 6 – 8 (2,5 ml) enthalten. Ausbeute: 3 mg reines Lyophilisat, Reinheit ca. 95 %.

c) <u>Anionenaustauschchromatographie an DEAE-Cellulose zu Gewinnung von $N^{\alpha}$-Acetyl-eglin C</u>

100 ml eines Überstands nach der Proteinfällung mittels Essigsäure (vgl. Beispiel 26a) wird konzentriert und einer Anionenaustausch-Chromatographie an DEAE-53 (Fa. Whatman) bei pH 6,6 unterworfen (Chromatographiebedingungen: Säule: 1,5 x 80 cm, Elutionspuffer: 30 mM Ammoniumacetat, pH 6,6, Fluss 15 ml/h. Fraktionsvolumen 3,5 ml). Die Säule wird mit dem Elutionspuffer äquilibriert und entwickelt, bis der erste Peak (Eglin C) zwischen Fraktionen 18 – 25 eluiert ist. Ab Fraktion 50 wird ein linearer Salzgradient aus je 300 ml Elutionspuffer und 0,06 M Amoniumacetat/0,4 M NaCl pH 4,5 ausgeschlossen. $N^{\alpha}$-Acetyl-eglin C eluiert zwischen Fraktionen 70 – 85. Der Nachweis gelingt mittels RP-HPLC, PAGE and HLE-Test. Die Reinheit der Produkte beträgt ca. 90 % bezüglich Proteingehalt.
IP (pool Frakt. 18 – 25): 6,5
IP (pool Frakt. 70 – 85): 5,4.

Auf diese beschriebene Weise kann auch $N^{\alpha}$-Acetyl-eglin B abgetrennt und gereinigt werden.

**Beispiel 27**

**Strukturbeweis und physikalisch-chemische Charakterisierung von $N^{\alpha}$-Acetyl-eglin C**

a. <u>Bestimmung der Aminosäurezusammensetzung</u>

200 µg $N^{\alpha}$-Acetyl-eglin C werden mit 6N HCl bei 110°C während 24 h hydrolysiert und dann nach S. Moore et al. (29) analysiert. Das Hydrolysat weist die folgende Zusammensetzung auf:

| Aminosäure | Hydrolysat | Aminosäure | Hydrolysat |
|---|---|---|---|
| Asn | 7,2 (7) | Met | 0 (0) |
| Thr | 4,6 (5) | Leu | 5,3 (5) |
| Ser | 3,5 (3) | Tyr | 4,9 (6) |
| Gln | 7,8 (7) | Phe | 4,9 (5) |
| Pro | 5,4 (6) | Lys | 2,3 (2) |
| Gly | 5,7 (5) | His | 2,5 (3) |
| Ala | 1,6 (1) | Trp | 0 (0) |
| Val | 10,1 (11) | Arg | 4,5 (4) |
| | | Total: | (70) |

b. <u>Peptid-Mapping von $N^{\alpha}$-Acetyl-eglin C</u>

Im folgenden Schema sind die Aminosäuresequenz von $N^{\alpha}$-Acetyl-eglin C und die Spaltstellen für Trypsin und *Staphylococcus aureus*-Protease (V8) markiert (vgl. Zitat 31):

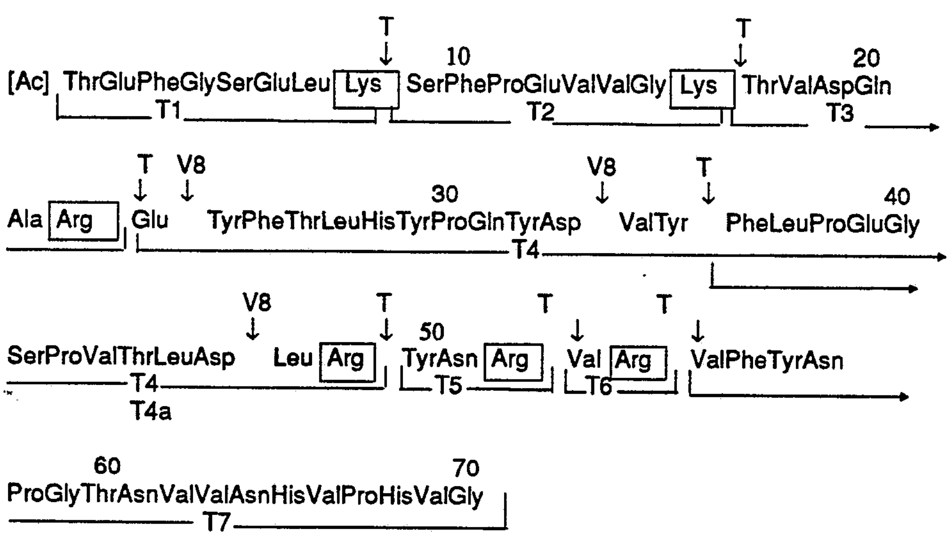

T: Spaltstellen für Trypsin; V8: Spaltstellen für *Staphylococcus aureus*-Protease (V8)

I) Tryptischer Abbau von $N^{\alpha}$-Acetyl-eglin C

$N^{\alpha}$-Acetyl-eglin C (9,6 mg, 1,18 µMol) wird in 2 ml 0,1 N Ammoniumacetat-Puffer; $10^{-3}$ M $CaCl_2$ suspendiert, der pH mit verdünntem Ammoniak wird auf 7,5 eingestellt und mit TPCK-Trypsin (Worthington, 500 µg) während 90 Stunden bei 37°C inkubiert. Die Enzymreaktion wird durch Zugabe von 50 µl Eisessig gestoppt. Durch Zentrifugation entfernt man ein tryptisches Fragment ($T_4$) und trennt anschliessend mittels Umkehrphasen-HPLC den klaren Überstand in die übrigen tryptischen Fragmente ($T_1$–$T_7$) auf (vgl. obiges Schema). Die Analyse erfolgt mittels FAB-Mapping (30).

Der tryptische Abbau von $N^{\alpha}$-Acetyl-eglin C (200 pMol) und die mikropräparative RP-HPLC-Isolierung von DABTC-Peptiden nach der Methode von R. Knecht et al. (32), sowie der Vergleich mit natürlichem Eglin C bestätigt die Identität der tryptischen Peptide $T_2$, $T_3$, $T_4$, $T_5$, $T_6$ und $T_7$ (vgl. obiges Schema).

Das Peptid $T_1$ (Threonin am N-Terminus) weist in beiden Experimenten eine im Vergleich zum natürlichen Eglin C verschiedene Retentionszeit in einer HPLC-Analyse auf (Nucleosil 5/C18, 4,6 x 120 mm; 1,2 ml/min.; Elutionsmittel: 0,1 % Trifluoressigsäure; Acetonitril-Wasser 8 : 2 mit 0,07 % Trifluoressigsäure):
$R_t$ 9,44 min. (zum Vergleich Peptid $T_1$ bei natürlichem Eglin C:
$R_t$ 7,34 min).

II) Staphylococcus aureus-Protease V8-Abbau des tryptischen Fragments $T_4$ von $N^{\alpha}$-Acetyl-eglin C

Der Abbau von ca. 100 µg des tryptischen Fragments $T_4$ von $N^{\alpha}$-Acetyl-eglin C (siehe oben) durch *Staphylococcus aureus*-Protease V8 wird in 100 µl 0,1 M Ammoniumacetat pH 8,0 bei 37°C während 4 h durchgeführt. Der Abbau ergibt die erwarteten Fragmente (vgl. obiges Schema; Gemischanalyse mittels FAB-MS).

c. Partielle Sequenzanalyse

I) Edman-Abbau

Das Misslingen der klassischen Sequenzanalyse nach Edman unter Standardbedingungen (33) (keine N-terminalen Aminosäurereste werden identifiziert) weist auf einen modifizierten (blockierten) N-Terminus beim $N^{\alpha}$-Acetyl-eglin C hin.

II) Sequenzierung mittels FAB-MS

Das N-terminale tryptische Fragment "$T_1$" hat laut FAB ("fast atom bombardment")-MS ein nominales Molekulargewicht von 951. Dieses liegt damit um 42 höher als im korrespondierenden $T_1$-Fragment aus natürlichem Eglin C (909). Auf Grund der Massendifferenzen muss die Modifikation an der N-terminalen Aminosäure Threonin vorliegen.

Die Molekulargewichte der übrigen tryptischen Fragmente aus obigem Experiment (Beispiel 27bI) entsprechen den Erwartungen.

d) Molekulargewichtsbestimmung von $N^{\alpha}$-Acetyl-eglin C (Vergleich mit natürlichem Eglin C)

| Probe 1 ($N^{\alpha}$-Acetyl-eglin C) | Probe 2 (natürliches Eglin C aus Blutegeln) |
|---|---|
| Summenformel: | Summenformel: |
| $C_{375}H_{522}N_{96}O_{108}$ | $C_{373}H_{550}N_{96}U_{107}$ |
| chemisches Molekulargewicht | chemisches Molekulargewicht |
| gefunden: 8133.1 | gefunden: 8091.4 |
| berechnet: 8133.06 | berechnet: 8091.03 |

Die chemischen Molekulargewichte sind gemittelt aus 3 verschiedenen Messungen (C 12.011; H 1.0079; N 14.0067; O 15.9994).

**Experimentelle Bedingungen**

ca. 30 µg Probe wird direkt in Thioglycerin als Matrix auf dem Probengeber gelöst, und mit einem ZAB-HF (Auflösung 1000)-Massenspektrometer der Fa. VG-Analytical Ltd. Manchester gemessen: Xenon bombardment; Ion energy 3 keV; scanning linear mode; Eichung: CsI/RbI-Referenz Mischung

e. Isoelektrische Fokussierung:

| | | | |
|---|---|---|---|
| Isoelektrischer Punkt | IP | $N^{\alpha}$-Acetyl-eglin C | 5,4 |
| | IP | natürliches Eglin C | 6,5 |

**Bedingungen**

Je 20 µg Probe in 20 µl $H_2O$ aufgetragen. PAGplate LKB-Ampholine pH 3,5 – 9,5, 5 % PAG 1 mm. Elektrolyt: Anode(+) 1 M $H_3PO_4$, Kathode(–) 1 N NaOH, 20 mA, 700V, 2,5 Std. Färbung mittels 10 % (wt./vol.) Trichloressigsäure-Lösung oder Coomassie Brilliant Blue R-250 in üblicher Weise.

f. Celluloseacetat-Elektrophorese (steigend)

$N^{\alpha}$-Acetyl-eglin C : 4,7 cm vom Start Richtung Kathode
Eglin C : 5,8 cm vom Start Richtung Kathode

**Bedingungen**

Je 2 µg Probe in 2 µl $H_2O$ aufgetragen auf Cellogel 8 x 17 cm Folie (Chemetron, Milano): Horiphor Flachbettelektrophoresekammer (Innovativ Labor), Elektrolyt pH 1,9, 250 Volt 1 Stunde; Nachweis mit den üblichen Färbereagentien, wie TDM. Ninhydrin, Ponceau S-Lösung (Biotec-Fischer).

g. Nachweis der N-Acetyl-Gruppe in $N^{\alpha}$-Acetyl-eglin C

I) 100 µg $N^{\alpha}$-Acetyl-eglin C werden in 100 µl 0,03 N Salzsäure für 16 Stunden bei 110°C partiell hydrolysiert und am Hochvakuum getrocknet. Mittels Gaschromatographie werden mehr als 0,5 Äquivalente Essigsäure identifiziert (34).

II) Die Acetylfunktion wird eindeutig mittels 360 MHz-Protonenresonanzspektroskopie im tryptischen Fragment "$T_1$" (vgl. Beispiel 27BI) identifiziert: 400 µg Fragment "$T_1$" aus $N^{\alpha}$-Acetyl-eglin C werden am Hochvakuum während 2 h getrocknet und in 1 ml $D_2O$ gelöst. Das 360 MHz $^1$H-NMR-Spektrum wird bei 297° K mit 4 000 SW über Nacht gemessen. Referenz $H_2O$ (δ 4,95 ppm).

δ 2,15 ppm Singulett (3H) $CH_3$ aus N-Acetyl-Gruppe
δ 1,2 ppm Dublett (3H, J = 7 Hz) γ-$CH_3$ aus Threonin.

**Beispiel 28**

**Transformation verschiedener *E. coli*-Stämme mit dem Plasmid pML147 und Kultivierung der transformierten Wirtszellen**

In analoger Weise, wie in Beispiel 18d beschrieben, werden die Stämme *E. coli* LM1035, *E. coli* JA221 und *E. coli* W3110 trpR, trp Δ ED24 (vgl. Referenz 38) mit dem Plasmid pML147 transformiert. Transformierte Kolonien werden auf das Vorhandensein von $F_1$ (C)-$F_2$-DNA geprüft, wie in Beispiel 15e beschrieben. Es werden 3, 5 bzw. 3 positive Kolonien erhalten, die die folgenden Bezeichnungen erhalten:

*E. coli* LM1035/pML147/1
*E. coli* LM1035/pML147/2
*E. coli* LM1035/pML147/3
*E. coli* JA221/pML147/1
*E. coli* JA221/pML147/2
*E. coli* JA221/pML147/3
*E. coli* JA221/pML147/4
*E. coli* JA221/pML147/5
*E. coli* W3110trpR, Δ trpED24/pML147/1
*E. coli* W3110trpR, Δ trpED24/pML147/2
*E. coli* W3110trpR, Δ trpED24/pML147/3

Die genannten Klone werden in einem modifizierten M9-Medium kultiviert, welches die folgende Zusammensetzung aufweist:

| | | |
|---|---|---|
| $Na_2HPO_4 \cdot 7H_2O$ | 9,0 | g |
| $KH_2PO_4$ | 3,0 | g |
| NaCl | 0,5 | g |
| $NH_4Cl$ | 3,5 | g |
| $CaCl_2 \cdot 2H_2O$ | 0,015 | g |
| $MgSO_4 \cdot 7H_2O$ | 0,25 | g |
| Casaminosäuren | 7,0 | g |
| Hefeextrakt | 5,0 | g |

| | |
|---|---|
| Vitamin $B_1$ | 0,0099 g |
| Eisen-III-citrat | 0,006 g |
| MOPS (3-Morpholinopropan-1-sulfonsäure) | 34,0 g |
| Glucose | 20,0 g |
| Ampicillin | 0,1 g |

Es wird bei 37°C und 180 upm solange kultiviert, bis die Bakteriensuspension eine optische Dichte ($OD_{623}$) von ca. 13,0 erreicht hat. Anschliessend erntet man die Zellen (5 ml der wachsenden Kultur) und resuspendiert die Bakterien in 0,5 ml einer Lösung von 50 mM Tris · HCl (pH 8) und 30 mM NaCl. Danach wird die Suspension auf 1 mg/ml Lysozym (Boehringer) eingestellt und 30 min. in Eis gestellt. Durch abwechselndes Einfrieren der Suspension in flüssigem Stickstoff und Auftauen bei 37°C werden die Bakterien zerstört. Dieser Vorgang wird 5 mal wiederholt. Anschliessend wird die Mischung 30 min. bei 16 000 upm bei 4°C zentrifugiert.

Jeder der Klone wird auf die Bildung von Eglin C-Aktivität getestet, wie in Beispiel 21 beschrieben. In den Bakterienextrakten werden Eglin C-Aktivitäten von 3,0 – 13 µg/ml Kultur erhalten. Beispielsweise werden die folgenden Aktivitäten erhalten:

| Stamm | Eglin C-Aktivität (µg/ml Kulturlösung) |
|---|---|
| *E. coli* LM1035/pML147/1 | 3,0 |
| *E. coli* JA 221/pML147/1 | 6,0 |
| *E. coli* W3110trpR, trp Δ ED24/pML147/1 | 11,0 |

**Beispiel 29**

**Fermentation des transformierten Stammes *E. coli* W3110trpR, trp Δ ED24/pML147/1 und Aufarbeitung der Kulturbrühe**

In analoger Weise, wie in Beispiel 28 beschrieben, werden in einem 5000 l Fermenter *E. coli* W3110trpR, trp Δ ED24/pML147/1-Zellen in 3000 l modifiziertem N9-Medium kultiviert, bis die Suspension eine optische Dichte ($OD_{623}$) von ca. 10 – 13 erreicht hat.

Die Kulturbrühe (pH 7,4) wird auf 10°C abgekühlt, und die Zellen werden mit einer Alfa-Laval BRPX-207 Entschlammvorrichtung behandelt. Der klare Überstand enthält keine Eglin-Aktivität und wird verworfen. Während der Entschlammung wird der Schlammraum fortwährend mit Lysepuffer A (50 mM Tris · HCl, 30 mM NaCl, eingestellt mit HCl auf pH 8,0) teilentschlammt und am Schluss der Inhalt der Zentrifugenschale (7 l) unter voller Entschlammung mit Lysepuffer A ausgestossen. Die erhaltene Zellmasse wird mit Puffer A auf 375 l eingestellt und besitzt einen pH Wert von 7,6. Nach dem Kühlen auf 5 – 10°C wird die Suspension durch eine Dyno-Mühle (Typ KD5) geleitet, welche mit 4,2 l Glasperlen mit einem Durchmesser von 0,5 – 0,75 mm ausgerüstet ist. Dabei werden die Zellen zerstört. Die so erhaltene Suspension wird mit Essigsäure auf einen Essigsäuregehalt um 2 % (v/v) eingestellt und über Nacht bei 10°C gerührt. Die Suspension mit einem pH von 3,9 wird mit der oben beschriebenen Technik entschlammt. Der klare Überstand von 300 l wird in einem Fallfilmverdampfer (Stundenleistung 60 l Wasser) auf 35 l aufkonzentriert. Das leicht trübe Konzentrat wird zentrifugiert und der so erhaltene klare Überstand auf einer Ultrafiltrationsanlage DDS = Lab 35, welche mit GR 81 PP Membrane bestückt ist (Fläche 2,5 $m^2$), gegen 2 % Essigsäure diafiltriert. Das Endvolumen beträgt 31 l.

Ein aliquoter Test von 2 l dieser klaren Proteinlösung wird auf eine Sephadex G-50 F-Säule (KS 370 Pharmacia) mit einem Bettvolumen von 96 l aufgetragen, wobei die Säule mit 2 % Essigsäure äquilibriert ist. Die in 15 l Eluat enthaltene Hauptfraktion wird mittels Ultrafiltration eingeengt und anschliessend gegen Wasser diafiltriert. Die so erhaltene klare wässrige Lösung wird lyophilisiert. Der Rückstand besteht aus reinen Eglin C-Verbindungen.

**Beispiel 30**

**Analyse des Produktgemisches aus der Fermentation von *E. coli* W3110trpR, trp Δ ED24/pML147/1**

Der in Beispiel 29 erhaltene, aus Eglin C-Verbindungen bestehende Rückstand wird einer HPLC-Analyse unterworfen.

**Experimentelle Bedingungen**

Vydac 218 TP510-RP-HPLC-Säule, 10 x 250 mm; 1 mg Eglin-Verbindungen pro Trennung; AUFC: 2,0 bei 220 nm; Durchflussgeschwindigkeit: 2 ml/min; Eluent: A: 0,1 % Trifluoressigsäure, B: Acetonitril/Wasser 8 : 2 + 0,07 % Trifluoressigsäure, 1 min. 40 % B, dann während 30 min. auf 60 % B steigern.

**Ergebnis**

Es werden sieben Produkte identifiziert, die fraktioniert und einzeln dem HLE-Test unterworfen werden. Ausserdem werden die isoelektrischen Punkte (IP; isoelektrische Fokussierung wie in Beispiel 27e beschrieben, LKB-Ampholine pH 4,0 – 6,5) bestimmt. Die Ergebnisse sind in der folgenden Tabelle zusammengefasst:

| Fraktion | Retentionszeit (min) | IP | HLE |
|---|---|---|---|
| F0 | 28,2 | 6,5 | + |
| F1 | 29,1 | { 6,4<br>6,3 | + |
| F1A | 30,0 | 5,3 | |
| F2 | 31,2 | 5,4 | + |
| F3 | 33,8 | 4,8 | + |
| F4 | 34,6 } | 4,8 | + |
| F4A | 35,4 | | |

Beim Hauptprodukt (Fraktion F2) handelt es sich auf Grund des gemessenen isoelektrischen Punktes, des HPLC-Wertes und der zur Kontrolle durchgeführten Molekulargewichtsbestimmung (gefundenes Molekulargewicht: 8133,2) um $N^{\alpha}$-Acetyl-eglin C. Die Substanz in Fraktion O (FO) ist natürliches Eglin C, wie der isoelektrische Punkt, der HPLC-Wert und die zur Kontrolle durchgeführten Molekulargewichtsbestimmung (gefundenes Molekulargewicht: 8091,2) beweisen.

**Beispiel 31**

**Test-Kit für Tandem ELISA mit monoklonalen anti-Eglin C Antikörpern**

Auf Mikrotiterplatten werden 300 ng/Vertiefung monoklonale Antikörper 299S18-20, gelöst in Natriumbikarbonat-Fixierungspuffer (pH 9,6), durch Inkubation über Nacht bei 4°C fixiert. Die Platten werden dreimal mit phosphatgepufferter Kochsalzlösung, enthaltend 0,005 % Tween 20 (H 7,2) gewaschen und die Vertiefungen anschliessend über Nacht bei 4°C mit 200 µl/Vertiefung phosphatgepufferter Kochsalzlösung enthaltend 0,2 % Gelatine und 0,02 % Natriumazid (PBS + Gelatine + A) behandelt. Wie zuvor wird dreimal gewaschen. Verschiedene Konzentrationen von Eglin C verdünnt in PBS + Gelatine + A werden zugefügt und 4 Std. bei Raumtemperatur inkubiert. Nach dreimaligem Waschen wie zuvor wird mit 100 µl/Vertiefung einer Mischung vom zweiten monoklonalen Antikörper-(299S22-1) gekuppelt an alkalische Phosphatase bei optimalem Titer (0,5 mg/ml Konjugat, für den Test 1 : 200 verdünnt mit PBS + Gelatine + A) versetzt und 2 Std. bei Raumtemperatur inkubiert, worauf nach Zugabe von 150 µl p-Nitrophenylphosphat in Diethanolaminpuffer (pH 9,8) die Farbe entwickelt wird. Die Farbintensität ($OD_{405}$) wird während einer Stunde alle 15 Minuten mit einem Multiscan ELISA Ablesegerät festgestellt.

Anhand einer Eichkurve mit bekannten Mengen natürlichem Eglin C, z. B. von $10^1$ bis $10^3$ ng/ml, wird durch Vergleich der gemessenen $OD_{405}$ der Gehalt an Eglin C in der zu untersuchenden Probe festgestellt.

Die Methode ist auch anwendbar zur Bestimmung von Eglin B oder einem anderen Eglin, z. B. $N^{\alpha}$-Acetyl-eglin C, und ist auch anwendbar, wenn die zu bestimmenden Egline sich im Plasma, z. B. in Ratten-, Katzen- oder Kaninchen-Plasma, befinden.

Ein Test-Kit für diesen Tandem ELISA umfasst die dafür notwendigen Reagentien, insbesondere monoklonale anti-Eglin Antikörper, z. B. 299S18-20 und 299S22-1, gegebenenfalls als Lösung in dem zu benutzenden Puffer, die zu benutzenden Puffer, inklusive den Substratpuffer, Waschlösungen, p-Nitrophenylphosphat als Substrat, eine Standardlösung enthaltend das zu bestimmende Eglin, z. B. Eglin C, eine Plastikmikrotiterplatte, und/oder gegebenenfalls eine Tabelle oder Eichkurve, z. B. die folgende, erhalten gemäss vorstehend beschriebenem Tandem ELISA:

| Natürliches Eglin C (ng/ml) | $OD_{405}$ |
|---|---|
| $10^0$ | 0,09 |
| $10^1$ | 0,18 |
| $10^2$ | 0,73 |
| $10^3$ | 1,23 |

| $N^{\alpha}$-Acetyl-eglin C (ng/ml) | $OD_{405}$ |
|---|---|
| $10^0$ | 0,08 |
| $10^1$ | 0,32 |
| $10^2$ | 1,00 |
| $10^3$ | 1,26 |

**Beispiel 32**

**Pharmazeutisches Präparat enthaltend $N^{\alpha}$-Acetyl-eglin C für parenterale Applikation**

Eine gemäss Beispiel 24 oder 25 hergestellte, $N^{\alpha}$-Acetyl-eglin C enthaltende Lösung wird gegen eine 0,9 %-ige NaCl-Lösung dialysiert. Die Konzentration der Lösung wird danach durch Verdünnen mit der gleichen NaCl-Lösung auf 1 mg/ml oder 10 mg/ml eingestellt. Diese Lösungen werden durch Ultrafiltration (Membranen mit 0,22 µm Poren) sterilisiert.

Die sterilisierten Lösungen sind direkt verwendbar für die intravenöse Verabreichung, für die Dauertropfinfusion, sowie zur Vernebelung in einem Inhalationsgerät (z. B. Bird).

Die erfindungsgemäss erhaltenen monoklonale anti-Eglin Antikörper produzierenden Hybridomazellen wurden am 6. November 1984 in der "Collection Nationale de Cultures de Microorganismes" des Institut Pasteur, Paris, Frankreich unter den folgenden Nummern hinterlegt:

| | |
|---|---|
| 299S18-20 | Nr. I-361 |
| 299S22-1 | Nr. I-362 |
| 299S22-10 | Nr. I-363 |

**Referenzen**

1. U. Seemüller et al, Hoppe-Seyler's Z. Physiol. Chem. *358*, 1105 (1977)
2. R. Knecht et al., Anal. Biochem. *130*, 65 (1983)
3. A.M. Maxam und W. Gilbert, Proc. Natl. Acad. Sci. USA *74*, 560 (1977); siehe auch Meth. Enzym. *65*, 499 (1980)
4. A. Hinnen et al., Proc. Natl. Acad. Sci. USA *75*, 1929 (1978)
5. Anagnostopoulos et al., J. Bacteriol. *81*, 741 (1961)
6. M. Mandel et al., J. Mol. Biol. *53*, 159 (1970)
7. U.K. Laemmli, Nature *227*, 680 (1970)
8. S. Tsunasawa und F. Sakiyama, in Methods Enzymol. *106*, 165 (1984)
9. S. Alkan et al., Mol. Immunol. *20*, 203 (1983)
10. T. Chard, An Introduction to Radioimmunoassay and related Techniques, North-Holland Publ. Comp., Amsterdam 1978
11. S.A. Narang, Tetrahedron *39*, 3 (1983)
12. K.L. Agarwal et al., Angew. Chem. *84*, 489 (1972)
13. C.B. Reese, Tetrahedron *34*, 3143 (1972)
14. R.L. Letsinger and W.B. Lunsford, J. Am. Chem. Soc. *98*, 3655 (1976)
15. K. Itakura et al., J. Am. Chem. Soc. *103*, 706 (1981)
16. H.G. Khorana et al., J. Biol. Chem. *251*, 565 (1976)
17. S.A. Narang et al., Anal. Biochem. *121*, 356 (1982)
18. K. Itakura et al., J. Biol. Chem. *257*, 9226 (1982)
19. Molecular Cloning, A Laboratory Manual (ed. T. Maniatis et al.), Cold Spring Harbor Lab., 1982, S. 125
20. A.E. Bolton und W.M. Hunter, Biochem. J. *133*, 529 (1973)
21. DE-OS-3 111 405 (Genentech)
22. A.C. Peacock et al., Biochemistry *6*, 1818 (1967)
23. W. Müller et al., J. Mol. Biol. *124*, 343 (1978)
24. M. Grunstein und D.S. Hogness, Proc. Natl. Acad. Sci. USA *72*, 3961 (1979)
25. Ish-Horowitz, in loc. cit. 19), S. 368
26. Köhler und Milstein, Nature *256*, 495 (1975)
27. H. Ako et al., Biochem. Biophys. Res. Comm., *46*, 1639 (1972)
28. H. Fritz et al., in: "Methoden der enzymatischen Analyse" (Hrsgb. H.U. Bergmeyer), 3. Auflage, Weinheim 1974, S. 1105
29. S. Moore et al., J. Biol. Chem. *192*, 663 (1951), D.H. Spadman et al., Anal. Chem. *30*, 1190 (1958)
30. H. Morris et al., Biochem. Biophys. Res. Comm. *117*, 299 (1983)
31. U. Seemüller et al., Hoppe-Seyler's Z. Physiol. Chem. *361*, 1841 (1980)
32. R. Knecht et al., Analyt. Biochem. *130*, 65 (1983)
33. W.F. Brandt et al., Z. Physiol. Chem. *357*, 1505 (1976)
34. A. Goldstein et al., Proc. Natl. Acad. Sci. USA *74*, 725 (1977)

35. R. Wetzel und D.V. Goeddel, in "The Peptides" (Hrsgb. E. Gross und J. Meienhofer), Academic Press, New York 1983, S. 1 – 64
36. J.G. Bieth, Bull. europ. physiopath. respirat. *16* (suppl.), 183 (1980)
37. L. Clarke und J. Carbon, J. Mol. Biol. *120*, 517 (1978)
38. D.S. Oppenheim und C. Yanofsky, J. Mol. Biol. *144*, 143 (1980)

**Patentansprüche** für die Vertragsstaaten: BE, CH, DE, FR, GB, IT, LI, LU, NL, SE

1. Eine Eglin-Verbindung der Formel

VGluPheGlySerGluLeuLysSerPheProGluValValGlyLysThrValAspGlnAlaArgGlu
TyrPheThrLeuHisTyrProGlnTyrAspValWPheLeuProGluGlySerProValThrLeuAsp
LeuArgTyrAsnArgValArgValPheTyrAsnProGlyThrAsnValValAsnHisValProHis (XIV'),
ValGly,

worin V für N-Acetyl-Thr steht und W Tyr oder His bedeutet und pharmazeutisch annehmbare Salze davon.

2. $N^{\alpha}$-Acetyl-eglin C gemäss Anspruch 1.

3. $N^{\alpha}$-Acetyl-eglin B gemäss Anspruch 1.

4. Pharmazeutisches Präparat enthaltend eine Verbindung der Formel XIV' gemäss Anspruch 1 oder ein pharmazeutisch annehmbares Salz davon.

5. Verbindungen der Formel XIV' oder pharmazeutisch annehmbare Salze davon gemäss Anspruch 1 zur Anwendung in einem Verfahren zur therapeutischen oder prophylaktischen Behandlung des menschlichen oder tierischen Körpers.

6. Verwendung von Verbindungen der Formel XIV' oder pharmazeutisch annehmbarer Salze davon gemäss Anspruch 1 zur Herstellung von pharmazeutischen Präparaten.

7. Verfahren zur Herstellung von $N^{\alpha}$-Acetyl-eglin B, $N^{\alpha}$-Acetyl-eglin C und Salzen davon, dadurch gekennzeichnet, dass man einen Escherichia coli- oder Saccharomyces cerevisiae-Stamm, der mit einem für die Expression in E. coli oder S. cerevisiae geeigneten DNA-Vektor enthaltend eine für Eglin B oder Eglin C kodierende DNA-Sequenz transformiert wurde, in einem flüssigen Nährmedium, welches assimilierbare Kohlenstoff- und Stickstoffquellen enthält, kultiviert und das Produkt aus den Wirtszellen freisetzt und isoliert, und, falls gewünscht, ein erhaltenes Salz in das freie Polypeptid oder ein erhaltenes Polypeptid in ein Salz desselben überführt.

8. Verfahren zur Herstellung von $N^{\alpha}$-Acetyl-eglin B gemäss Anspruch 7.

9. Verfahren zur Herstellung von $N^{\alpha}$-Acetyl-eglin C gemäss Anspruch 7.

**Patentansprüche** für den Vertragsstaat: AT

1. Verfahren zur Herstellung von $N^{\alpha}$-Acetyl-eglin B, $N^{\alpha}$-Acetyl-eglin C und Salzen davon, dadurch gekennzeichnet, dass man einen Escherichia coli- oder Saccharomyces cerevisiae-Stamm, der mit einem für die Expression in E. coli oder S. cerevisiae geeigneten DNA-Vektor enthaltend eine für Eglin B oder Eglin C kodierende DNA-Sequenz transformiert wurde, in einem flüssigen Nährmedium, welches assimilierbare Kohlenstoff- und Stickstoffquellen enthält, kultiviert und das Produkt aus den Wirtszellen freisetzt und isoliert, und, falls gewünscht, ein erhaltenes Salz in das freie Polypeptid oder ein erhaltenes Polypeptid in ein Salz desselben überführt.

2. Verfahren zur Herstellung von $N^{\alpha}$-Acetyl-eglin B gemäss Anspruch 1.

3. Verfahren zur Herstellung von $N^{\alpha}$-Acetyl-eglin C gemäss Anspruch 1.

**Claims** for the Contracting States: BE, CH, DE, FR, GB, IT, LI, LU, NL, SE

1. An eglin compound of the formula

VGluPheGlySerGluLeuLysSerPheProGluValValGlyLysThrValAspGlnAlaArgGlu
TyrPheThrLeuHisTyrProGlnTyrAspValWPheLeuProGluGlySerProValThrLeuAsp
LeuArgTyrAsnArgValArgValPheTyrAsnProGlyThrAsnValValAsnHisValProHis (XIV')
ValGly

in which V is N-acetyl-Thr and W is Tyr or His, and a pharmaceutically acceptable salt thereof.

2. $N^{\alpha}$-acetyl-eglin C according to claim 1.

3. $N^{\alpha}$-acetyl-eglin B according to claim 2.

4. A pharmaceutical product containing a compound of the formula XIV' according to claim 1 or a pharmaceutically acceptable salt thereof.

5. A compound of the formula XIV' or a pharmaceutically acceptable salt thereof according to claim 1, for use in a process for the therapeutic or prophylactic treatment of the human or animal body.

6. The use of a compound of the formula XIV' or of a pharmaceutically acceptable salt thereof according to claim 1, for the preparation of a pharmaceutical product.

7. A process for the preparation of $N^{\alpha}$-acetyl-eglin B, $N^{\alpha}$-acetyl-eglin C and a salt thereof which comprises culturing an Escherichia coli or Saccharomyces cerevisiae strain which has been transformed with a DNA vector containing a DNA sequence coded for eglin B or eglin C and suitable for expression in E. coli or S. cerevisiae, in a liquid nutrient medium containing assimilatable sources of carbon and nitrogen, releasing the product from the host cells and isolating it, and, if desired, converting a resulting salt into the free polypeptide and converting a resulting polypeptide into a salt thereof.

8. A process for the preparation of $N^{\alpha}$-acetyl-eglin B according to claim 7.

9. A process for the preparation of $N^{\alpha}$-acetyl-eglin C according to claim 7.

**Claims** for the Contracting State: AT

1. A process for the preparation of $N^{\alpha}$-acetyl-eglin B, $N^{\alpha}$-acetyl-eglin C and a salt thereof which comprises culturing an Escherichia coli or Saccharomyces cerevisiae strain which has been transformed with a DNA vector containing a DNA sequence coded for eglin B or eglin C and suitable for expression in E. coli or S. cerevisiae, in a liquid nutrient medium containing assimilatable sources of carbon and nitrogen, releasing the product from the host cells and isolating it, and, if desired, converting a resulting salt into the free polypeptide and converting a resulting polypeptide into a salt thereof.

2. A process for the preparation of $N^{\alpha}$-acetyl-eglin B according to claim 1.

3. A process for the preparation of $N^{\alpha}$-acetyl-eglin C according to claim 1.

**Revendications** pour les Etats Contractants: BE, CH, DE, FR, GB, IT, LI, LU, NL, SE

1. Un composé de l'égline de formule

VGluPheGlySerGluLeuLysSerPheProGluValValGlyLysThrValAspGlnAlaArgGlu
TyrPheThrLeuHisTyrProGlnTyrAspValWPheLeuProGluGlySerProValThrLeuAsp (XIV')
LeuArgTyrAsnArgValArgValPheTyrAsnProGlyThrAsnValValAsnHisValProHis
ValGly

où V représente Thr N-acétyle et W représente Tyr ou His et ses sels pharmaceutiquement acceptables.

2. Egline C $N^{\alpha}$-acétyle conforme à la revendication 1.

3. Egline B $N^{\alpha}$-acétyle conforme à la revendication 1.

4. Préparation pharmaceutique contenant un composé de formule XIV' conforme à la revendication 1 ou l'un de ses sels pharmaceutiquement acceptables.

5. Composés de formule XIV' ou leurs sels pharmaceutiquement acceptables destinés à être utilisés dans un procédé pour le traitement thérapeutique ou prophylactique du corps humain ou animal.

6. Utilisation des composés de formule XIV' ou de leurs sels pharmaceutiquement acceptables conformes à la revendication 1 pour la préparation de préparations pharmaceutiques.

7. Procédé de préparation de l'égline B $N^{\alpha}$-acétyle, de l'égline C $N^{\alpha}$-acétyle et de leurs sels, caractérisé en ce que l'on cultive une souche d'Escherichia coli ou de Saccharomyces cerevisiae transformée par un vecteur d'ADN approprié

pour l'expression dans E. coli ou S. cerevisiae contenant une séquence d'ADN codant pour l'égline B ou l'égline C, dans un milieu nutritif liquide contenant des sources de carbone et d'azote assimilables, en ce qu'on libère le produit des cellules hôtes, en ce qu'on l'isole et, si désiré, en ce que l'on transforme le sel obtenu en un polypeptide libre ou en ce que l'on transforme le polypeptide obtenu en un sel.

8. Procédé de préparation de l'égline B $N^{\alpha}$-acétyle conforme à la revendication 7.

9. Procédé de préparation de l'égline C $N^{\alpha}$-acétyle conforme à la revendication 7.

**Revendications** pour l'Etat Contractant: AT

1. Procédé de préparation de l'égline B $N^{\alpha}$-acétyle, de l'égline C $N^{\alpha}$-acétyle et de leurs sels, caractérisé en ce que l'on cultive une souche d'Escherichia coli ou de Saccharomyces cerevisiae transformée par un vecteur d'ADN approprié pour l'expression dans E. coli ou S. cerevisiae contenant une séquence d'ADN codant pour l'égline B ou l'égline C, dans un milieu nutritif liquide contenant des sources de carbone et d'azote assimilables, en ce qu'on libère le produit des cellules hôtes, en ce qu'on l'isole et, si désiré, en ce que l'on transforme le sel obtenu en un polypeptide libre ou en ce que l'on transforme le polypeptide obtenu en un sel.

2. Procédé de préparation de l'égline B $N^{\alpha}$-acétyle conforme à la revendication 1.

3. Procédé de préparation de l'égline C $N^{\alpha}$-acétyle conforme à la revendication 1.

**Fig. 1** SYNTHESESCHEMA FÜR DIE TEILSTÜCKE $F_1$(C) UND $F_2$ DES EGLIN C-GENS

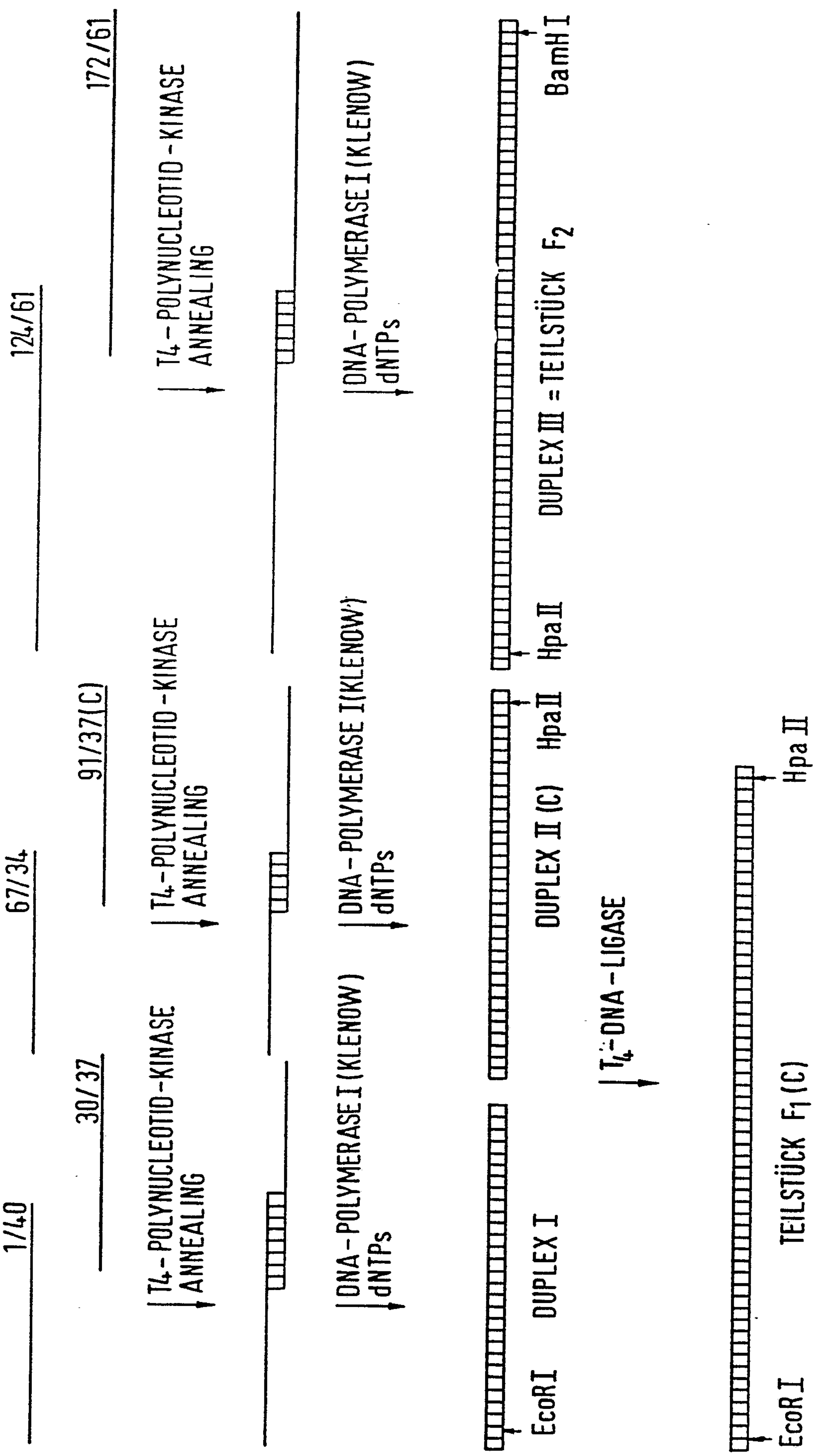

Fig. 2 KONSTRUKTION DES PLASMIDS pML87

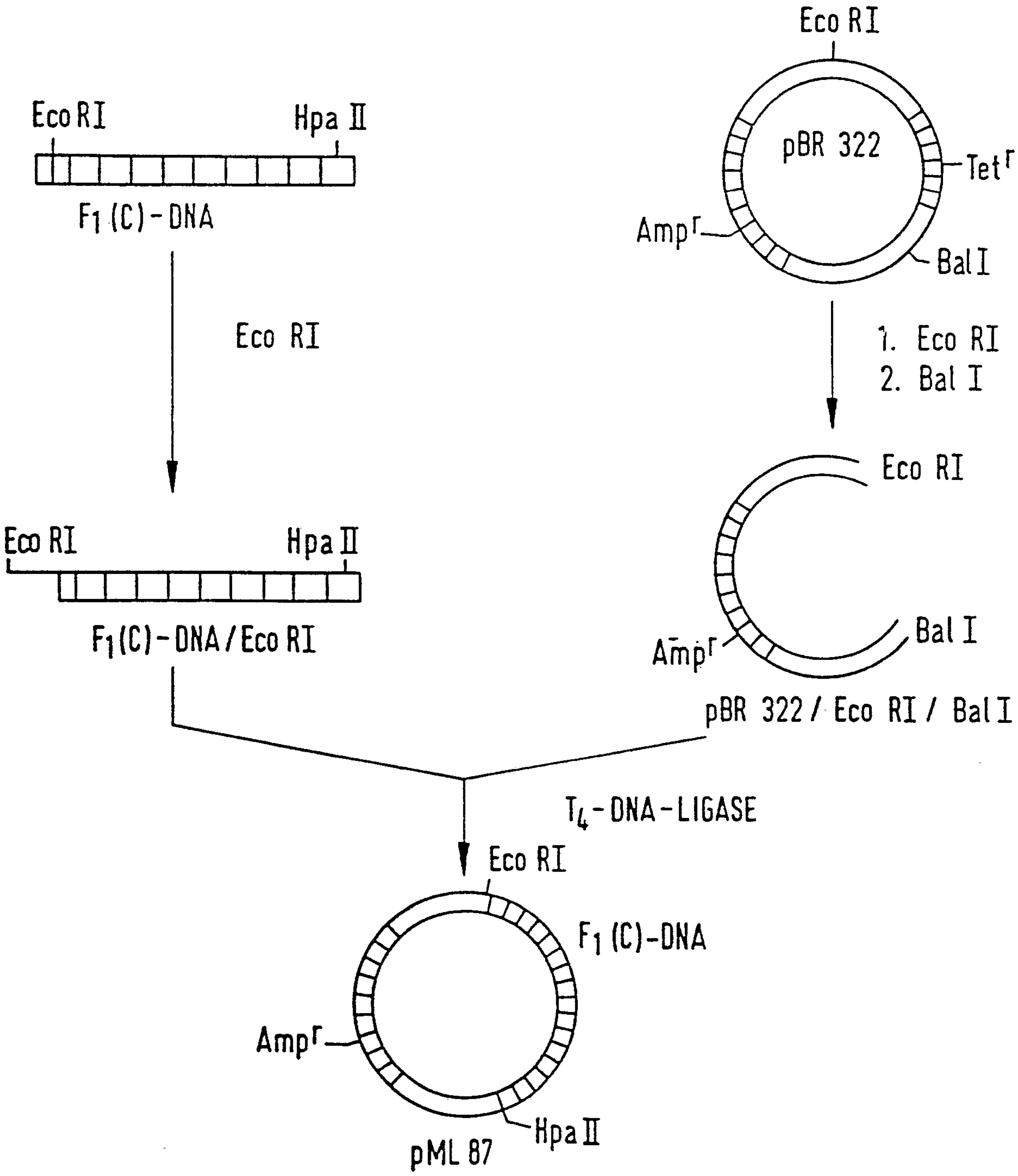

Fig. 3 KONSTRUKTION DES PLASMIDS pML 136

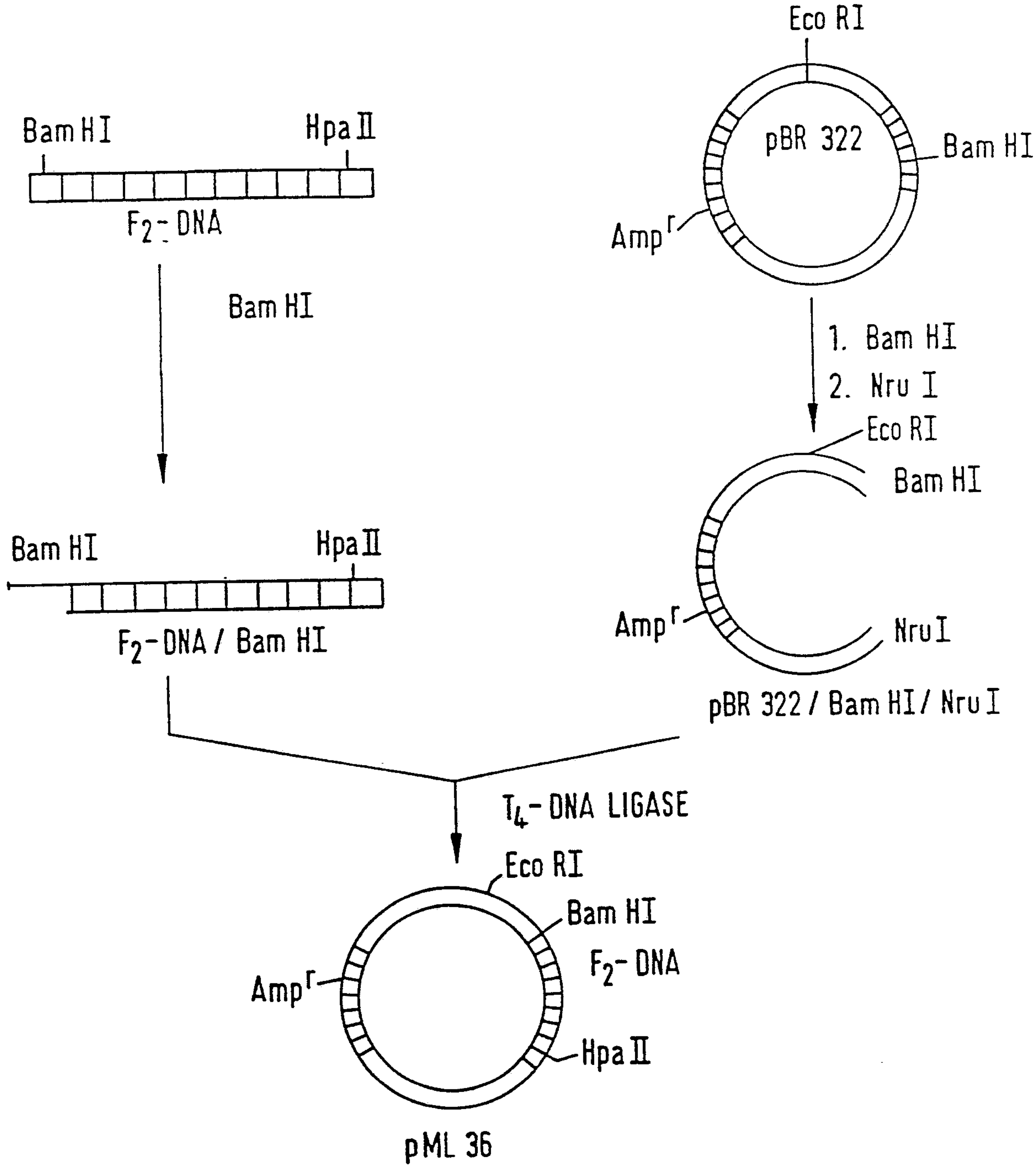

Fig.4 KONSTRUKTION DES PLASMIDS pML 141

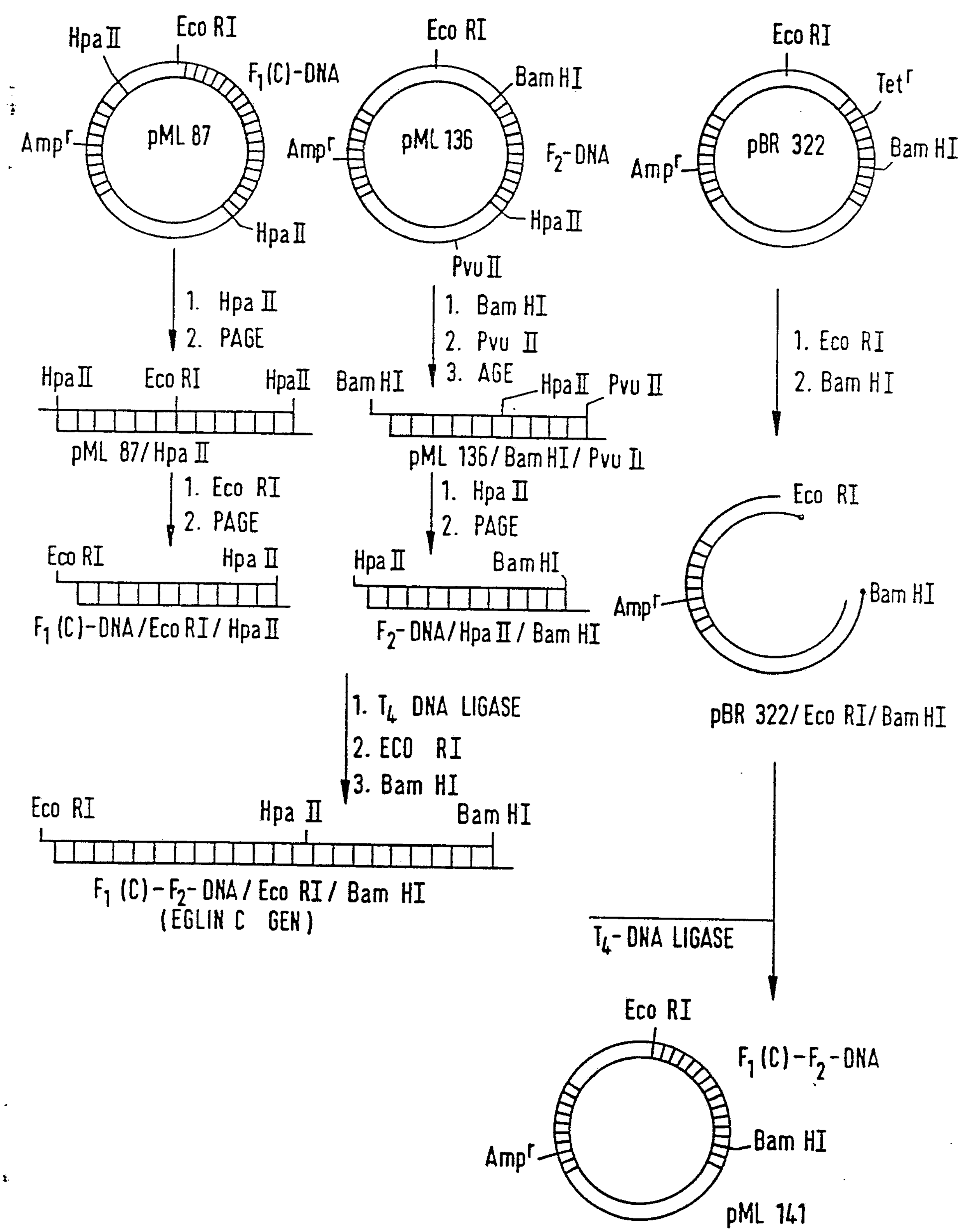

Fig. 5 KONSTRUKTION DES PLASMIDS pHRi 148

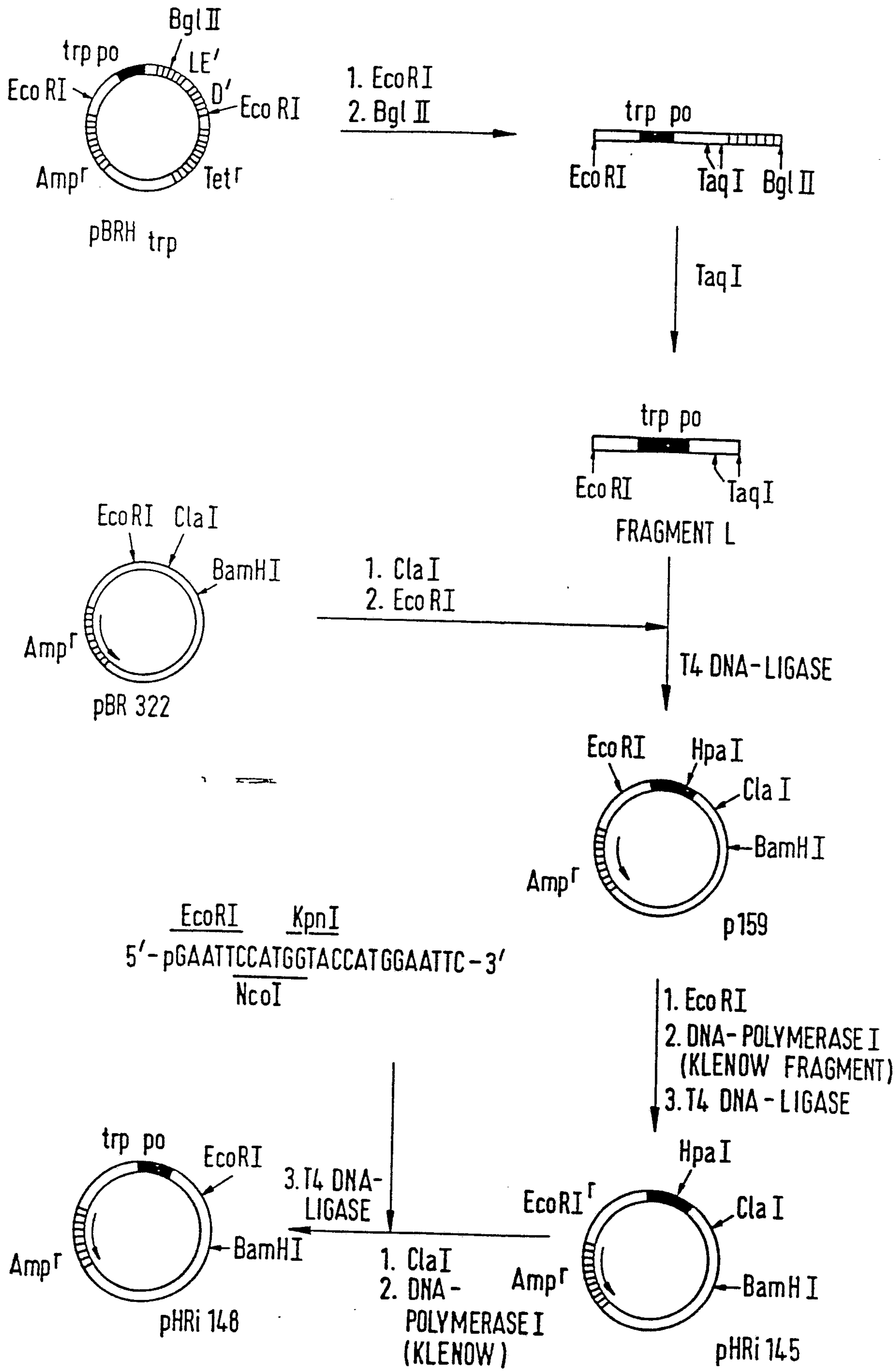

Fig. 6 KONSTRUKTION DES EXPRESSIONSPLASMIDS pML 147